# EUROPEAN PATENT APPLICATION

(11) **EP 1 972 628 A1**
(43) Date of publication of application: **24.09.2008**
(21) Application number: 07005765.8
(22) Date of filing: 21.03.2007
(51) Int. Cl.: C07D 471/04, A61K 31/437, A61P 25/18

(54) **Indolizines and aza-analog derivatives thereof as CNS active compounds**

(71) Applicant: SCHWARZ PHARMA AG, 40789 Monheim (DE)
(72) Inventor: Huebner, Harald, 91336 Heroldsbach (DE); Gmeiner, Peter, 91054 Erlangen-Buckendorf (DE); Skultety, Marika, 96342 Stockheim (DE)
(74) Representative: Dressen, Frank

(57) **Abstract**

Presently disclosed are indolicine-based compounds of the general formula 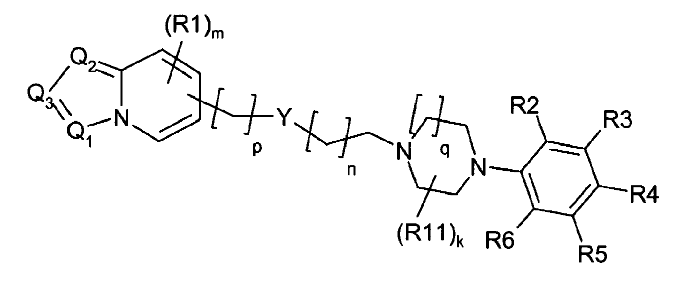 which have medical utility, for example as antipsychotics.

## Description

### Background:

Schizophrenia and other psychotic disorders including manic phases of bipolar disorder, acute idiopathic psychotic illnesses, and other conditions marked by severe agitation exhibit major disturbances in reasoning, often with delusions and hallucinations. Schizophrenia affects about 1% of the population and about 5-10% of the affected patients commit suicide. The cost to the society is assessed to be several billions of dollars, and there is thus a significant need to provide effective pharmacological treatment options for the treatment of schizophrenia and other psychotic disorders.

It is currently believed that excessive dopamine activity is one of the major underlying causes of schizophrenia. Support for this hypothesis comes from the observation that most of the currently available antipsychotic agents are dopamine D2 receptor antagonists, so called neuroleptics. Examples of typical neuroleptics are haloperidol, loxapine, pimozide, phenothiazine derivatives such as chlorpromazine and fluphenazine, or thioxanthene derivatives such as thiothixene. However, due to their strong and selective D2 antagonism, these first generation antipsychotics have a substantial risk of adverse extrapyramidal neurological side effects as well as an increased release of prolactin. Also, pure D2 antagonists only have an impact on the so-called "positive symptoms" of psychotic disorders, like delusions and halluzinations, which are caused by the overexpression of dopamine receptors in some brain segments but are not effective on "negative symptoms" such as apathy or alogia which are thought to be caused by reduced dopaminergic activity in other areas of the CNS. Finally, about 30% of the patients do not respond well to typical D2 antagonists.

As a consequence, "atypical antipsychotics" or "2^{nd} generation antipsychotics" have been developed. Examples include clozapine, risperidone, and ziprasidone. Atypical antipsychotics have a more complex receptor profile than first generation antipsychotics and besides showing high affinity binding to and inhibition of dopaminergic D2 receptors, also modulate other receptors, like serotonergic and/or adrenergic receptors. For example, clozapine also interacts with a couple of adrenergic, serotonergic, muscarinergic and histaminergic receptors and has proved effective in patients who respond poorly to standard neuroleptics (Goodman&Gilman's "The pharmacological Basis of therapeutics", Editor Laurence L. Brunton, 11th edition, 2005). Likewise, risperidone and ziprasidone are combined D2/5-HT2a receptor antagonists and 5-HT1 a agonists. It was thus suggested that atypicality may arise from interactions with the 5-hydroxytryptamine 5-HT2 receptor in addition to the dopaminergic D2 antagonism. Compounds also acting as serotonin 5-HT1A agonists may additionally alleviate negative symptoms of schizophrenia and reduce the likelihood of extrapyramidal symptoms.

A full blockade of D2 receptors can lead to dopamine hypoactivity which is associated with side effects such as extrapyramidal motor dysfunction. A novel strategy to overcome this shortcoming of existing antipsychotics is thus the development of partial dopamine receptor antagonist. Such compounds, like aripiprazole and bifeprunox, are sometimes called "3^{rd} generation antipsychotics", and do act as antagonist at D2 when there is an overstimulation of dopamine receptors while activating these D2 receptors when there is insufficient stimulation by endogenous dopamine. It is thus believed that partial D2 agonists act as "dopamine stabilisers", and offer the advantage of full therapeutic efficacy with minimal side effect liability.

Moreover, the modulation of the dopaminergic D3 and the D4 receptors is thought to be useful in the treatment of cognitive and affective symptoms associated with psychotic diseases.

The chemical structures of clozapine, risperidone, ziprasidone and aripiprazole are depicted below: 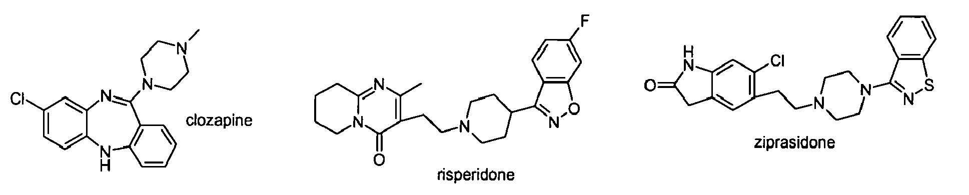 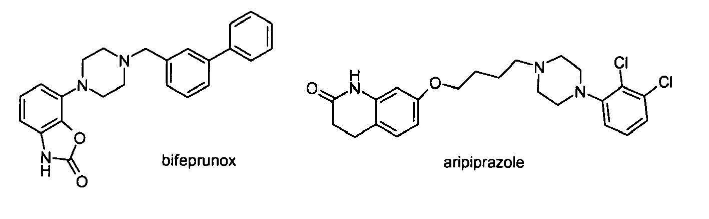 In one embodiment, the present patent application provides novel compounds that show high affinity to the dopaminergic D2 receptor, but which also interact with significant affinity with the 5-HT2 serotonergic receptor, the 5-HT1a serotonergic receptor and the dopaminergic D3 and D4 receptors.

A preferred embodiment of the present invention relates to new compounds which exert partial dopaminergic activity at the D2 receptor e.g. by having an intrinsic dopaminergic D2 activity that is up to about 50% of a full agonist.

It has been now found, surprisingly, that in one embodiment indolicine derivatives having the general formula A 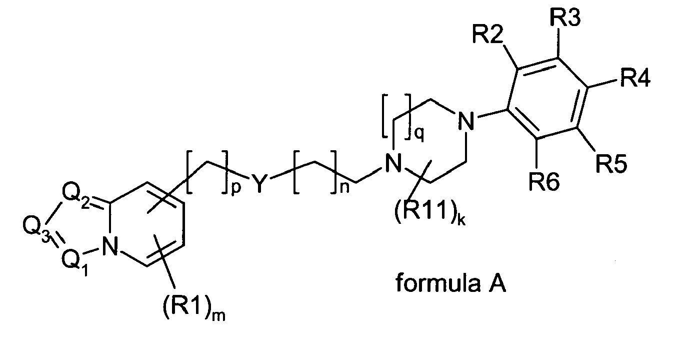 exhibit the target profile of a modern atypical antipsychotic.

In particular, the compounds as described in more detail further below and in the claims inter alia have a remarkable affinity at the D2 receptor, and do also show binding to the 5-HT2, the 5-HT1a, the D3 and D4 receptors in a range comparable to or even superior than other atypical antipsychotics like aripiprazole.

### Detailed description of the invention

One embodiment of the present invention relates to compounds of the general formula I, 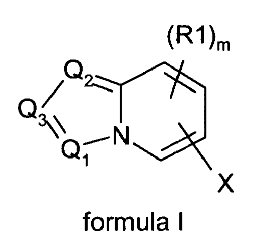 wherein:
Q1 and Q2 are independently of each other N, CH or C-R1;
Q3 is CH or C-R1 if at least one of Q1 and Q2 is different from nitrogen, and Q3 is selected from CH, C-R1 and N if Q1 and Q2 are both nitrogen;
m is 0, 1, 2 or 3;
any R1 is bonded to a C-atom of the heterocycle of formula I and is independently of each other selected from the group comprising of hydroxy, formyl, oxime, cyano, nitro, amino, NR7R8, carboxy, carbamoyl, alkyl, cycloalkyl, alkyloxy, alkylthio, alkenyl, alkynyl, phenyl, heteroaryl, phenoxy, halogen, trifluoromethyl, alkylcarbonyl, alkylaminocarbonyl, dialkylaminocarbonyl, phenylcarbonyl, phenylalkyl, phenylalkyloxy, phenylalkylcarbonyl, phenylalkyloxycarbonyl, alkyloxycarbonyl, alkylsulfonyl, phenylsulfonyl,sulfamoyl, alkylaminosulfonyl, dialkylaminosulfonyl, phenylsulfonylamino and alkylsulfonylamino; wherein each alkyl, alkenyl or alkynyl may be unsubstituted or substituted with one or more residues selected from among hydroxyl, alkyloxy, halogen, and NR7R8; and wherein each heteroaryl is a monocyclic ring and wherein each phenyl or heteroaryl can be unsubstituted or substituted with one or more residues selected from among hydroxyl, alkyloxy, halogen, alkyl, cycloalkyl which is preferably unsubstituted, carboxy, NR7R8, cyano, trifluoromethyl and nitro; and wherein each cycloalkyl can be unsubstituted or substituted with hydroxyl, alkyloxy, halogen, alkyl, phenyl which is preferably unsubstituted, amino and NR7R8.
X is a group having the formula 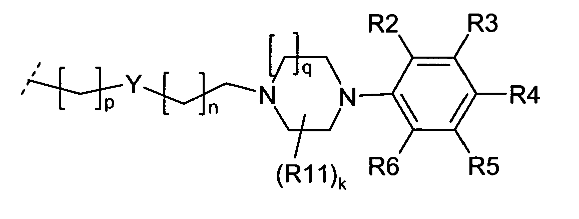
wherein:
Y is chosen from among S and O;
k is 0, 1 or 2;
n is 1-5;
p is 0, 1 or 2;
q is 1 or 2;
R2, R3, R4, R5 and R6 are in each case and independently of each other selected from the group comprising hydrogen, hydroxy, formyl, oxime, cyano, nitro, amino, NR7R8, carboxy, carbamoyl, alkyl, alkyloxy, alkylthio, alkenyl, alkynyl, cycloalkyl, phenyl, heteroaryl, phenoxy, halogen, trifluoromethyl, alkylcarbonyl, alkylaminocarbonyl, dialkylaminocarbonyl, phenylcarbonyl, phenylalkyl, phenylalkyloxy, phenylalkylcarbonyl, phenylalkyloxycarbonyl, alkyloxycarbonyl, alkylsulfonyl, phenylsulfonyl, sulfamoyl, alkylaminosulfonyl, dialkylaminosulfonyl, phenylsulfonylamino and alkylsulfonylamino; wherein each alkyl, alkenyl or alkynyl may be unsubstituted or substituted with one or more residues selected from among hydroxyl, alkyloxy, halogen, and NR7R8; and wherein each heteroaryl is a monocyclic ring and wherein each phenyl or heteroaryl can be unsubstituted or substituted with one or more residues selected from among hydroxyl, alkyloxy, halogen, alkyl, cycloalkyl which is preferably unsubstituted, carboxy, NR7R8, cyano, trifluormethyl and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, alkyl, cycloalkyl, phenyl, heteroaryl, phenylalkyl, alkylsulfonyl, phenylsulfonyl, heteroarylsulfonyl, alkylcarbonyl, alkyloxycarbonyl, aminocarbonyl, alkylaminocarbonyl, phenylcarbonyl, and heteroarylcarbonyl; wherein each alkyl may be unsubstituted or substituted with one or more residues selected from among hydroxyl, alkyloxy, phenyl, fluoro, carboxy, and NR9R10; and wherein R7 and R8 may form a 5- to 7- membered cycle; and wherein each heterocycle is a monocyclic ring and wherein phenyl or heteroaryl can be unsubstituted or substituted with one or more residues selected from among hydroxyl, alkyloxy, halogen, alkyl, carboxy, NR9R10, cyano, trifluormethyl and nitro;
R9 and R10 are independently selected from among hydrogen and alkyl;
R11 is hydroxyl, (C1-C3)alkyl, hydroxy-(C1-C3)alkyl, halogen-(C1-C3)alkyl or oxo;
provided that the heterocycle of formula I carries precisely one group X;
in the form of the free base, their physiologically acceptable salts, possible conformational isomers, enatiomers, and diastereomers.

One embodiment of the present invention relates to compounds of formulas IIa-IId 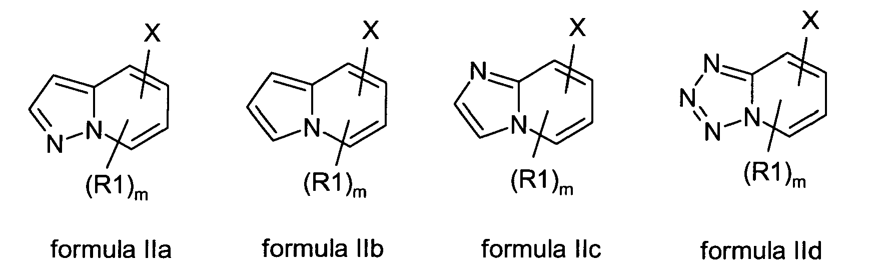 wherein:
m is 0, 1, 2 or 3;
any R1 is bonded to a C-atom of a heterocycle of formulas IIa-IId and is independently of each other selected from the group comprising of hydroxy, formyl, oxime, cyano, nitro, amino, NR7R8, carboxy, carbamoyl, (C1-C10)alkyl, (C1-C10)alkyloxy, (C1-C10)alkylthio, (C2-C10)alkenyl, (C2-C10)alkynyl, (C3-C7)cycloalkyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C7)cycloalkyl, carboxy, NR7R8, cyano, trifluoromethyl, and nitro;
X is a group having the formula 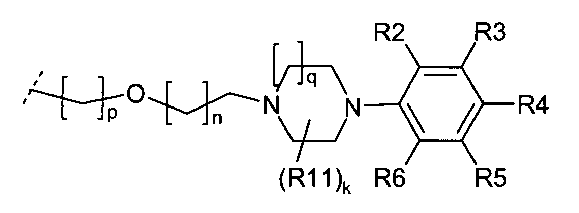 wherein:
   k is 0, 1 or 2;
   n is 1-5;
   p is 0, 1 or 2;
   q is 1 or 2;
   R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C1-C10)alkyloxy, (C1-C10)alkylthio, (C2-C10)alkenyl, (C2-C10)alkynyl, (C3-C7)cycloalkyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C7)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
   R7 and R8 are independently selected from hydrogen, (C1-C6)alkyl, (C3-C7)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
   R9 and R10 are independently selected from among hydrogen, and alkyl;
   R11 is methyl, ethyl, hydroxyl, hydroxymethyl, or oxo;
      provided that each heterocycle of formulas IIa-IId carries precisely one group X;
      in the form of the free base, their physiologically acceptable salts and possible enatiomers and diastereomers.

One preferred embodiment of the present invention is a compound of formula I or formula IIa, IIb or IIc as described further above, wherein q is 1.

Another embodiment of the present invention is a compound of formula I or formula IIa, IIb or IIc as described further above, wherein k is 0.

Another embodiment of the present invention is a compound of formula I or formula IIa, IIb or IIc as described further above, wherein R4 is fluoro or hydrogen, particularly hydrogen.

Another embodiment of the present invention is a compound of formula I or formula IIa, IIb or IIc as described further above, wherein q is 1, k is 0 and R4 is fluoro or hydrogen, particularly hydrogen.

Another embodiment of the present invention is a compound of formula I or formula IIa, IIb or IIc as described further above, wherein k is 1.

Another embodiment of the present invention is a compound of formula I or formula IIa, IIb or IIc as described further above, wherein k is 1 and R11 is preferably an C1-C6 alkyl, a hydroxyl, or an oxo group.

Another embodiment of the present invention is a compound of formula I or formula IIa, IIb or IIc as described further above, wherein k is 2. In this embodiment, the two substituents R11 may be the same or different, and may be located at different or at the same C-Atom of the N-containing ring. Preferably, the two substituents are the same and are bound to the same ring C-atom such that they do not form a stereocenter.

Another embodiment of the present invention is a compound of formula I or formula IIa, IIb or IIc as described further above, wherein
m is 0, 1 or 2;
p is 0 or 1;
n is 2, 3 or 4; and
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8; wherein R7 and R8 are selected from among hydrogen, (C1-C3)alkyl, (C1-C3)alkylcarbonyl and phenyl(C1-C3)alkyl.

Another embodiment of the present invention is a compound of formula I or formula IIa, IIb, IIc or IId as described further above, wherein
m is 0, 1 or 2;
p is 0 or 1;
n is 2, 3 or 4;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8, and is preferably hydrogen or fluoro, and particularly preferably hydrogen; and
R5 and R6 are independently selected from hydrogen, or fluoro;
   wherein R7 and R8 are independently selected from hydrogen, (C1-C3)alkylcarbonyl, (C1-C3)alkyl, and phenyl(C1-C3)alkyl.

A preferred embodiment of the present invention is a compound according to anyone of the preceding claims, having the formula IIa 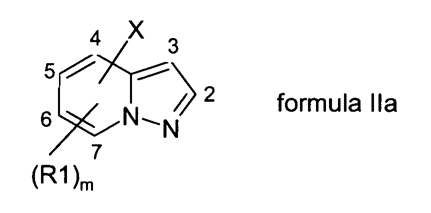 wherein X, R1 and m have the meaning as described further above.

Another embodiment of the present invention is a compound of formula IIa as described above, wherein the group X is bonded to the 2-, 4-, 5- or 6-position of the heteroaromatic ring system of formula IIa.

Another embodiment of the present invention is a compound of formula IIa as described above, wherein m is 1, and R1 is in 3-position of the heteroaromatic ring system of the general formula IIa.

Another embodiment of the present invention is a compound of formula IIa as described further above, wherein X is 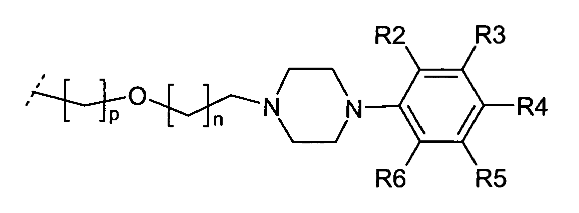 wherein p is 0 or 1, and the sum of p and n is 3; and R2-R6 have the meaning as defined further above.

Another embodiment of the present invention is a compound of formula IIa as described above, wherein R4 is hydrogen or fluoro.

Another preferred embodiment of the present invention is a compound having the formula III 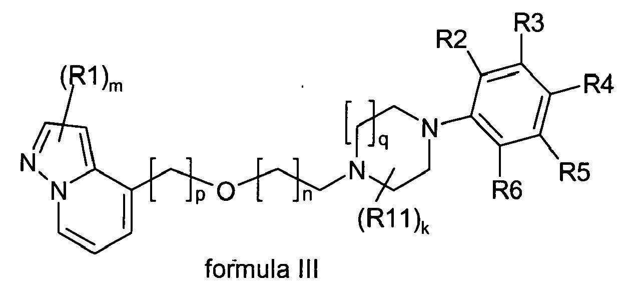 wherein:
R1, R2, R3, R4, R5, R6, R11, k, m, n, p and q are as defined for compounds of formula I, further above.
In a preferred embodiment,
k is 0, 1, or 2;
m is 0, 1, 2 or 3;
n is 1-5;
p is 0, 1 or 2;
q is 1 or 2, and is preferably 1;
any R1 is bonded to a C-atom of the heterocycle of formula III, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C6)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
provided that the heterocycle of formula III carries precisely one group X;
in the form of the free base, their physiologically acceptable salts and possible enatiomers and diastereomers.

Another embodiment of the present invention is a compound of formula III as described above, wherein R4 is hydrogen or fluoro.

Another preferred embodiment of the present disclosure is a compound having the general formula III, wherein
k is 0 or 1;
m is 0, 1 or 2;
p is 0 or 1;
n is 2, 3 or 4;
q is 1;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8, and is preferably hydrogen or fluoro, and particularly preferably hydrogen;
R5 and R6 are independently selected from hydrogen, or fluoro;
wherein R7 and R8 are independently selected from hydrogen, (C1-C3)alkylcarbonyl, (C1-C3)alkyl, and phenyl(C1-C3)alkyl.

Another preferred embodiment of the present disclosure is a compound having the general formula III, wherein
k is 0;
m is 0 or 1;
n is 3;
p is 0;
qis1;
R1 is hydrogen, or formyl,
R2 and R3 are independently selected from hydrogen, methoxy, chloro, amino, and acetylamino;
R4, R5 and R6 are all hydrogen.

Another embodiment of the present disclosure is a compound having the formula IV 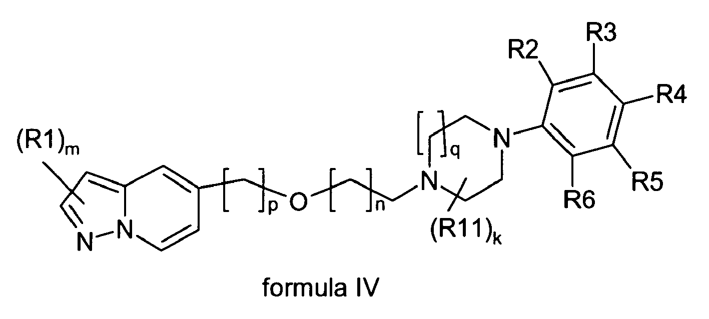 wherein:
R1, R2, R3, R4, R5, R6, R11, k, m, n, p and q are as defined for compounds of formula I, further above.
In a preferred embodiment,
k is 0,1 or 2;
m is 0, 1, 2 or 3;
n is 1-5;
p is 0, 1 or 2;
q is 1 or 2, and is preferably 1;
any R1 is bonded to a C-atom of the heterocycle of formula IV, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C6)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
provided that the heterocycle of formula IV carries precisely one group X;
in the form of the free base, their physiologically acceptable salts and possible enatiomers and diastereomers.

Another embodiment of the present invention is a compound of formula IV as described above, wherein R4 is hydrogen or fluoro.

Another preferred embodiment of the present disclosure is a compound of formula IV as described above, wherein
k is 0 or 1;
m is 0, 1 or 2;
p is 0 or 1;
n is 2, 3 or 4;
q is 1;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8, and is preferably hydrogen or fluoro, particularly preferably hydrogen;
R5 and R6 are independently selected from hydrogen, or fluoro;
   wherein R7 and R8 are independently selected from hydrogen, (C1-C3)alkylcarbonyl, (C1-C3)alkyl, and phenyl(C1-C3)alkyl.

Another preferred embodiment of the present disclosure is a compound of formula IV as described above, wherein
k is 0;
m is 0 or 1;
n is 3;
p is 0;
q is 1;
R1 is hydrogen, or formyl, cyano, oxime, (C1-C3)alkyloxycarbonyl, chloro, bromo, iodo, hydroxymethyl, nitro, NR7R8;
R2 and R3 are independently selected from hydrogen, methoxy, chloro, amino, and acetylamino;
R4 is selected from hydrogen, fluoro, and chloro, and is particularly preferably hydrogen;
R5 and R6 are both hydrogen.

Another preferred embodiment of the present invention are compounds having the formula V 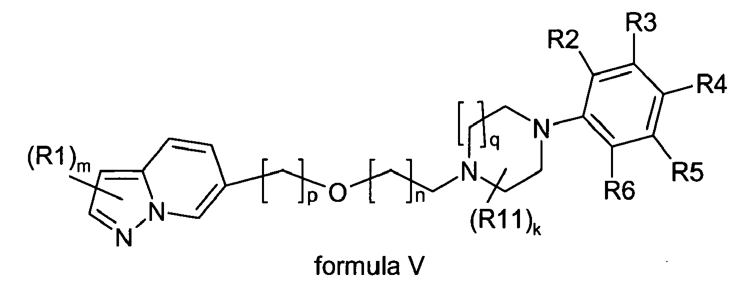 wherein
R1, R2, R3, R4, R5, R6, R11, k, m, n, p and q are as defined for compounds of formula I, further above.
In a preferred embodiment,
k is 0, 1 or 2;
m is 0, 1, 2 or 3;
n is 1-5;
p is 0, 1 or 2;
q is 1 or 2, and is preferably 0;
any R1 is bonded to a C-atom of the heterocycle of formulas V, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C6)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
provided that the heterocycle of formula V carries precisely one group X;
in the form of the free base, their physiologically acceptable salts and possible enatiomers and diastereomers.

Another embodiment of the present invention is a compound of formula V as described above, wherein R4 is hydrogen or fluoro.

Another preferred embodiment of the present disclosure is a compound of formula V as described above, wherein
k is 0 or 1;
m is 0, 1 or 2;
p is 0 or 1;
n is 2, 3 or 4;
q is 1;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8, and is preferably hydrogen or fluoro, and particularly preferably hydrogen;
R5 and R6 are independently selected from hydrogen, or fluoro;
wherein R7 and R8 are independently selected from hydrogen, (C1-C3)alkylcarbonyl, (C1-C3)alkyl, and phenyl(C1-C3)alkyl.

Another preferred embodiment of the present disclosure is a compound of formula IV as described above, wherein
K is 0;
m is 0 or 1
n is 3;
p is 0;
q is 1;
R1 is hydrogen, or formyl,
R2 and R3 are independently selected from hydrogen, methoxy, chloro, amino, and acetylamino;
R4, R5 and R6 are all hydrogen.

Another preferred embodiment of the present invention are compounds having the general formula VI 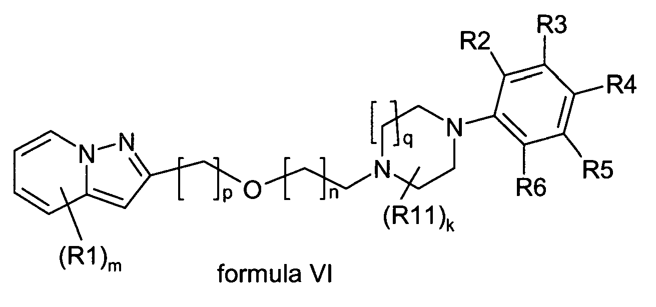 wherein:
R1, R2, R3, R4, R5, R6, R11, k, m, n, p and q are as defined for compounds of formula I, further above.
In a preferred embodiment,
k is 0,1 or 2;
m is 0, 1, 2 or 3;
n is 1-5;
p is 0, 1 or 2;
q is 1 or 2, and is preferably 1;
any R1 is bonded to a C-atom of the heterocycle of formula VI, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C6)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
   provided that the heterocycle of formula VI carries precisely one group X;
   in the form of the free base, their physiologically acceptable salts and possible enatiomers and diastereomers.

Another preferred embodiment of the present disclosure is a compound of formula VI as described above, wherein R4 is hydrogen or fluoro, and is preferably hydrogen.

Another preferred embodiment of the present disclosure is a compound of formula VI as described above, wherein
kis0or1;
m is 0, 1 or 2;
p is 0 or 1;
n is 2, 3 or 4;
q is 1;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8, and is preferably hydrogen or fluoro, and particularly preferably hydrogen;
R5 and R6 are independently selected from hydrogen, or fluoro;
wherein R7 and R8 are independently selected from hydrogen, (C1-C3)alkylcarbonyl, (C1-C3)alkyl, and phenyl(C1-C3)alkyl.

Another preferred embodiment of the present disclosure is a compound of formula VI as described above, wherein
k is 0;
m is 0 or 1;
n is 3;
p is 0;
q is 1;
R1 is hydrogen, or formyl,
R2 and R3 are independently selected from hydrogen, methoxy, chloro, amino, and acetylamino;
R4, R5 and R6 are all hydrogen.

Also presently disclosed are compounds having the general formula VII 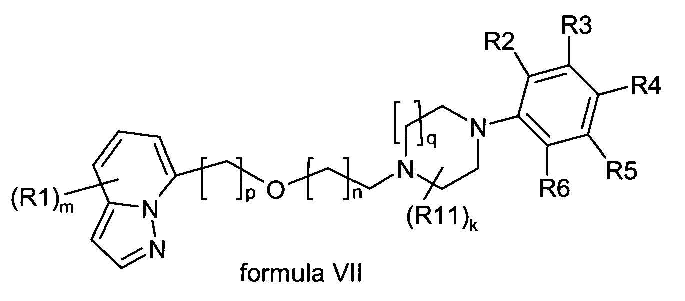 wherein:
R1, R2, R3, R4, R5, R6, R11, k, m, n, p and q are as defined for compounds of formula I, further above.
In a preferred embodiment,
k is 0,1 or 2;
m is 0, 1, 2 or 3;
n is 1-5;
p is 0, 1 or 2;
q is 1 or 2, and is preferably 1;
any R1 is bonded to a C-atom of the heterocycle of formula VII, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C6)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
provided that the heterocycle of formula VII carries precisely one group X;
in the form of the free base, their physiologically acceptable salts and possible enatiomers and diastereomers.

Another preferred embodiment of the present disclosure is a compound of formula VII as described above, wherein R4 is hydrogen or fluoro, and is preferably hydrogen.

Another preferred embodiment of the present disclosure is a compound of formula VII as described above, wherein
k is 0 or 1;
m is 0, 1 or 2;
p is 0 or 1;
n is 2, 3 or 4;
qis1;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8, and is preferably hydrogen or fluoro, and particularly preferably hydrogen;
R5 and R6 are independently selected from hydrogen, or fluoro;
wherein R7 and R8 are independently selected from hydrogen, (C1-C3)alkylcarbonyl, (C1-C3)alkyl, and phenyl(C1-C3)alkyl.

Another preferred embodiment of the present disclosure is a compound of formula VII as described above, wherein
k is 0;
m is 0 or 1;
n is 3;
p is 0;
q is 1;
R1 is hydrogen, or formyl,
R2 and R3 are independently selected from hydrogen, methoxy, chloro, amino, and acetylamino;
R4, R5 and R6 are all hydrogen.

Another embodiment of the present invention is a compound having formula VIII 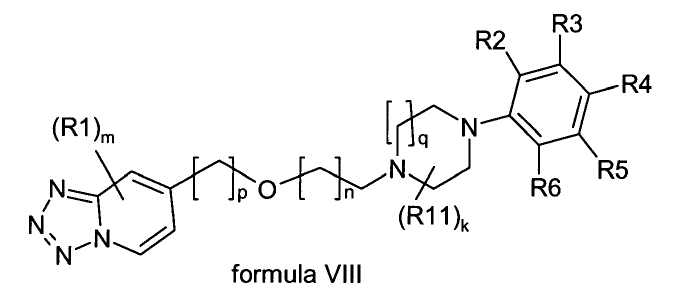 wherein:
R1, R2, R3, R4, R5, R6, R11, k, m, n, p and q are as defined for compounds of formula I, further above.
In a preferred embodiment,
k is 0,1 or 2;
m is 0, 1, 2 or 3;
n is 1-5;
p is 0, 1 or 2;
q is 1 or 2, and is preferably 1;
any R1 is bonded to a C-atom of the heterocycle of formula VIII, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C6)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
   provided that the heterocycle of formula VIII carries precisely one group X;
   in the form of the free base, their physiologically acceptable salts and possible enatiomers and diastereomers.

Another preferred embodiment of the present disclosure is a compound of formula

VII as described above, wherein R4 is hydrogen or fluoro, and is preferably hydrogen.

Another preferred embodiment of the present disclosure is a compound of formula VIII as described above, wherein
k is 0 or 1;
m is 0, 1 or 2;
p is 0 or 1;
n is 2, 3 or 4;
q is 1;
   each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
   R2 and R3 are independently selected from among hydrogen, fluoro, chloro, bromo, trifluoromethyl, (C1-C3)alkyloxy, (C1-C3)alkyl, -NR7R8, or R2 and R3 form together
   with the phenylring to which they are attached a dihydrobenzofurane, chromane, tetrahydrobenzoxepine or benzodioxole group;
   R4 is selected from hydrogen, (C1-C3)alkyloxy, hydroxy, chloro, fluoro, trifluoromethyl, or NR7R8, and is preferably hydrogen or fluoro, and particularly preferably hydrogen;
   R5 and R6 are independently selected from hydrogen, or fluoro;
   wherein R7 and R8 are independently selected from hydrogen, (C1-C3)alkylcarbonyl, (C1-C3)alkyl, and phenyl(C1-C3)alkyl.

Another preferred embodiment of the present disclosure is a compound of formula VIII as described above, wherein
k is 0;
m is 0 or 1;
n is 3;
p is 0;
q is 1;
R1 is hydrogen, or formyl,
R2 and R3 are independently selected from hydrogen, methoxy, chloro, amino, and acetylamino;
R4, R5 and R6 are all hydrogen.

Specific compounds in accordance with the present invention are
- 2-[4-[4-(2,3-Dichlorophenyl)piperazin-1-yl]butoxy]pyrazolo[1,5-a]pyridine
- 2-[4-[4-(2-Methoxyphenyl)piperazin-1-yl]butoxy]pyrazolo[1,5-a]pyridine
- 2-[4-[4-(2-Chlorophenyl)piperazin-1-yl]butoxy]pyrazolo[1,5-a]pyridine
- 2-[4-[4-(2,3-Dichlorophenyl)piperazin-1-yl]butoxy]pyrazolo[1,5-a]pyridin-3-carbaldehyde
- 2-[4-[4-(2-Methoxyphenyl)piperazin-1-yl]butoxy]pyrazolo[1,5-a]pyridin-3-carbaldehyde
- 2-[4-[4-(2-Chlorophenyl)piperazin-1-yl]butoxy]pyrazolo[1,5-a]pyridin-3-carbaldehyde
- 4-[4-[4-(2,3-Dichlorophenyl)piperazin-1-yl]butoxy]pyrazolo[1,5-a]pyridine
- 4-[4-[4-(2-Methoxyphenyl)piperazin-1-yl]butoxy]pyrazolo[1,5-a]pyridine
- 4-[4-[4-(2-Chlorophenyl)piperazin-1-yl]butoxy]pyrazolo[1,5-a]pyridine
- 4-[4-[4-(2,3-Dichlorophenyl)piperazin-1-yl]butoxy]pyrazolo[1,5-a]pyridin-3-carbaldehyde
- 4-[4-[4-(2-Methoxyphenyl)piperazin-1-yl]butoxy]pyrazolo[1,5-a]pyridin-3-carbaldehyde
- 4-[4-[4-(2-Chlorophenyl)piperazin-1-yl]butoxy]pyrazolo[1,5-a]pyridin-3-carbaldehyde
- 5-[3-[4-(2,3-Dichlorophenyl)piperazin-1-yl]propoxymethyl]pyrazolo[1,5-a]pyridine
- 5-[3-[4-(2,3-Dichlorophenyl)piperazin-1-yl]propoxymethyl]pyrazolo[1,5-a]pyridin-3-carbaldehyde
- 5-[4-[4-(2,3-Dichlorophenyl)piperazin-1-yl]butoxy]pyrazolo[1,5-a]pyridine
- 5-[4-[4-(2-Methoxyphenyl)piperazin-1-yl]butoxy]pyrazolo[1,5-a]pyridine
- 5-[4-[4-(2-Chlorophenyl)piperazin-1-yl]butoxy]pyrazolo[1,5-a]pyridine
- 5-[4-[4-(4-Methoxyphenyl)piperazin-1-yl]butoxy]pyrazolo[1,5-a]pyridine
- 5-[4-[4-(4-Chlorophenyl)piperazin-1-yl]butoxy]pyrazolo[1,5-a]pyridine
- 5-[4-[4-(4-Hydroxyphenyl)piperazin-1-yl]butoxy]pyrazolo[1,5-a]pyridine
- 5-[4-[4-(2,3-Dichlorophenyl)piperazin-1-yl]butoxy]pyrazolo[1,5-a]pyridin-3-carbaldeyhde
- 5-[4-[4-(2-Methoxyphenyl)piperazin-1-yl]butoxy]pyrazolo[1,5-a]pyridin-3-carbaldeyhde
- 5-[4-[4-(2-Chlorophenyl)piperazin-1-yl]butoxy]pyrazolo[1,5-a]pyridin-3-carbaldeyhde
- 5-[4-[4-(4-Methoxyphenyl)piperazin-1-yl]butoxy]pyrazolo[1,5-a]pyridin-3-carbaldeyhde
- 5-[4-[4-(4-Chlorophenyl)piperazin-1-yl]butoxy]pyrazolo[1,5-a]pyridin-3-carbaldeyhde
- 5-[4-[4-(4-Hydroxyphenyl)piperazin-1-yl]butoxy]pyrazolo[1,5-a]pyridin-3-carbaldeyhde
- 1-[5-[4-[4-(2,3-Dichlorophenyl)piperazin-1-yl]butoxy]pyrazolo[1,5-a]pyridin-3-yl]ethanone
- 1-[5-[4-[4-(2-Methoxyphenyl)piperazin-1 -yl]butoxy]pyrazolo[1,5-a]pyridin-3-yl]ethanone
- 1-[5-[4-[4-(2-Chlorophenyl)piperazin-1-yl]butoxy]pyrazolo[1,5-a]pyridin-3-yl]ethanone
- *(s-trans)*-5-[4-[4-(2,3-Dichlorophenyl)piperazin-1-yl]butoxy]pyrazolo[1,5-a]pyridin-3-carbaldehyde oxime
- *(s-trans)*-5-[4-[4-(2-Methoxyphenyl)piperazin-1-yl]butoxy]pyrazolo[1,5-a]pyridin-3-carbaldehyde oxime
- *(s-trans)*-5-[4-[4-(2-Chlorophenyl)piperazin-1-yl]butoxy]pyrazolo[1,5-a]pyridin-3-carbaldehyde oxime
- *(s-cis)*-5-[4-[4-(2,3-Dichlorophenyl)piperazin-1-yl]butoxy]pyrazolo[1,5-a]pyridin-3-carbaldehyde oxime
- *(s-cis)*-5-[4-[4-(2-Methoxyphenyl)piperazin-1-yl]butoxy]pyrazolo[1,5-a]pyridin-3-carbaldehyde oxime
- *(s-cis)*-5-[4-[4-(2-Chlorophenyl)piperazin-1-yl]butoxy]pyrazolo[1,5-a]pyridin-3-carbaldehyde oxime
- 5-[4-[4-(2,3-Dichlorophenyl)piperazin-1-yl]butoxy]pyrazolo[1,5-a]pyridin-3-carbonitrile
- 5-[4-[4-(2-Methoxyphenyl)piperazin-1-yl]butoxy]pyrazolo[1,5-a]pyridin-3-carbonitrile
- 5-[4-[4-(2-Chlorophenyl)piperazin-1-yl]butoxy]pyrazolo[1,5-a]pyridin-3-carbonitrile
- 5-[4-[4-(2,3-Dichlorophenyl)piperazin-1-yl]butoxy]pyrazolo[1,5-a]pyridin-3-carboxylic acid ethyl ester
- 5-[4-[4-(2-Methoxyphenyl)piperazin-1-yl]butoxy]pyrazolo[1,5-a]pyridin-3-carboxylic acid ethyl ester
- 5-[4-[4-(2-Chlorophenyl)piperazin-1-yl]butoxy]pyrazolo[1,5-a]pyridin-3-carboxylic acid ethyl ester
- 3-Bromo-5-[4-[4-(2,3-dichlorophenyl)piperazin-1-yl]butoxy]pyrazolo[1,5-a]pyridine
- 3-Bromo-5-[4-[4-(2-methoxyphenyl)piperazin-1-yl]butoxy]pyrazolo[1,5-a]pyridine
- 3-Bromo-5-[4-[4-(2-chlorophenyl)piperazin-1-yl]butoxy]pyrazolo[1,5-a]pyridine
- 3-Chloro-5-[4-[4-(2,3-dichlorophenyl)piperazin-1-yl]butoxy]pyrazolo[1,5-a]pyridine
- 3-Chloro-5-[4-[4-(2-methoxyphenyl)piperazin-1-yl]butoxy]pyrazolo[1,5-a]pyridine
- 3-Chloro-5-[4-[4-(2-chlorophenyl)piperazin-1-yl]butoxy]pyrazolo[1,5-a]pyridine
- 5-[4-[4-(2,3-Dichlorophenyl)piperazin-1-yl]butoxy]-3-iodopyrazolo[1,5-a]pyridine
- 3-Iodo-5-[4-[4-(2-methoxyphenyl)piperazin-1-yl]butoxy]pyrazolo[1,5-a]pyridine
- 5-[4-[4-(2-Chlorophenyl)piperazin-1-yl]butoxy]-3-iodopyrazolo[1,5-a]pyridine
- 6-[4-[4-(2,3-Dichlorophenyl)piperazin-1-yl]butoxy]pyrazolo[1,5-a]pyridine
- 6-[4-[4-(2-Methoxyphenyl)piperazin-1-yl]butoxy]pyrazolo[1,5-a]pyridine
- 6-[4-[4-(2-Chlorophenyl)piperazin-1-yl]butoxy]pyrazolo[1,5-a]pyridine
- 6-[4-[4-(2,3-Dichlorophenyl)piperazin-1-yl]butoxy]pyrazolo[1,5-a]pyridin-3-carbaldehyde
- 6-[4-[4-(2-Methoxyphenyl)piperazin-1-yl]butoxy]pyrazolo[1,5-a]pyridin-3-carbaldehyde
- 6-[4-[4-(2-Chlorophenyl)piperazin-1-yl]butoxy]pyrazolo[1,5-a]pyridin-3-carbaldehyde
- 7-[4-[4-(2,3-Dichlorophenyl)piperazin-1-yl]butoxy]pyrazolo[1,5-a]pyridine
- 7-[4-[4-(2-Methoxyphenyl)piperazin-1-yl]butoxy]pyrazolo[1,5-a]pyridine
- 7-[4-[4-(2-Chlorophenyl)piperazin-1-yl]butoxy]pyrazolo[1,5-a]pyridine
- 7-[4-[4-(2,3-Dichlorophenyl)piperazin-1-yl]butoxy]pyrazolo[1,5-a]pyridin-3-carbaldehyde
- 7-[4-[4-(2-Methoxyphenyl)piperazin-1-yl]butoxy]pyrazolo[1,5-a]pyridin-3-carbaldehyde
- 7-[4-[4-(2-Chlorophenyl)piperazin-1-yl]butoxy]pyrazolo[1,5-a]pyridin-3-carbaldehyde
- 7-[4-[4-(2,3-Dichlorophenyl)piperazin-1-yl]butoxy]tetrazolo[1,5-a]pyridine
- 7-[4-[4-(2-Methoxyphenyl)piperazin-1-yl]butoxy]tetrazolo[1,5-a]pyridine
- 7-[4-[4-(2-Chloroyphenyl)piperazin-1-yl]butoxy]tetrazolo[1,5-a]pyridine

Other examples of compounds according to the present inventions are:
- 5-[4-[4-(3-Trifluoromethylphenyl)piperazin-1-yl]butoxy]pyrazolo[1,5-a]pyridine
- 5-[4-[4-(3-Nitrophenyl)piperazin-1-yl]butoxy]pyrazolo[1,5-a]pyridine
- 5-[4-[4-(3-Aminophenyl)piperazin-1-yl]butoxy]pyrazolo[1,5-a]pyridine
- *N*-[3-[4-[4-(Pyrazolo[1,5-a]pyridin-5-yloxy)butyl]piperazin-1-yl]phenyl]acetamide
- 5-[4-[4-(3-Trifluoromethylphenyl)piperazin-1-yl]butoxy]pyrazolo[1,5-a]pyridin-3-carbaldehyde
- 5-[4-[4-(3-Nitrophenyl)piperazin-1-yl]butoxy]pyrazolo[1,5-a]pyridin-3-carbaldehyde
- *N*-[3-[4-[4-(3-Formylpyrazolo[1,5-a]pyridin-5-yloxy)butyl]piperazin-1-yl]phenyl]acetamide
- 5-[4-[4-(2,3-Dichlorophenyl)piperazin-1-yl]butoxy]-3-hydroxymethylpyrazolo[1,5-a]pyridine
- 3-Hydroxymethyl-5-[4-[4-(2-methoxyphenyl)piperazin-1-yl]butoxy]methylpyrazolo[1,5-a]pyridine
- 5-[4-[4-(2-Chlorophenyl)piperazin-1-yl]butoxy]-3-hydroxymethylpyrazolo[1,5-a]pyridine
- 5-[4-[4-(2,3-Dichlorophenyl)piperazin-1-yl]butoxy]-3-methylpyrazolo[1,5-a]pyridine
- 5-[4-[4-(2-Methoxyphenyl)piperazin-1-yl]butoxy]-3-methylpyrazolo[1,5-a]pyridine
- 5-[4-[4-(2-Chlorophenyl)piperazin-1-yl]butoxy]-3-methylpyrazolo[1,5-a]pyridine
- 3-Aminomethyl-5-[4-[4-(2,3-dichlorophenyl)piperazin-1-yl]butoxy]pyrazolo[1,5-a]pyridine
- 3-Aminomethyl-5-[4-[4-(2-methoxyphenyl)piperazin-1-yl]butoxy]pyrazolo[1,5-a]pyridine
- 3-Aminomethyl-5-[4-[4-(2-chlorophenyl)piperazin-1-yl]butoxy]pyrazolo[1,5-a]pyridine
- *N*-[5-[4-[4-(2,3-Dichlorophenyl)piperazin-1-yl]butoxy]pyrazolo[1,5-a]pyridin-3-ylmethyl]acetamide
- *N*-[5-[4-[4-(2-Methoxyphenyl)piperazin-1-yl]butoxy]pyrazolo[1,5-a]pyridin-3-ylmethyl]acetamide
- *N*-[5-[4-[4-(2-Chlorophenyl)piperazin-1-yl]butoxy]pyrazolo[1,5-a]pyridin-3-ylmethyl]acetamide
- 5-[4-(4-(2,3-Dihydrobenzofuran-7-yl)piperazin-1-yl)butoxy]pyrazolo[1,5-a]pyridine
- 5-[4-(4-(Chroman-8-yl)piperazin-1-yl)butoxy]pyrazolo[1,5-a]pyridine
- 5-[4-(4-(2,3-Dichlorophenyl)-1,4-diazepan-1-yl)butoxy]pyrazolo[1,5-a]pyridine
- 5-[4-(4-(2-Methoxyphenyl)-1,4-diazepan-1-yl)butoxy]pyrazolo[1,5-a]pyridine
- 5-[4-(4-(2-Chlorophenyl)-1,4-diazepan-1-yl)butoxy]pyrazolo[1,5-a]pyridine
- 5-[4-(4-(2,3-Dihydrobenzofuran-7-yl)-1,4-diazepan-1-yl)butoxy]pyrazolo[1,5-a]pyridine
- 5-[4-(4-(Chroman-8-yl)-1,4-diazepan-1-yl)butoxy]pyrazolo[1,5-a]pyridine
- 5-[4-(4-(2,3-Dihydrobenzofuran-7-yl)piperazin-1-yl)butoxy]pyrazolo[1,5-a]pyridin-3-carbaldehyde
- 5-[4-(4-(Chroman-8-yl)piperazin-1-yl)butoxy]pyrazolo[1,5-a]pyridin-3-carbaldehyde
- 5-[4-(4-(2,3-Dichlorophenyl)-1,4-diazepan-1-yl)butoxy]pyrazolo[1,5-a]pyridin-3-carbaldehyde
- 5-[4-(4-(2-Methoxyphenyl)-1,4-diazepan-1-yl)butoxy]pyrazolo[1,5-a]pyridin-3-carbaldehyde
- 5-[4-(4-(2-Chlorophenyl)-1,4-diazepan-1-yl)butoxy]pyrazolo[1,5-a]pyridin-3-carbaldehyde
- 5-[4-(4-(2,3-Dihydrobenzofuran-7-yl)-1,4-diazepan-1-yl)butoxy]pyrazolo[1,5-a]pyridin-3-carbaldehyde
- 5-[4-(4-(Chroman-8-yl)-1,4-diazepan-1-yl)butoxy]pyrazolo[1,5-a]pyridin-3-carbaldehyde
- 7-[4-[4-(2,3-Dichlorophenyl)piperazin-1-yl]butoxy]indolizine
- 7-[4-[4-(2-Methoxyphenyl)piperazin-1-yl]butoxy]indolizine
- 7-[4-[4-(2-Chloroyphenyl)piperazin-1-yl]butoxy]indolizine
- 6-[4-[4-(2,3-Dichlorophenyl)piperazin-1-yl]butoxy]imidazo[1,2-a]pyridine
- 6-[4-[4-(2-Methoxyphenyl)piperazin-1-yl]butoxy]imidazo[1,2-a]pyridine
- 6-[4-[4-(2-Chlorophenyl)piperazin-1-yl]butoxy]imidazo[1,2-a]pyridine
- 8-[4-[4-(2,3-Dichlorophenyl)piperazin-1-yl]butoxy]imidazo[1,2-a]pyridine
- 8-[4-[4-(2-Methoxyphenyl)piperazin-1-yl]butoxy]imidazo[1,2-a]pyridine
- 8-[4-[4-(2-Chlorophenyl)piperazin-1-yl]butoxy]imidazo[1,2-a]pyridine

The compounds of the present invention can be prepared as follows:

A compound of formula XI is reacted with an activated alkylene having the formula XII to give a compound of formula XIII 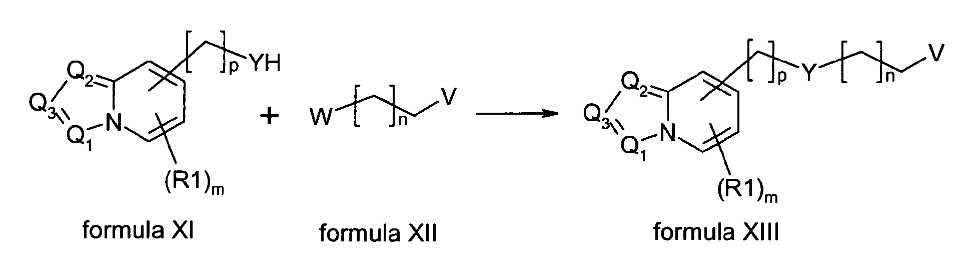 which compound of formula XIII, optionally after (partial) purification, is then combined with a compound of formula XIV 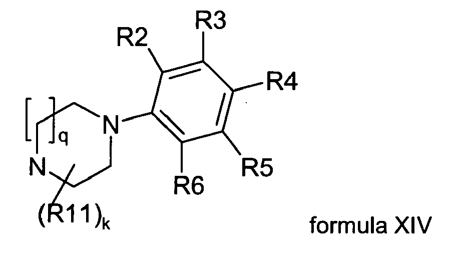 to give a compound of formula XV 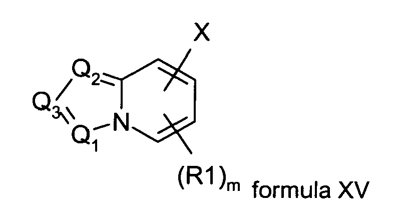 wherein in anyone of formulas XI, XII, XIII, XIV, and XV
R1, Q1, Q2, X, Y, n, R11, q, k, R2, R3, R4, R5, and R6 are as defined further above and in the claims for compounds of the formulas I, IIa, IIb, IIc, IId, III, IV, V, VI, VII or VIII; and
W and V are activating groups, which may be the same or different from each other;
and optionally adding to the compound of formula XV, optionally after (partial) purification, one or more additional groups R1 to give a compound of anyone of formula I, IIa, IIb, IIc, IId, III, IV, V, VI, VII or VIII as described further above.

In the general method described above, the activating group is selected from among bromo, chloro, mesylate, triflate or tosylate.

In the production method described above all R1 groups can be present in the starting material of the general formula XI. Alternatively, one or more R1 groups may be added to a compound of formula XVa in which one or more hydrogen atom(s) is/are then replaced by additional R1 group(s) to give a compound of I, IIa, IIb, IIc, IId, III, IV, V, VI, VII or VIII as described further above and in the claims. This synthesis takes place by reaction of a compound of formula XVa with an activated precursor to get a compound of formula XVI as described as follows: 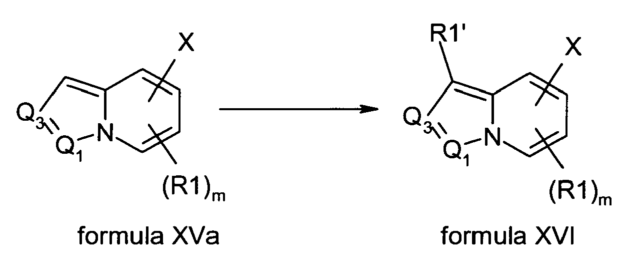 wherein in formulas XVa and XVI
R1' is selected from among bromo, chloro, iodine, formyl, hydroxymethyl, alkyl, oxime, cyano, aminomethyl, acylaminomethyl; and
R1, Q1, Q3, X and m are as defined above.

A preferred embodiment relates to a method of producing a compound according to the present invention, comprising the synthesis of a compound of formula XVI as described above, wherein R1' is selected from among bromo, chloro, iodine and formyl and wherein R1' is introduced by the reaction of a compound of formula XVa with an activated precursor to get a compound of formula XVI. Examples of activated precursors are *N-*halogensuccinimide, hypohalogenic acids, sodium halogenites and phosphorousoxychloride in *N*,*N*-dimethylformamide or other well established formylation reactants and are preferably *N*-chlorosuccinimide, *N-*bromosuccinimide, *N-*iodosuccinimide and phosphorousoxychloride in *N,N*-dimethylformamide.

Alternatively, the group R1 can be attached to the heterocyclic ring system by a two-step sequence starting with the introduction of a formyl group to give a compound of formula XVIa 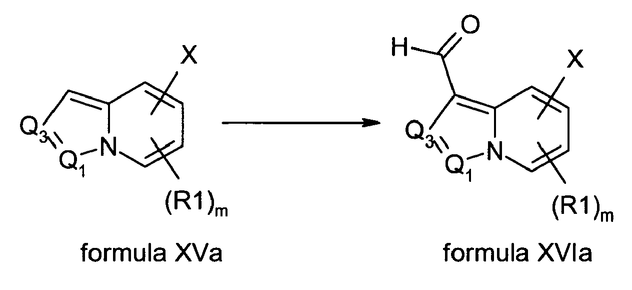 and subsequently transforming the formyl group of formula XVIa to give a compound of formula XVII as follows: 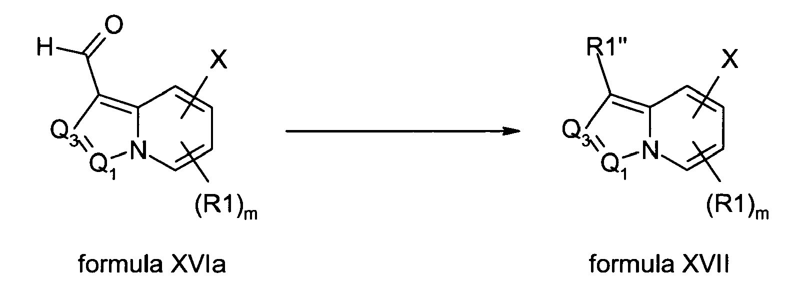 wherein
the compound of formula XVII is comprised by formula I, IIa, IIB, IIc, IId, III, IV, V, VI, VII and/or VIII, and wherein in formula XVII
R1" is selected from among hydroxymethyl, alkyl, oxime, cyano, aminomethyl and acylaminomethyl,
and Q1, Q3, X and m are as defined further above and in the claims.

The transfer of the formyl group to the group R1 " comprises condensation reactions, the reduction of the formyl group or a combination of both, condensation and reduction reactions.

The production of the individual compounds of the present invention is described in more detail in the experimental part of the present application.

The compounds of the present invention are useful medicines, and may be used for the treatment of various diseases of the CNS system.

Due to their remarkable affinity to the dopaminergic D2 receptor, as well as due to their modulating potency at the serotonergic 5-HT2 and 5-HT1a receptors, the present compounds in one mbodiment of the present invention may be of use for the production of a medicament for the treatment of a variety of diseases such as like a psychotic disease including manic phases of bipolar disorder, acute idiopathic psychotic illnesses, psychoses associated with other diseases, drug-induced psychoses, and particularly schizophrenia; attention deficit hyperactivity disorder (ADHD); autism; bipolar disorder; cognitive impairment; idiopathic or drug-induced movement disorders such as akinesia and dyskinesia; Parkinson's disease; mood disorders including major depressive disorders, substance-induced mood disorders or other forms of depression; anxiety disorders including panic attack, social phobia, or generalized anxiety disorders; obsessive-compulsive disorders; stress-related disorders; addiction disorders; sleep disorders; sexual dysfunction; amnesic and/or cognitive disorders, especially dementia; eating disorders including anorexia and bulemia; pain; and neurodegenerative diseases including Chorea Huntington and multiple sclerosis.

Another embodiment of the present disclosure is a method of treating a subject having a disease selected from a psychotic disease including manic phases of bipolar disorder, acute idiopathic psychotic illnesses, psychoses associated with other diseases, drug-induced psychoses, and particularly schizophrenia; attention deficit disorder; autism; bipolar disorder; cognitive impairment; idiopathic or drug-induced movement disorders such as dyskinesia; Parkinson's disease; or depression by administering a compound as described herein, and specifically in the claims. According to one aspect of the invention, the subject to be treated with the presently disclosed compounds have been determined to be in need of a treatment of one or more of the above diseases based on a prior diagnosis of said disease or said diseases.

Prior to the administration to the patient, the presently disclosed compounds may be added to a pharmaceutically acceptable excipient or carrier. One aspect of the present invention is thus a pharmaceutical composition comprising a compound as described in this specification and in the claims, and a pharmaceutical acceptable carrier. Hereinafter, some formulating methods and kinds of excipients will be described, but the present invention is not limited to them.
The pharmaceutical composition of the present invention can be administered to a mammalian subject such as domestic animals or human beings via various routes.
The methods of administration which may easily be expected include oral, bukkal, sublingual, nasal, pulmonal, and rectal administration; intravenous, intramuscular, subcutaneous, and intracerebroventricular injections, wherein oral delivery is preferred.

The oral administration may be performed by providing the compounds of the present invention in the form of a tablet, a capsule, a drage', a powder, a granulate, or in form of a liquid or a semi-solid.

A compound of formula (I), (IIa), (IIb), (IIc), (IId), (III), (IV), (V), (VI), (VII) or (VIII), an isomer thereof or a pharmaceutically acceptable salt thereof according to the present invention can be prepared as a pharmaceutical composition containing pharmaceutically acceptable carriers, adjuvants, diluents and the like.

For instance, the compounds of the present invention can be dissolved in oils, propylene glycol or other solvents which are commonly used to produce an injection. Suitable examples of the carriers include, but not limited to, physiological saline, polyethylene glycol, ethanol, vegetable oils, isopropyl myristate, etc. The compounds of the present invention may be formulated into injections by dissolving, suspending or emulsifying in water-soluble solvent such as saline and 5% dextrose, or in water-insoluble solvents such as vegetable oils, synthetic fatty acid glyceride, higher fatty acid esters and propylene glycol. The formulations of the invention may include any of conventional additives such as dissolving agents, isotonic agents, suspending agents, emulsifiers, stabilizers and preservatives.

Oral formulations may comprise e.g. sustained release agents, disintegrants, fillers, lubricants, stabilizers, antioxidants, stabilizers, flavours, dispersion agents, electrolytes, buffers or conservation agents. Suitable excipients and formulations are known to those skilled in the art and are disclosed in standard monographs such as like Remington ("The science and practice of pharmacy", Lippincott, Williams &

Wilkins, 2000). Typical sustained release agents are for example those that swell upon contact with water such as polyvinylpyrrolidone, hydroxyethylcellulose, hydroxypropylcellulose, other cellulose ethers, starch, pregelatinised starch, polymethacrylate, polyvinylacetate, microcrystalline cellulose, dextranes or mixtures thereof. The pharmaceutical composition of the present invention can also contain disintegrants, such as pregelatinised starch, sodium starch glycolate, microcrystalline cellulose, carboxymethylcellulose sodium (CMC-Na), cross-linked CMC-Na, low-substituted hydroxypropylcellulose or mixtures thereof. The pharmaceutical compositions according to the invention can further contain fillers and binders such as microcrystalline cellulose, powdered cellulose, lactose (anhydrous or monohydrate), compressible sugar, starch (e.g. corn starch or potato starch), pregelatinised starch, fructose, sucrose, dextrose, dextranes, other sugars such as mannitol, maltitol, sorbitol, lactitol and saccharose, siliconised microcrystalline cellulose, calcium hydrogen phosphate, calcium hydrogen phosphate dihydrate, dicalciumphosphate dihydrate, tricalciumphophate, calcium lactate or mixtures thereof. The oral composition according to the invention can also comprise lubricants, antiadherents and/or glidants, such as stearic acid, magnesium stearate, calcium stearate, sodium lauryl sulphate, hydrogenated vegetable oil, hydrogenated castor oil, sodium stearyl fumarate, macrogols, glycerol dibehenate, talc, corn starch, silicone dioxide or mixtures thereof.

The preferable dose level of the compounds according to the present invention depends upon a variety of factors including the condition and body weight of the patient, severity of the particular disease, dosage form, and route and period of administration, but may appropriately be chosen by those skilled in the art. The compounds of the present invention may be administered in an amount ranging from 0.001 to 10 mg/kg of body weight per day, and more preferably from 0.03 to 1 mg/kg of body weight per day. Individual doses may range from about 0.1 to 500 mg of active ingredient per day, preferably from about 1 to 100 mg/day, and most preferably of between about 5 and 100 mg/day. Doses may be administered once a day, or several times a day with each divided portions.

Another aspect of the present invention is a Kit comprising a medicine or a pharmaceutical composition as described above, and instructions for its use.

The medicine according to the present invention may comprise one of the presently disclosed compounds as "stand alone" treatment of a psychological illness such as e.g. schizophrenia or bipolar disorder.

In one embodiment, a presently disclosed compound may be administered together with other useful drugs in a combination therapy. For example, a compound according to the present invention may be combined with an antidepressant to treat a psychoses associated with depressions, e.g. bipolar disorder. Likewise a compound of the present invention may be combined with a cognition enhancer. In combination therapies the two or more active principles may be provided via the same formulation or as a "kit of parts", i.e. in separate galenic units. Also, the two or more active principles may be administered to the patient at the same time or subsequently, e.g. in an interval therapy.

### Definitions:

"Alkyl" includes monovalent saturated aliphatic hydrocarbyl groups. The hydrocarbon chain may be either straight-chained or branched. "Alkyl" has preferably 1-15 carbon atoms ("C1-C15 alkyl"), more preferably 1-10 carbon atoms ("C1-C10 alkyl"), even more preferably 1-8 carbon atoms ("C1-C8 alkyl") or 1-6 carbon atoms ("C1-C6 alkyl"), and in some instances even more preferably 1-5 carbon atoms ("C1-C5 alkyl"), 1-4 carbon atoms ("C1-C4 alkyl"), or only 1-3 carbon atoms ("C1-C3 alkyl"). This term is exemplified by groups such as methyl, ethyl, n-propyl, isopropyl, n-butyl, iso-butyl, tert-butyl, t-amyl, and the like.

"Alkylamino" includes the group -NHR', wherein R' is alkyl group as defined herein. "Alkylsulfonyl" includes a radical-S(O)₂R, wherein R is an alkyl group as defined herein. Representative examples include, but are not limited to, methanesulfonyl, ethylsulfonyl, propylsulfonyl, butylsulfonyl and the like.

"Alkylthio" includes a radical-S-R wherein R is an alkyl group as defined herein that may be optionally substituted as defined herein. Preferably, "alkylthio" is a C1-C6-alkyl-S-group. Representative examples include, but are not limited to, methylthio, ethylthio, propylthio, butylthio, and the like.

"Alkylaminosulfonyl" includes the group -SO₂-NH-Alkyl, wherein "alkyl" is preferably selected from the groups specified in the definition of "alkyl" further above.

Most preferably "alkyl" in "alkylaminosulfonyl" is a C1-C6-alkylgroup. Examples of "alkylaminosulfonyl" are e.g. methylaminosulfonyl, ethylaminosulfonyl or butylaminosulfonyl.

"Dialkylaminosulfonyl" includes the group -SO₂-N-dialkyl, wherein "alkyl" is preferably selected from the groups specified in the definition of "alkyl" further above.

Most preferably "alkyl" in "dialkylaminosulfonyl" is a C1-C6-alkylgroup. Examples of "alkylaminosulfonyl" are e.g. N,N-dimethylaminosulfonyl, N,N-methylethylaminosulfonyl or N,N-methylbutylaminosulfonyl.

"Alkylsulfonylamino" includes the group -NH-SO₂-Alkyl, wherein alkyl is preferably selected from the groups specified in the definition of "alkyl" further above. Most preferably "alkyl" in "alkylsulfonylamino " is a C1-C6-alkylgroup, such as e.g. methanesulfonylamino.

"Alkylcarbonyl" includes the group -C(O)-alkyl, wherein alkyl is preferably selected from the groups specified in the definition of "alkyl" further above. "Alkylcarbonyl" is particularly preferably -C(O)-C1-C6-Alkyl, and most preferably acetyl, propionyl oder butyryl.

"Alkylaminocarbonyl" includes the groups -C(O)-NH-alkyl wherein "alkyl" is preferably selected from the groups specified in the definition of "alkyl" further above. "Alkylaminocarbonyl" is particularly preferably -C(O)-NH-(C1-C6)Alkyl

"Dialkylaminocarbonyl" includes the group -CO-N-dialkyl, wherein "alkyl" is preferably selected from the groups specified in the definition of "alkyl" further above. "Dialkylaminocarbonyl" is particularly preferably -C(O)-N-di(C1-C6)alkyl

"Alkyloxy" or "alkoxy" includes the group -OR wherein R is "alkyl" as defined further above. Particular alkyloxy groups include, by way of example, methoxy, ethoxy, n-propoxy, isopropoxy, n-butoxy, tert-butoxy, sec-butoxy, n-pentoxy, 1,2-dimethylbutoxy, and the like.

"Alkyloxyalkyloxy" refers to the group -OROR', wherein R and R' are the same or different "alkyl" groups as defined further above.

"Alkyloxyalkyloxyalkyl" refers to the group -ROR'OR", wherein R, R' and R" are the same or different "alkyl" groups as defined further above.

"Alkyloxyalkyamino" refers to the group -NH(ROR'), wherein R and R' are the same or different "alkyl" groups as defined further above.

"N-Alkyloxyalky-N-alkylamino" refers to the group -NR(R'OR"), wherein R, R' and R" are the same or different "alkyl" groups as defined further above.

"Alkyloxycarbonyl" refer to the radical -C(=O)-O-R, wherein R is an alkyl group as defined herein. Bevorzugt ist "Alkyloxycarbonyl" eine (C1-C6-Alkyl)oxycarbonylgruppe.

"Alkenyl" includes monovalent olefinically unsaturated hydrocarbyl groups being straight-chained or branched and having at least 1 double bond. "Alkenyl" has preferably 2-15 carbon atoms ("C2-C15 alkenyl"), more preferably 2-10 carbon atoms ("C2-C10 alkenyl"), even more preferably 2-8 carbon atoms ("C2-C8 alkenyl") or 2-6 carbon atoms ("C2-C6 alkenyl"), and in some instances even more preferably 2-5 carbon atoms ("C1-C5 alkenyl"), 2-4 carbon atoms ("C2-C4 alkenyl"), or only 2-3 carbon atoms ("C2-C3 alkenyl"). Particular alkenyl groups include ethenyl (-CH=CH2), n-propenyl (-CH2CH=CH2), isopropenyl (C (CH3) =CH2), and the like. A preferred "alkenyl" group is ethenyl (vinyl).

"Alkynyl" includes acetylenically unsaturated hydrocarbyl groups being straight-chained or branched and having at least 1 triple bond. "Alkynyl" has preferably 2-15 carbon atoms ("C2-C15 alkynyl"), more preferably 2-10 carbon atoms ("C2-C10 alkynyl"), even more preferably 2-8 carbon atoms ("C2-C8 alkynyl") or 2-6 carbon atoms ("C2-C6 alkynyl"), and in some instances even more preferably 2-5 carbon atoms ("C1-C5 alkynyl"), 2-4 carbon atoms ("C2-C4 alkynyl"), or only 2-3 carbon atoms ("C2-C3 alkynyl"). A preferred alkynyl group is ethynyl (acetylenyl).

"Dialkylamino" includes the group -NR'R", wherein R' and R" are alkyl group as defined herein.

"Amino" refers to the radical-NH₂.

"Aryl" refers to an aromatic hydrocarbyl radical. Examples of "aryl" radicals are phenyl, naphthyl, indenyl, azulenyl, fluorine or anthracene, wherein phenyl is preferred.

"Carboxy" refers to the radical -C(=O)OH.

"Cycloalkyl" refers to cyclic saturated aliphatic hydrocarbyl groups. The numbers of C-atoms referenced in connection with a given cycloalkyl group corresponds to the number of ring forming carbon atoms, e.g. "C3-C6 cycloalkyl" refers to a cycloalkyl with between three and six ring-forming C atoms. Examples of "cycloalkyl" are C3-C8 cycloalkyls, C3-C7 cycloalkyls, or more specifically C3-C6 cycloalkyls such as e.g. cyclopropyl, cyclobutyl, cyclopentyl, cyclohexyl etc. If indicated, a

"cycloalkyl" group may be unsubstituted or substituted with e.g. hydroxyl, alkyloxy, halogen, phenyl, amino, a group NR7R8 or with one or more alkyl groups, e.g. with C1-C6 alkyl groups, preferably with C1-C3 alkyl groups, particularly preferably with methyl groups. If a "cycloalkyl" carries more than one substituent, e.g. one or more alkyl substituent these substituents may be attached to the same or to different ring-forming carbon atoms.

"Cycloalkyloxy" refers to the group -OR, wherein R is "cycloalkyl" group as defined further above.

"Cycloalkylamino" refers to the group -NHR, wherein R is "cycloalkyl" group as defined further above.

"N-Cycloalkylamino-N-alkylamino" refers to the group -NRR', wherein R is the same or different "alkyl" group as defined further above and R' is "cycloalkyl" group as defined further above.

"Cyano" refers to the radical -C=N.

"Formyl" refers to the group -C(=O)H

"Halo" or "halogen" refers to fluoro, chloro, bromo and iodo. Preferred halo groups are either fluoro or chloro.

"Haloalkyl" includes an "alkyl" group as defined further above which is substituted with one or more halogens which may be the same, e.g. in trifluoromethyl or pentafluoroethyl, or which may be different.

"Heteroaryl" refers to aromatic ring system containing at least one heteroatom such as O, S or N. Examples of heteroaryl radicals are furanyl, thienyl, pyrollyl, thiazolyl, oxazolyl, imidazolyl, pyrazolyl, isoxazolyl, isothiazolyl, triazolyl, oxadiazolyl, thiadiazolyl, pyranyl, pyridinyl, pyridazinyl, pyrimidinyl, pyrazinyl, triazinyl, tetrazinyl, indolinyl, indolyl, isoindolyl, benzofuranyl, benzothiophenyl, benzoimidazolyl, benzthiazolyl, purinyl, quinazolinyl, quinolinyl, isoquinolinyl, quinolizinyl, pteridinyl, carbazolyl, wherein one-ring sytems, and in particular pyridinyl, and imidazolyl are preferred.

"Heteroarylcarbonyl" refers to the group -CO-heteroaryl.

"Hydroxy" refers to the radical -OH.

"Hydroxyalkyl" includes an "alkyl" group as defined further above which is substituted with one or more hydroxy groups.

"Nitro" refers to the radical-NO₂.

"Oxime" refers to the group -CH=N-OH.

"Phenyl" is the aromatic radical -C6H5. Whether a phenyl group is substituted with one or more substituents, is specified throughout this specification and the claims.

"Phenylalkyl" comprises the group -alkyl-phenyl, wherein "phenyl" and "alkyl" have the meaning as defined further above. Examples of phenylalkyl groups are phenylethyl and benzyl, wherein benzyl is a particularly preferred phenylalkyl group.

"Phenylalkyloxy" comprises the group -O-alkyl-phenyl, wherein "phenyl" and "alkyl" have the meaning as defined further above. Examples of phenylalkyloxy groups are phenylethyloxy and benzyloxy.

"Phenoxy" comprises the group-O-phenyl, wherein phenyl" has the meaning as defined further above

"Phenylcarbonyl" is -C(O)-phenyl, wherein phenyl" has the meaning as defined further above

"Phenylalkylcarbonyl" is -C(O)-alkyl-phenyl, wherein "phenyl" and "alkyl" have the meaning as defined further above

"Phenylalkyloxycarbonyl" is the group -C(O)-O-alkyl-phenyl, wherein "phenyl" and "alkyl" have the meaning as defined further above

"Phenylsulfonyl" is -SO₂-phenyl, wherein phenyl" has the meaning as defined further above

"Sulfamoyl" includes the group -SO₂-NH₂.

"Sulfonylamino" includes the group -NH-SO₂H

"Trifluormethyl" refers to the group -CF₃.

Unless expressly specified otherwise, any "alkyl", "alkenyl", "alkynyl", "phenyl", or "heteroaryl" is meant to be unsubstituted. If any "alkyl", "alkenyl", "alkynyl", "phenyl", or "heteroaryl", is expressly stated to be substituted, this usually also refers to the respective "alkyl", "alkenyl", "alkynyl", "phenyl", or "heteroaryl" partial structures of more complex structures such as "alkyloxy", "alkylsulfonyl", "alkenyloxy", "phenoxy", "heteroaryloxy", etc.

"Pharmaceutically acceptable" means being approved by a regulatory agency of the Federal or a state government or being listed in the U. S. Pharmacopoeia or other generally recognized pharmacopoeia for use in animals, and more particularly in humans.

"Pharmaceutically acceptable salt" refers to a salt of a compound of the invention that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid,3- (4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4methylbicyclo [2.2.2]-oct-2-ene-1-carboxylic acid, glucoheptonic acid,3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, 2,2-dichloroacetate, adipate, alginate, ascorbate, aspartate, 2-acetamidobenzoate, caproate, caprate, camphorate, cyclamate, laurylsulfate, edisilate, esylate, isetionate, formate, galactarate, gentisate, gluceptate, glucuronate, oxoglutarate, hippurate, lactobionate, napadisilate, xinafoate, nicotinate, oleate, orotate, oxalate, palmitate, embonate, pidolate, p-aminosalicylate, sebacate, tannate, rhodanide, undecylenate, and the like; or (2) salts formed when an acidic proton present in the parent compound is replaced, such as e.g. ammonia, arginine, benethamine, benzathine, calcium, choline, deanol, diethanolamine, diethylammonium, ethanolamine, ethylendiamine, meglumine, hydrabamine, imidazole, lysine, magnesium, hydroxyethylmorpholine, piperazine, potassium, epolamine, sodium, trolamine, tromethamine or zinc.

"Pharmaceutically acceptable carrier" refers to a diluent, adjuvant, excipient or carrier with which a compound of the invention is administered.

"Preventing" or "prevention" refers to a reduction in risk of acquiring a disease or disorder (i. e., causing at least one of the clinical symptoms of the disease not to develop in a subject that may be exposed to or predisposed to the disease but does not yet experience or display symptoms of the disease).

"Subject" includes humans. The terms "human," "patient" and "subject" are used interchangeably herein.

"Therapeutically effective amount" means the amount of a compound that, when administered to a subject for treating a disease, is sufficient to effect such treatment for the disease. The "therapeutically effective amount" can vary depending on the compound, the disease and its severity, and the age, weight, etc., of the subject to be treated.

"Treating" or "treatment" of any disease or disorder refers, in one embodiment, to ameliorating the disease or disorder (i. e., arresting or reducing the development of the disease or at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically, (e. g., stabilization of a discernible symptom), physiologically, (e. g., stabilization of a physical parameter), or both. In yet another embodiment, "treating" or "treatment" refers to delaying or preventing the onset of the disease or disorder.

### Modes for carrying out the invention:

### 1. Synthesis of heteroarene components according to formula XI

### 1a) 5-Hydroxypyrazolo[1,5-a]pyridine (C4a), 4-hydroxypyrazolo[1,5-a]pyridine (C4b), 6-hydroxypyrazolo[1,5-a]pyridine (C4c), 7-hydroxypyrazolo[1,5-a]pyridine (C4d), 5-hydroxymethylpyrazolo[1,5-a]pyridine (C4e)

Synthesis of the heteroarene components was accomplished when methoxypyridines (C1) were aminated with O-(2,4-dinitrophenyl)hydroxylamine to get the N-amino pyridinium salts (C2) which were cyclicyzed with methyl priolate to the methyl pyrazolopyridine carboxylates C3. Acidic hydrolysis and decarboxylation achieved the pyrazolopyridines C4.

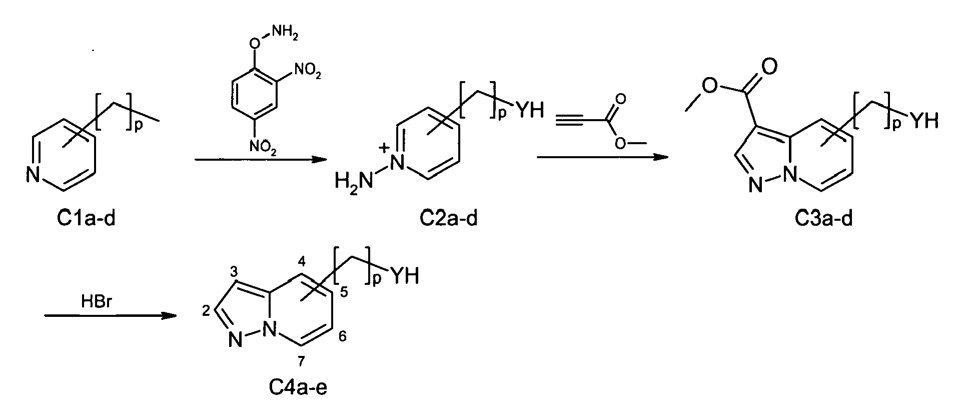

### N-Amino-(4-methoxy)pyridinium-2,4-dinitrophenolate (C2a)

A suspension of 11.4 ml (0.112 mol) 4-methoxypyridine and 24.6 g (0.124 mol) O-(2,4-dinitrophenyl)hydroxylamine in 371 ml dichloromethane was stirred for 20 hrs at room temperature. After addition of diethylether the precipitate was filtered off and dried in under reduced pressure.
Yield: 34 g (99 %) yellow solid.
Mp.: 140°C. MS (EI): m/z 184 (M+1)⁺ C₆H₃N₂O₅; 124 (M-1)⁺ C₆H₉N₂O. IR (KBr) v (cm⁻¹): 3096; 1597; 1552; 1535; 1508; 1256; 739; 714. ¹H NMR (DMSO-d₆, 360 MHz) δ (ppm): 4.05 (s, 3H, OCH₃); 6.36 (d, J = 9.8 Hz, 1 H, H-6 DNP); 7.52-7.54 (m, 2H, H-3/5 Pyr); 7.75 (br s, 2H, NH₂); 7.81 (dd, J = 9.8 Hz, 3.2 Hz, 1H, H-5 DNP); 8.60 (d, J = 3.2 Hz, 1H, H-3 DNP); 8.65-8.67 (m, 2H, H-2/6 Pyr).

### Methyl 5-methoxypyrazolo[1,5-a]pyridine-3-carboxylate (C3a)

To a mixture of 44.1 g (0.143 mol) aminopyridinium salt and 27.7 g (0.201 mol) K₂CO₃ in 310 ml dry DMF was added dropwise 12.6 ml (0.151 mol) methyl propiolate and stirred at room temperature for 16 hrs. The residue was filtered off and the filtrate was evaporated in vacuum. Then saturated NaHCO₃ solution was added, extracted with dichloromethane and washed with 1 N HCl and H₂O. Then, the organic layer was dried with MgSO₄ and evaporated in vacuum. The crude product was purified by flash-chromatography (CH₂Cl₂ and subsequently CH₂Cl₂/MeOH 85:15).
Yield: 13.8 g (51 %) yellow solid.
MS (EI): m/z 206 (M)⁺. IR (NaCl) v (cm⁻¹): 2952; 2840; 1699; 1649; 1538; 1280; 1217; 1052; 774. ¹H NMR (CDCl₃, 360 MHz) δ (ppm): 3.90 (s, 3H, COOCH₃); 3.93 (s, 3H, OCH₃); 6.62 (dd, J = 7.5 Hz, 2.8 Hz, 1 H, H-6); 7.42 (dd, J = 2.8 Hz, 0.7 Hz, 1H, H-4); 8.28 (s, 1 H, H-2); 8.32 (dd, J = 7.5 Hz, 0.7 Hz, 1 H, H-7).

### 5-Hydroxypyrazolo[1,5-a]pyridine (C4a)

Methyl 5-methoxypyrazolo[1,5-a]pyridine-3-carboxylate (589 mg = 2.86 mmol) were treated with 9.5 ml hydrobromic acid (48%) and refluxed for 16 hrs. After cooling to room temperature the mixture was neutralized with 5N NaOH and extracted with diethylether. The organic layer was dried with MgSO₄, evaporated and purified by flash-chromatography (hexane/EtOAc 1:1).
Yield: 278 mg (73 %) light yellow solid.
Mp.: 216°C. MS (EI): m/z 134 (M)⁺. IR (KBr) v (cm⁻¹): 3139; 3094; 1649; 1521; 1188; 737; 715. ¹H NMR (DMSO-d₆, 360 MHz) δ (ppm): 6.23 (d, J = 1.8 Hz, 1H, H-3); 6.46 (dd, J = 7.4 Hz, 2.5 Hz, 1H, H-6); 6.77 (d, J = 2.5 Hz, 1H, H-4); 7.79 (d, J = 1.8 Hz, 1 H, H-2); 8.45 (d, J = 7.4 Hz, 1 H, H-7); 10.07 (s, 1 H, OH).

### N-Amino-(3-methoxy)pyridinium-2,4-dinitrophenolate (C2b)

Synthesis worked according to the preparation of C2a when using 3-methoxypyridine.
Yield: 9.2 g (85 %) yellow solid.
Mp.: 84°C. IR (KBr) v (cm⁻¹): 3093; 1610; 1568; 1527; 1255; 748; 708. ¹H NMR (DMSO-d₆, 600 MHz) δ (ppm): 3.98 (s, 3H, OCH₃); 6.60 (d, J = 9.6 Hz, 1 H, H-6 DNP); 7.90-7.92 (m, 2H, H-2/4 Pyr); 7.94 (dd, J = 9.6 Hz, 3.2 Hz, 1H, H-5 DNP); 8.41 (td, J = 5.4 Hz, 1.5 Hz, 1 H, H-5 Pyr); 8.48 (br s, 2H, NH₂); 8.57-8.59 (m, 1H, H-6 Pyr); 8.63 (d, J = 3.2 Hz, 1 H, H-3 DNP).

### Methyl 4-methoxypyrazolo[1,5-a]pyridine-3-carboxylate, Methyl 6-methoxypyrazolo[1,5-a]pyridine-3-carboxylate (C3b)

Synthesis worked according to the preparation of C3a when using N-amino-(3-methoxy)pyridinium-2,4-dinitrophenolate (C2b). Both isomers were separated by flash-chromatographie (CH₂Cl₂ and subsequently CH₂Cl₂/MeOH 85:15).
*Methyl 4-methoxypyrazolo[1,5-a]pyridine-3-carboxylate* (C3b - isomere 1)
Yield: 5.96 g (51 %) orange solid.
Mp.: 99°C. MS (EI): m/z 206 (M)⁺. IR (NaCl) v (cm⁻¹): 3113; 2950; 2844; 1717; 1556; 1520; 1285; 1211; 1057; 759. ¹H NMR (CDCl₃, 600 MHz) δ (ppm): 3.89 (s, 3H, COOCH₃); 4.02 (s, 3H, OCH₃); 6.67 (d, J = 7.6 Hz, 1 H, H-5); 6.85 (dd, J = 7.6 Hz, 6.8 Hz, 1H, H-6); 8.18 (d, J = 6.8 Hz, 1H, H-7); 8.36 (s, 1 H, H-2). ¹³C NMR (CDCl₃, 360 MHz) δ (ppm): 51.5; 56.3; 104.3; 105.0; 113.5; 122.6; 134.1; 145.4; 152.0; 162.9.

*Methyl 6-methoxypyrazolo[1,5-a]pyridine-3-carboxylate* (C3b - isomere 2)
Yield: 1.57 g (13 %) orange solid.
Mp.: 154°C. MS(EI) m/z 206 (M⁺). IR (NaCl) v (cm⁻¹): 3118; 2951; 2850; 1689; 1554; 1533; 1281; 1230; 1111; 744. ¹H NMR (CDCl₃, 600 MHz) δ (ppm): 3.87 (s, 3H, OCH₃); 3.90 (s, 3H, COOCH₃); 7.20 (dd, J = 9.6 Hz, 2.3 Hz, 1 H, H-5); 8.04 (d, J = 9.6 Hz, 1 H, H-4); 8.10 (d, J = 2.3 Hz, 1 H, H-7); 8.31 (s, 1 H, H-2). ¹³C NMR (CDCl₃, 360 MHz) δ (ppm): 51.2; 56.2; 103.6; 111.8; 118.7; 122.2; 136.9; 144.1; 150.3; 163.8.

### 4-Hydroxypyrazolo[1,5-a]pyridine (C4b)

Synthesis worked according to the preparation of C4a when using methyl 4-methoxypyrazolo[1,5-a]pyridine-3-carboxylate (C3b - isomere 1).
Yield: 2.6 g (72 %) yellow solid.
Mp.: 200°C. MS (APCI): m/z 135 (M+1)⁺. IR (KBr) v (cm⁻¹): 3447; 3053; 1644; 1557; 1257; 1177; 740. ¹H NMR (DMSO-d₆, 600 MHz) δ (ppm): 6.44 (d, J = 7.6 Hz, 1H, H-5); 6.59-6.60 (m, 1 H, H-3); 6.68 (dd, J = 7.6 Hz, 6.9 Hz, 1 H, H-6); 7.84 (d, J = 2.5 Hz, 1 H, H-2); 8.15 (d, J = 6.9 Hz, 1 H, H-7); 10.40 (s, 1 H, OH).

### 6-Hydroxypyrazolo[1,5-a]pyridine (C4c)

Synthesis worked according to the preparation of C4a when using methyl 6-methoxypyrazolo[1,5-a]pyridine-3-carboxylate (C3b - isomere 2).
Yield: 760 mg (75 %) light violet solid.
Mp.: 117°C. MS (EI): m/z 134 (M)⁺. IR (KBr) v (cm⁻¹): 3278; 3028; 1645; 1520; 1254; 1138; 800. ¹H NMR (DMSO-d₆, 600 MHz) δ (ppm): 6.46 (d, J = 2.1 Hz, 1 H, H-3); 6.94 (dd, J = 9.5 Hz, 2.0 Hz, 1 H, H-5); 7.53 (d, J = 9.5 Hz, 1 H, H-4); 7.76 (d, J = 2.0 Hz, 1H, H-7); 8.07-8.08 (m, 1 H, H-2); 9.51 (s, 1 H, OH).

### N-Amino-(2-methoxy)pyridinium-2,4-dinitrophenolate (C2c)

Synthesis worked according to the preparation of C2a when using 2-methoxypyridine. Yield: 214 mg (13 %) yellow solid.
Mp.: 134°C. MS (EI): m/z 184 (M+1)⁺ C₆H₃N₂O₅; 124 (M-1)⁺ C₆H₉N₂O. IR (KBr) v (cm⁻¹):
3284; 3099; 1603; 1558; 1522; 1132; 773. ¹H NMR (DMSO-d₆, 360 MHz) δ (ppm): 4.26 (s, 3H, OCH₃); 6.32 (d, J = 9.8 Hz, 1 H, H-6 DNP); 7.50 (ddd, J = 7.8 Hz, 6.5 Hz, 1.3 Hz, 1 H, H-5 Pyr); 7.69 (br s, 2H, NH₂); 7.72 (dd, J = 8.8 Hz, 1.3 Hz, 1 H, H-3 Pyr); 7.78 (dd, J = 9.8 Hz, 3.2 Hz, 1 H, H-5 DNP); 8.27 (ddd, J = 8.8 Hz, 7.8 Hz, 1.6 Hz, 1 H, H-4 Pyr); 8.55 (dd, J = 6.5 Hz, 1.6 Hz, 1 H, H-6 Pyr); 8.59 (d, J = 3.2 Hz, 1H, H-3 DNP).

### Methyl 7-methoxypyrazolo[1,5-a]pyridine-3-carboxylate (C3c)

Synthesis worked according to the preparation of C3a when using N-amino-(2-methoxy)pyridinium-2,4-dinitrophenolate (C2c).
Yield: 640 mg (39 %) light yellow solid.
Mp.: 135°C. MS(EI) m/z 206 (M⁺). IR (NaCl) v (cm⁻¹): 3099; 2953; 2850; 1701; 1637; 1533; 1246; 1043; 785. ¹H NMR (CDCl₃, 600 MHz) δ (ppm): 3.91 (s, 3H, COOCH₃); 4.18 (s, 3H, OCH₃); 6.29 (dd, J = 7.8 Hz, 0.8 Hz, 1 H, H-6); 7.42 (dd, J = 8.7 Hz, 7.8 Hz, 1 H, H-5); 7.81 (dd, J = 8.7 Hz, 0.8 Hz, 1 H, H-4); 8.44 (s, 1H, H-2). ¹³C NMR (CDCl₃, 600 MHz) δ (ppm): 51.2; 57.0; 91.0; 103.6; 110.7; 129.1; 142.6; 145.0; 151.5; 164.0.

### 7-Hydroxypyrazolo[1,5-a]pyridine (C4d)

Synthesis worked according to the preparation of C4a when using methyl 7-methoxypyrazolo[1,5-a]pyridine-3-carboxylate (C3c).
Yield: 499 mg (57 %) blue solid.
Mp.: 133°C. MS (APCI): m/z 135 (M+1)⁺. IR (KBr) v (cm⁻¹): 3435; 3084; 1635; 1512; 1088; 770. ¹H NMR (DMSO-d₆, 600 MHz; 323K) δ (ppm): 5.99 (br d, J = 6.1 Hz, 1H, H-6); 6.45-6.46 (m, 1 H, H-3); 6.94 (br d, J = 8.1 Hz, 1 H, H-4); 7.09 (dd, J = 8.1 Hz, 6.1 Hz, 1 H, H-5); 7.91-7.92 (m, 1 H, H-2).

### N-Amino-(4-hydroxymethyl)pyridinium-2,4-dinitrophenolate (C2d)

Synthesis worked according to the preparation of C2a when using 4-hydroxymethylypyridine.
Yield: 3.3 g (86 %) yellow solid.
Mp.: 110°C. MS (EI): m/z 184 (M+1)⁺ C₆H₃N₂O₅; 124 (M-1)⁺ C₆H₉N₂O, ¹H NMR (DMSO-d₆, 600 MHz) δ (ppm): 4.73 (s, 3H, CH₂OH); 6.35 (d, J = 9.7 Hz, 1 H, H-6 DNP); 7.80 (dd, J = 9.7 Hz, 3.1 Hz, 1H, H-5 DNP); 7.89-7.91 (m, 2H, H-3/5 Pyr); 8.28 (br s, 2H, NH₂); 8.59 (d, J = 3.1 Hz, 1 H, H-3 DNP); 8.70-8.71 (m, 2H, H-2/6 Pyr).

### Methyl 5-hydroxymethylpyrazolo[1,5-a]pyridine-3-carboxylate (C3d)

Synthesis worked according to the preparation of C3a when using N-amino-(4-hydroxymethyl)pyridinium-2,4-dinitrophenolate (C3c).
Yield: 468 mg (32 %) beige solid.
Mp.: 105°C. MS (APCl): m/z 207 (M+1)⁺. ¹H NMR (CDCl₃, 600 MHz) δ (ppm): 3.90 (s, 3H, COOCH₃); 4.79 (s, 2H, CH₂OH); 6.96 (dd, J = 7.1 Hz, 1.7 Hz, 1 H, H-6); 8.06-8.07 (m, 1 H, H-4); 8.35 (s, 1H, H-2); 8.44 (d, J = 7.1 Hz, 1 H, H-7).

### 5-Hydroxymethylpyrazolo[1,5-a]pyridine (C4e)

A mixture of 402 mg (1.95 mmol) methyl 5-hydroxymethylpyrazolo[1,5-a]pyridine-3-carboxylate (C3d) and 13.6 ml sulphuric acid (40%) was refluxed for 3 hrs and neutralized with 5N NaOH solution after cooling to room temperature. The solution was extracted with dichloromethane, the organic layers were dried with Na₂SO₄, evaporated and purified by flash-chromatography (EtOAc).
Yield: 289 mg (60 %) yellow solid.
Mp.: 47°C. MS (EI): m/z 148 (M)⁺. IR (NaCl) v (cm⁻¹): 3335; 2849; 1648; 1439; 1339; 1054; 774. ¹H NMR (CDCl₃, 360 MHz) δ (ppm): 2.12 (br s, 1H, OH); 4.70 (s, 2H, CH₂); 6.46 (dd, J = 2.2 Hz, 0.8 Hz, 1H, H-3); 6.71 (dd, J = 7.2 Hz, 1.7 Hz, 1 H, H-6); 7.48 (dd, J = 1.7 Hz, 0.8 Hz, 1 H, H-4); 7.92 (d, J = 2.2 Hz, 1H, H-2); 8.40 (d, J = 7.2 Hz, 1 H, H-7).

### 1b) 2-Hydroxypyrazolo[1,5-a]pyridine (C4f)

The synthesis of 2-hydroxypyrazolo[1,5-a]pyridine was done according to literature (Ochi, M.; Miyasaka, T.; Kanada, K.; Arakawa, K. Bull. Chem. Soc. Japan, (1976), 49, 1980-1984).

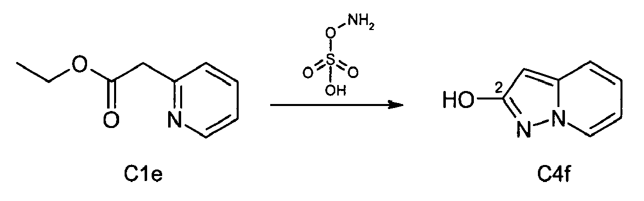

A solution of 4.14 g (36.6 mmol) hydroxylamine-O-sulfonic acid in 13.8 ml water was neutralized with KOH (2.6 N) at 0°C and added dropwise to 26.2 ml (0.172 mol) ethyl 2-pyridylacetate. After stirring for 30 h at room temperature the mixture was extracted with dichloromethane, the aqueous layer was adjusted to pH 9 with Na₂CO₃ solution (10%) and extracted with dichloromethane. Then, the aqueous layer was adjusted to pH 5 with acetic acid, extracted with dichloromethane, the organic layer was dried with MgSO₄ and evaporated. The crude product was purified by flash-chromatography (hexane/EtOAc 90:10).
Yield: 607 mg (39 %) white solid.
Mp.: 127-128°C. MS (APCI): m/z 135 (M+1)⁺. ¹H NMR (DMSO-d₆, 360 MHz) δ (ppm): 5.72 (d, J = 0.8 Hz, 1 H, H-3); 6.63 (ddd, J = 6.8 Hz, 6.8 Hz, 1.4 Hz, 1 H, H-6); 7.08 (ddd, J = 8.9 Hz, 6.8 Hz, 1.1 Hz, 1 H, H-5); 7.35 (ddd, J = 8.9 Hz, 1.4 Hz, 0.8 Hz, 1 H, H-4); 8.32 (dd, J = 6.8 Hz, 1.1 Hz, 1 H, H-7); 10.40 (s, 1 H, OH).

### 1c) 1-(5-Hydroxypyrazolo[1,5-a]pyridine-3-yl)ethanone (C4g), methyl 5-hydroxypyrazolo[1,5-a]pyridine-3-carboxylate (C4h)

The synthesis of the 3-substituted 5-hydroxypyrazolo[1,5-a]pyridine derivatives C4g-h was achieved by cycloaddition reaction of *O*-protected 4-hydroxy-*N*-aminopyridines with 3-butyn-2-one to get the acetyl substituted derivative C5a or with methyl propiolate to obtain the methyloxycarbonyl analogue C5b. Subsequent deprotection yielded the pyrazolo[1,5-a]pyridines C4g-h.

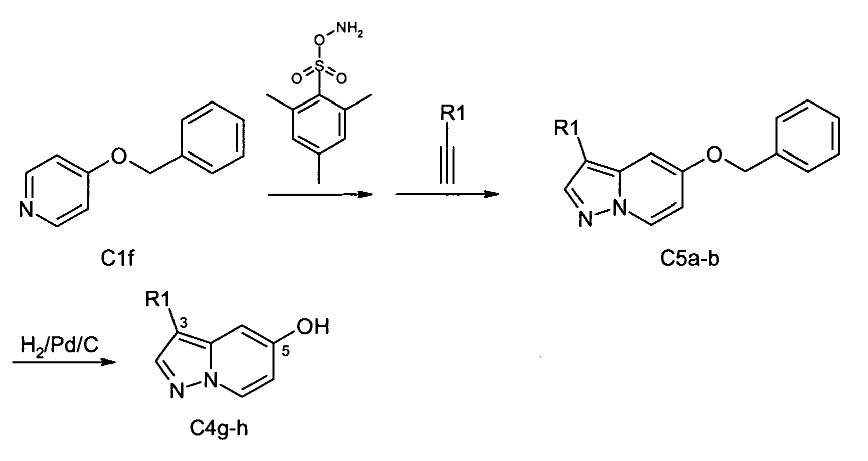

### 1-(5-Benzyloxypyrazolo[1,5-a]pyridine-3-yl)ethanone (C5a)

For the synthesis 1.5 g (1.92 mmol / 28 % content) hydroxylamine-*O*-mesitylene sulfonic acid in 3 ml dichloromethane were dried over Na₂SO₄. This solution was added dropwise to a solution of 355 mg (1.92 mmol) 4-benzyloxypyridine in 3 ml dichloromethane and the mixture was stirred for 1 h at 0°C and for 1 h at room temperature. After addition of diethylether an oil was formed, which was washing with diethylether and dried at the rotavapor. To the residue was added 15 ml DMF, 250 mg (1.81 mmol) K₂CO₃ and 0.18 ml (2.24 mmol) 3-butyn-2-one, the mixture was stirred for 18 h at room temperature and filtered over celite, evaporated and extracted with dichloromethan after addition of saturated NaHCO₃-solution. The organic layer was washed with 1 N HCl and water, dried with MgSO₄, evaporated and purified by flash-chromatography (hexane/EtOAc 4:1).
Yield: 225 mg (49 %) beige solid.
Mp.:158°C. MS (EI): m/z 266 (M)⁺. IR (NaCl) v (cm⁻¹): 3099; 2922; 1655; 1525; 1275; 1217; 744. ¹H NMR (CDCl₃, 360 MHz) δ (ppm): 2.52 (s, 3H, COCH₃); 5.18 (s, 2H, CH₂); 6.73 (dd, J = 7.6 Hz, 2.6 Hz, 1 H, H-6); 7.36-7.49 (m, 5H, Phenyl); 7.82 (d, J = 2.6 Hz, 1H, H-4); 8.24 (s, 1 H, H-2); 8.35 (d, J = 7.6 Hz, 1H, H-7). ¹³C NMR (CDCl₃, 360 MHz) δ (ppm): 27.8; 70.9; 98.5; 109.1; 112.5; 127.9; 128.6; 128.8; 129.9; 135.3; 142.0; 145.6; 159.7; 191.7.

### 1-(5-Hydroxypyrazolo[1,5-a]pyridine-3-yl)ethanone (C4g)

A suspension of 50 mg (0.11 mmol) 1-(5-benzyloxypyrazolo[1,5-a]pyridine-3-yl)ethanone (C5a) and 5 mg Pd/C (10%) in 3 ml ethanol were stirred for four days under H₂-atmosphere at room temperature. Then the mixture was filtered over celite, evaporated and purified by flash-chromatography (CH₂Cl₂/MeOH 95:5).
Yield: 26 mg (79 %) beige solid.
Mp.:210°C. MS (EI): m/z 176 (M)⁺. IR (NaCl) v (cm⁻¹): 3456; 3089; 2885; 1649; 1599; 1525; 1269; 1225. ¹H NMR (CDCl₃, 360 MHz) δ (ppm): 2.59 (s, 3H, COCH₃); 6.77 (dd, J = 7.6 Hz, 2.6 Hz, 1H, H-6); 8.27 (s, 1H, H-2); 8.38 (d, J = 2.6 Hz, 1H, H-4); 8.40 (d, J = 7.6 Hz, 1H, H-7). ¹³C NMR (CDCl₃, 360 MHz) δ (ppm): 27.9; 100.1; 109.5; 110.9; 131.2; 142.3; 146.3; 161.2; 190.5.

### Methyl 5-benzyloxypyrazolo[1,5-a]pyridine-3-carboxylate (C5b)

Synthesis worked according to the preparation of C5a and as described in literature (Elsner, J.; Boeckler, F.; Heinemann, F.W.; Hübner, H.; Gmeiner, P. J. Med. Chem. (2005), 48, 5771-5779) when using methyl propiolate.
Yield: 2.5 g (23 %) yellow solid.
MS (APCI): m/z 283 (M+1)⁺. ¹H NMR (CDCl₃, 600 MHz) δ (ppm): 3.89 (s, 3H, COOCH₃); 5.17 (s, 2H, CH₂); 6.68 (dd, J = 7.6 Hz, 2.6 Hz, 1H, H-6); 7.36-7.48 (m, 5H, Phenyl); 7.53 (d, J = 2.6 Hz, 1H, H-4); 8.28 (s, 1H, H-2); 8.33 (d, J = 7.6 Hz, 1H, H-7).

### Methyl 5-hydroxypyrazolo[1,5-a]pyridine-3-carboxylate (C4h)

A suspension of 30 mg (0.11 mmol) methyl 5-benzyloxypyrazolo[1,5-a]pyridine-3-carboxylate (C5b) and 8 mg Pd/C (10%) in 5 ml ethanol were stirred over night under H₂-atmosphere at room temperature. Then, the mixture was filtered over celite, evaporated and purified by flash-chromatography (hexane/EtOAc 1:1).
Yield: 16 mg (76 %) white solid.
Mp.: 264°C. MS (APCI): m/z 193 (M+1)⁺. IR (KBr) v (cm⁻¹): 3435; 3057; 2949; 1699; 1647; 1541; 1277; 1248; 1045. ¹H NMR (DMSO-d₆, 600 MHz) δ (ppm): 3.79 (s, 3H, COOCH₃); 6.70 (dd, J = 7.6 Hz, 2.6 Hz, 1H, H-6); 7.29 (d, J = 2.6 Hz, 1H, H-4); 8.27 (s, 1H, H-2); 8.67 (d, J = 7.6 Hz, 1H, H-7); 10.92 (s, 1H, OH). ¹³C NMR (DMSO-d₆, 360 MHz) δ (ppm): 51.3; 99.2; 100.7; 108.7; 131.9; 143.0; 145.5; 158.8; 163.7.

### 1c) 7-Hydroxytetrazolo[1,5-a]pyridine (C4i)

The synthesis of 7-hydroxytetrazolo[1,5-a]pyridine was achieved according to literature (Keith, J.M. J. Org. Chem. (2006), 71, 9540-9543) by reacting a protected hydroxypyridine and an activated azide derivative as follows:

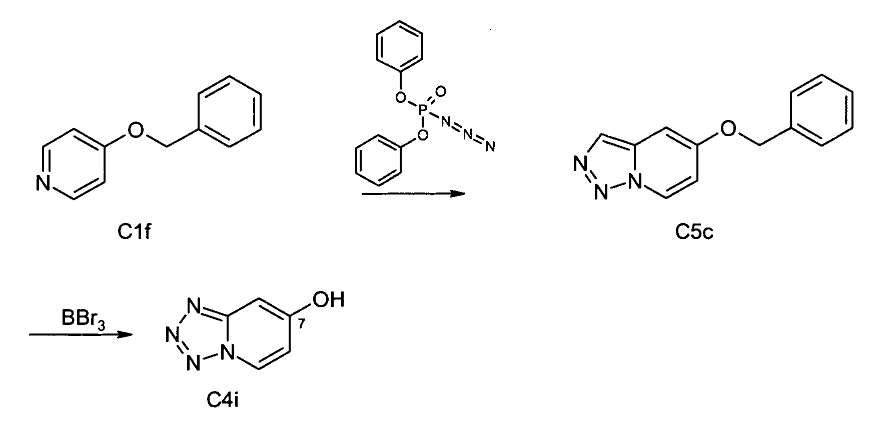

### 7-Benzyloxytetrazolo[1,5-a]pyridine (C5c)

A mixture consisting of 1.0 g (4.98 mol) 4-benzyloxypyridine-N-oxide (C1f), 2.15 ml (9.95 mol) diphenylphosphorylazide and 0.8 ml (9.95 mol) pyridine was refluxed for 24 hrs and passed through a silica gel column (CH₂Cl₂). After recrystallization with ethyl acetate, the product was filtered off, washed with hexane and dried in vacuum.
Yield: 234 mg (21 %) crystalline white solid.
Mp.:173°C. MS (EI) m/z 226 (M)⁺. IR (NaCl) v (cm⁻¹): 3053; 2926; 1643; 1545; 1213; 804. ¹H NMR (CDCl₃, 600 MHz) δ (ppm): 5.21 (s, 2H, OCH₃); 6.94 (dd, J = 7.5 Hz, 2.2 Hz, 1H, H-6); 7.24 (d, J = 2.2Hz, 1H, H-8); 7.36-7.46 (m, 5H, Phenyl); 8.63 (d, J = 7.5 Hz, 1H, H-5). ¹³C NMR (CDCl₃, 600 MHz) δ (ppm): 71.4; 93.1; 112.2; 125.5; 127.8; 128.9; 129.0; 134.4; 150.3; 161.2.

### 7-Hydroxytetrazolo[1,5-a]pyridine (C4i)

The solution of 50 mg (0.22 mmol) 7-benzyloxytetrazolo[1,5-a]pyridine (C5c) in 5 ml dichloromethane were treated with 0.44 ml (0.44 mmol) boron tribromide (1 M solution in dichloromethane) and stirred for 4 hs at room temperature. After addition of 2 ml ethanol, stirring was continued for further 30 minutes at room temperature. Then the solvent was evaporated and the residue purified by flash-chromatography (EtOAc).
Yield: 30 mg (99 %) beige solid.
Mp.:67°C. MS (EI) m/z 136 (M)⁺. IR (KBr) v (cm⁻¹): 3433; 3047; 1647; 1207; 825. ¹H NMR (DMSO-d₆, 600 MHz) δ (ppm): 7.00 (dd, J = 7.3 Hz, 1.9 Hz, 1H, H-6); 7.15 (d, J = 1.9 Hz, 1H, H-8); 9.10 (d, J = 7.3 Hz, 1H, H-5); 11.52 (br s, 1H, OH). ¹³C NMR (CD₃OD, 600 MHz) δ (ppm): 95.4; 114.1; 128.7; 152.6; 164.2.

### 2. Synthesis of the heteroarene components according to formula XIII 5-(4-Bromobutoxy)pyrazolo[1,5-a]pyridine (C6a), 2-(4-bromobutoxy)pyrazolo[1,5-a]pyridine (C6b), 4-(4-bromobutoxy)pyrazolo[1,5-a]pyridine (C6c), 6-(4-bromobutoxy)pyrazolo[1,5-a]pyridine (C6d), 7-(4-bromobutoxy)pyrazolo[1,5-a]pyridine (C6e), 5-(3-Bromopropoxymethyl)pyrazolo[1,5-a]pyridine (C6f), 1-[5-(4-brombutoxy)pyrazolo[1,5-a]pyridine-3-yl]ethanone (C6g), methyl 5-(4-bromobutoxy)pyrazolo[1,5-a]pyridine-3-carboxylate (C6h), 7-(4-bromobutoxy)fetrazolo[1,5-a]pyridine (C6i)

The synthesis of the components according to formula XIII were done by alkylation of the functional group in formula XI with dibromoalkylidene derivatives of different length to get the compounds C6.

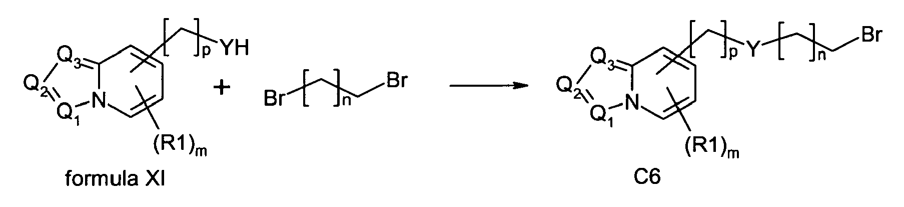

### 5-(4-Bromobutoxy)pyrazolo[1,5-a]pyridine (C6a)

To a mixture of 1.19 g (8.88 mmol) 5-hydroxypyrazolo[1,5-a]pyridine (C4a), 1.23 g (8.89 mmol) potassium carbonate and 44.1 ml of dry dimethylformamide was added dropwise 3.18 ml (26.67 mmol) 1,4-dibromobutane. The suspension was heated to 60°C for 8 hrs. After adding water the solution was extracted with ethylacetate. The organic layer was dried with Na₂SO₄, evaporated and purified by flash-chromatography (hexane/EtOAc 20:30).
Yield: 1.7 g (71 %) orange oil.
MS (EI): m/z 270 (M+1)⁺, 268 (M-1)⁺. IR (NaCl) v (cm⁻¹): 3097; 2945; 2874; 1648; 1537; 1290; 1227; 1040; 746. ¹H NMR (CDCl₃, 360 MHz) δ (ppm): 1.95-2.02 (m, 2H, OCH₂CH₂); 2.04-2.12 (m, 2H, CH₂CH₂Br); 3.50 (t, J = 6.5 Hz, 2H, CH₂Br); 4.02 (t, J = 6.5 Hz, 2H, OCH₂); 6.30-6.31 (m, 1H, H-3); 6.43 (dd, J = 7.8 Hz, 2.6 Hz, 1H, H-6); 6.72 (d, J = 2.6 Hz, 1H, H-4); 7.85 (d, J = 2.1 Hz, 1H, H-2); 8.28 (d, J = 7.8 Hz, 1H, H-7). ¹³C NMR (CDCl₃, 360 MHz) δ (ppm): 27.6; 29.4; 33.2; 67.2; 95.4; 95.5; 106.6; 129.4; 141.0; 142.8; 155.5. C H N (%): C₁₁H₁₃N₂OBr; calculated: C 49.09; H 4.87; N 10.41; found: C 48.94; H 4.78; N 10.23.

### 2-(4-Bromobutoxy)pyrazolo[1,5-a]pyridine (C6b)

Synthesis worked according to the preparation of C6a when using 2-hydroxypyrazolo[1,5-a]pyridine (C4f).
Yield: 1.04 g (85 %) light yellow oil.
MS (EI): m/z 270 (M+1)⁺, 268 (M-1)⁺. IR (NaCl) v (cm⁻¹): 3089; 2949; 2858; 1635; 1531; 1257; 1149; 754. ¹H NMR (CDCl₃, 360 MHz) δ (ppm): 1.94-2.02 (m, 2H, OCH₂CH₂); 2.05-2.13 (m, 2H, CH₂CH₂Br); 3.50 (t, J = 6.6 Hz, 2H, CH₂Br); 4.29 (t, J = 6.1 Hz, 2H, OCH₂); 5.82 (d, J = 0.8 Hz, 1H, H-3); 6.59 (ddd, J = 7.0 Hz, 7.0 Hz, 1,3 Hz, 1H, H-6); 7.04 (ddd, J = 8.9 Hz, 7.0 Hz, 1.1 Hz, 1H, H-5); 7.29 (ddd, J = 8.9 Hz, 1.3 Hz, 0.8 Hz, 1H, H-4); 8.21 (dd, J = 7.0 Hz, 1.1 Hz, 1H, H-7). ¹³C NMR (CDCl₃, 360 MHz) δ (ppm): 28.0; 29.4; 33.5 ; 68.3; 79.8; 109.8; 116.4; 124.0; 128.3; 141.7; 165.6.

### 4-(4-Bromobutoxy)pyrazolo[1,5-a]pyridine (C6c)

Synthesis worked according to the preparation of C6a when using 4-hydroxypyrazolo[1,5-a]pyridine (C4b).
Yield: 3.21 g (66 %) light green solid.
Mp.: 40°C. MS (APCl): m/z 271 (M+2)⁺, 268 (M)⁺. IR (NaCl) v (cm⁻¹): 3109; 2952; 2873; 1548; 1279; 1241; 1094; 748. ¹H NMR (CDCl₃, 360 MHz) δ (ppm): 2.01-2.16 (m, 4H, CH₂CH₂CH₂CH₂); 3.52 (t, J = 6.4 Hz, 2H, CH₂Br); 4.13 (t, J = 5.7 Hz, 2H, OCH₂); 6.34 (d, J = 7.6 Hz, 1H, H-5); 6.61-6.65 (m, 2H, H-3, H-6); 7.87 (d, J = 2.4 Hz, 1H, H-2); 8.13 (d, J = 6.9 Hz, 1H, H-7). ¹³C NMR (CDCl₃, 360 MHz) δ (ppm): 27.6; 29.5; 33.3; 67.4; 95.3; 99.8; 111.4; 121.9; 135.3; 140.8; 150.3.
C H N (%):C₁₁H₁₃N₂OBr; calculated: C 49.09; H 4.87; N 10.41; found: C 48.90; H 5.05; N 10.33.

### 6-(4-Bromobutoxy)pyrazolo[1,5-a]pyridine (C6d)

Synthesis worked according to the preparation of C6a when using 6-hydroxypyrazolo[1,5-a]pyridine (C4c).
Yield: 153 mg (70 %) beige solid.
Mp.: 55°C. MS (EI): m/z 270 (M+1)⁺, 268 (M-1)⁺. IR (NaCl) v (cm⁻¹): 3105; 2943; 2873; 1642; 1525; 1291; 1239; 1199; 1025; 756. ¹H NMR (CDCl₃, 600 MHz) δ (ppm): 1.96-2.01 (m, 2H, OCH₂CH₂); 2.07-2.11 (m, 2H, CH₂CH₂Br); 3.50 (t, J = 6.6 Hz, 2H, CH₂Br); 3.99 (t, J = 5.9 Hz, 2H, OCH₂); 6.45 (d, J = 2.1 Hz, 1H, H-3); 6.91 (dd, J = 9.5 Hz, 2.2 Hz, 1H, H-5); 7.41 (d, J = 9.5 Hz, 1H, H-4); 7.84 (d, J = 2.2 Hz, 1H, H-7); 8.05-8.06 (m, 1H, H-2). ¹³C NMR (CDCl₃, 600 MHz) δ (ppm): 27.8; 29.4; 33.3; 68.0; 96.8; 111.8; 117.9; 119.0; 136.5; 140.8; 148.2.
C H N (%):C₁₁H₁₃N₂OBr; calculated: C 49.09; H 4.87; N 10.41; found: C 49.26; H 4.84; N 10.44.

### 7-(4-Bromobutoxy)pyrazolo[1,5-a]pyridine (C6e)

Synthesis worked according to the preparation of C6a when using 7-hydroxypyrazolo[1,5-a]pyridine (C4d).
Yield: 364 mg (31 %) white solid.
Mp.: 66°C. MS (APCI): m/z 271 (M+2)⁺, 269 (M)⁺. IR (NaCl) v (cm⁻¹): 3099; 2953; 1633; 1547; 1103; 773. ¹H NMR (CDCl₃, 360 MHz) δ (ppm): 2.13-2.17 (m, 4H, OCH₂CH₂CH₂CH₂Br); 3.49-3.52 (m, 2H, CH₂Br); 4.30-4.33 (m, 2H, OCH₂); 6.05 (dd, J = 7.3 Hz, 1.1 Hz, 1H, H-6); 6.50 (d, J = 2.3 Hz, 1H, H-3); 7.07 (dd, J = 8.8 Hz, 7.3 Hz, 1H, H-5); 7.18 (dd, J = 8.8 Hz, 1.1 Hz, 1H, H-4); 7.99 (d, J = 2.3 Hz, 1H, H-2). ¹³C NMR (CDCl₃, 360 MHz) δ (ppm): 27.5; 29.2; 33.2; 68.8; 89.2; 96.9; 110.1; 124.5; 142.1; 150.5.

### 5-(3-Bromopropoxymethyl)pyrazolo[1,5-a]pyridine (C6f)

To a solution of 290 mg (7.25 mmol) NaOH in 0.60 ml water 173 mg (1.17 mmol) 5-hydroxymethylpyrazolo[1,5-a]pyridine (C4e), 0.75 ml (7.31 mmol) 1,3-dibromopropane and 23.8 mg (0.07 mmol) tetrabutylammonium hydrogensulfate was added. After stirring over night at room temperature, water was added and the mixture was extracted with hexane. The organic layer was washed with brine, dried with Na₂SO₄, evaporated and purified by preparative HPLC (RP-18; H₂O with 0.1% TFA 100% to MeCN 100%).
Yield: 88 mg (28 %) light yellow oil.
MS (EI): m/z 270 (M+1)⁺, 268 (M-1)⁺. IR (NaCl) v (cm⁻¹): 3088; 2962; 2858; 1645; 1516; 1257; 1101; 783. ¹H NMR (CDCl₃, 600 MHz) δ (ppm): 2.14-2.18 (m, 2H, CH₂CH₂Br); 3.56 (t, J = 5.8 Hz, 2H, CH₂Br); 3.65 (t, J = 6.4 Hz, 2H, OCH₂CH₂); 4.53 (s, 2H, CH₂O); 6.48 (d, J = 2.0 Hz, 1H, H-3); 6.72 (dd, J = 7.2 Hz, 1.5 Hz, 1H, H-6); 7.47-7.48 (m, 1H, H-4); 7.94 (d, J = 2.0 Hz, 1H, H-2); 8.43 (d, J = 7.2 Hz, 1H, H-7). ¹³C NMR (CDCl₃, 360 MHz) δ (ppm): 30.4; 32.8; 68.0; 72.0; 96.8; 111.4; 115.7; 128.6; 134.2; 139.9; 142.3.

### 1-[5-(4-Brombutoxy)pyrazolo[1,5-a]pyridine-3-yl]ethanone (C6g)

Synthesis worked according to the preparation of C6a when using 1-(5-hydroxypyrazolo[1,5-a]pyridine-3-yl)ethanone (C4g).
Yield: 58 mg (78 %) light yellow solid.
Mp.:62°C. MS (EI): m/z 312 (M+1)⁺, 310 (M-1)⁺. IR (NaCl) v (cm⁻¹): 3109; 2933; 2875; 1654; 1633; 1524; 1277; 1217; 795. ¹H NMR (CDCl₃, 600 MHz) δ (ppm): 2.00-2.04 (m, 2H, OCH₂CH₂); 2.07-2.11 (m, 2H, CH₂CH₂Br); 2.52 (s, 3H, COCH₃); 3.50 (t, J = 6.4 Hz, 2H, CH₂Br); 4.14 (t, J = 6.0 Hz, 2H, OCH₂); 6.66 (dd, J = 7.6 Hz, 2.6 Hz, 1H, H-6); 7.67 (d, J = 2.6 Hz, 1H, H-4); 8.24 (s, 1H, H-2); 8.34 (d, J = 7.6 Hz, 1H, H-7). ¹³C NMR (CDCl₃, 600 MHz) δ (ppm): 27.5; 27.8; 29.4; 33.1; 67.9; 98.0; 109.0; 112.4; 129.9; 142.0; 145.7; 159.9; 191.8.

### Methyl 5-(4-bromobutoxy)pyrazolo[1,5-a]pyridine-3-carboxylate (C6h)

Synthesis worked according to the preparation of C6a when using methyl 5-hydroxypyrazolo[1,5-a]pyridine-3-carboxylate (C4h).
Yield: 200 mg (59 %) light yellow solid.
Mp.: 84°C. MS (EI): m/z 328 (M+1)⁺, 326 (M-1)⁺. IR (NaCl) v (cm⁻¹): 3107; 2947; 1699; 1647; 1539; 1277; 1213; 1049; 773. ¹H NMR (CDCl₃, 600 MHz) δ (ppm): 2.00-2.05 (m, 2H, OCH₂CH₂); 2.07-2.11 (m, 2H, CH₂CH₂Br); 3.50 (t, J = 6.4 Hz, 2H, CH₂Br); 3.89 (s, 3H, COOCH₃); 4.13 (t, J = 6.1 Hz, 2H, OCH₂); 6.61 (dd, J = 7.5 Hz, 2.6 Hz, 1H, H-6); 7.40 (d, J = 2.6 Hz, 1H, H-4); 8.28 (s, 1H, H-2); 8.33 (d, J = 7.5 Hz, 1H, H-7). ¹³C NMR (CDCl₃, 360 MHz) δ (ppm): 27.5; 29.4; 33.1; 51.1; 67.8; 96.9; 102.4; 108.3; 130.1; 142.8; 145.3; 158.8; 164.1.

### 7-(4-Bromobutoxy)tetrazolo[1,5-a]pyridine (C6i)

Synthesis worked according to the preparation of C6a when using methyl 7-hydroxytetrazolo[1,2-a]pyridine (C4i).
Yield: 17 mg (36 %) beige solid.
Mp.:77°C. MS (APCI) m/z 273 (M+2)⁺, 271 (M)⁺. IR (NaCl) v (cm-¹): 3062; 2943; 1647; 1547; 1211; 1014; 818. ¹H NMR (CDCl₃, 600 MHz) δ (ppm): 2.04-2.13 (m, 4H, CH₂CH₂CH₂Br); 3.51 (t, J = 6.5 Hz, 2H, CH₂Br); 4.16 (t, J = 5.9 Hz, 2H, OCH₂); 6.87 (dd, J = 7.5 Hz, 2.3 Hz, 1H, H-6); 7.15 (d, J = 2.3 Hz, 1H, H-8); 8.62 (d, J = 7.5 Hz, 1H, H-5). ¹³C NMR (CDCl₃, 600 MHz) δ (ppm): 27.3; 29.2; 32.9; 68.5; 92.3; 112.1; 125.5; 150.5; 161.5.

### 3. Phenylpiperazine derivatives according to formula XIV

*1-(2,3-Dichlorophenyl)piperazine* (C7a), *1-(2-methoxyphenyl)piperazine* (C7b), 1-(2-*chlorophenyl)piperazine* (C7c), *1-(4-methoxyphenyl)piperazine* (C7Xd), *1-(4-chlorophenyl)piperazine* (C7e), *1-(4-hydroxyphenyl)piperazine* (C7f)

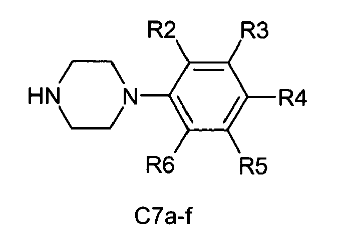

Derivatives according to formula C7a-f were purchased:
*1-(2,3-Dichlorophenyl)piperazine* (C7a)
from *Alfa Aesar, Karlsruhe (Germany)* (R2, R3 = Cl, R4-R6 = H - [Order number: L18697] *1-(2-Methoxyphenyl)piperazine* (C7b)
from *Acros Organics Janssen, Geel (Belgium)* (R2 = OMe, R3-R6 = H - [Order number: 232872500]
*1-(2-chlorophenyl)piperazine* (C7c)
from *Sigma-Aldrich, Taufkirchen (Germany)* (R2 = Cl, R3-R6 = H - [Order number: C67605])
*1-(4-Methoxyphenyl)piperazine* (C7d)
from *Aesar, Karlsruhe (Germany)* (R4=OMe, R2-R3 and R5-R6=H - [Order number: 2007])
*1-(4-chlorophenyl)piperazine* (C7e)
from Acros *Organics Janssen, Geel (Belgium)* (R4 = Cl, R2-R3 and R5-R6 = H - [Order number: 10992];
*1-(4-hydroxyphenyl)piperazine* (C7f)
from *Acros Organics Janssen, Geel (Belgium)* (R4 = OH, R2-R3 and R5-R6 = H - [Order number: 1003300502])

### 4. Synthesis of Example compounds according to formula XV

### Example E15:

### 5-[4-[4-(2,3-Dichlorophenyl)piperazin-1-yl]butoxy]pyrazolo[1,5-a]pyridine

The synthesis of 5-[4-[4-(2,3-Dichlorophenyl)piperazin-1-yl]butoxy]pyrazolo[1,5-a]pyridine (E15) was started by reacting 455 mg (1.69 mmol) 5-(4-bromobutoxy)pyrazolo[1,5-a]pyridine (C6a) with 381 mg (2.54 mmol) in sodium iodide in 7.6 ml acetonitrile and refluxing for 30 minutes at 95°C. Then, 430 mg (1.86 mmol) 1-(2,3-dichlorophenyl)-piperazine (C7a) in 0.26 ml (1.86 mmol) triethylamine was added and heated for futher 5 hrs at 85°C. The mixture was cooled down, mixed with NaHCO₃-solution and extracted with dichloromethane. The organic layer was dried with Na₂SO₄, evaporated and purified by flash-chromatography (CH₂Cl₂/MeOH 98:2).
Yield: 545 mg (77 %) beige solid.
Mp.: 105°C. MS (APCl): m/z 421 (M+2)⁺, 419 (M)⁺. IR (NaCl) v (cm⁻¹): 2944; 2817; 1647; 1578; 1290; 1227; 1190; 1044; 774. ¹H NMR (CDCl₃, 360 MHz) δ (ppm): 1.69-1.77 (m, 2H, CH₂CH₂N); 1.84-1.92 (m, 2H, OCH₂CH₂); 2.50 (t, J = 7.2 Hz, 2H, CH₂N); 2.65-2.67 (m, 4H, Piperazine); 3.06-3.09 (m, 4H, Piperazine); 4.02 (t, J = 6.5 Hz, 2H, OCH₂); 6.29-6.30 (m, 1H, H-3); 6.44 (dd, J = 7.6 Hz, 2.6 Hz, 1H, H-6); 6.73 (d, J = 2.6 Hz, 1H, H-4); 6.95 (dd, J = 6.7 Hz, 2.9 Hz, 1H, Phenyl H6); 7.13-7.15 (m, 2H, Phenyl H4, Phenyl H5); 7.85 (d, J = 2.1 Hz, 1H, H-2); 8.28 (d, J = 7.6 Hz, 1H, H-7). ¹³C NMR (CDCl₃, 360 MHz) δ (ppm): 23.4; 27.0; 51.4; 53.3; 58.2; 68.0; 95.3; 95.4; 106.8; 118.6; 124.6; 127.5; 127.6; 129.4; 134.1; 141.1; 142.8; 151.3; 155.7.
C H N (%):C₂₁H₂₄N₄OCl₂; calculated.: C 60.15; H 5.77; N 13.36; found.: C 59.79; H 5.81; N 13.14.

### Example E16:

### 5-[4-[4-(2- Methoxyphenyl)piperazin-1-yl]butoxy]pyrazolo[1,5-a]pyridine

Synthesis worked according to the preparation of E15 when using 5-(4-bromobutoxy)pyrazolo[1,5-a]pyridine (C6a) and 1-(2-methoxyphenyl)piperazine (C7b). Yield: 246 mg (89 %) beige solid.
Mp.: 121°C. MS (EI): m/z 380 (M)⁺. IR (NaCl) v (cm⁻¹): 3098; 2942; 2815; 1648; 1500; 1291; 1241; 1228; 1190; 748. ¹H NMR (CDCl₃, 600 MHz) δ (ppm): 1.71-1.76 (m, 2H, CH₂CH₂N); 1.85-1.90 (m, 2H, OCH₂CH₂); 2.49 (t, J = 7.6 Hz, 2H, CH₂N); 2.66-2.70 (m, 4H, Piperazine); 3.08-3.14 (m, 4H, Piperazine); 3.86 (s, 3H, OCH₃); 4.02 (t, J = 6.3 Hz, 2H, OCH₂); 6.29-6.30 (m, 1H, H-3); 6.44 (dd, J = 7.6 Hz, 2.6 Hz, 1H, H-6); 6.73 (d, J = 2.6 Hz, 1H, H-4); 6.86 (dd, J = 7.8 Hz, 0.9 Hz, 1H, Phenyl H3); 6.92 (ddd, J = 7.5 Hz, 7.0 Hz, 0.9 Hz, 1H, Phenyl H5); 6.95 (dd, J = 7.5 Hz, 1.7 Hz, 1H, Phenyl H6); 7.00 (ddd, J = 7.8 Hz, 7.0 Hz, 1.7 Hz, 1H, Phenyl H4); 7.84 (d, J = 2.1 Hz, 1H, H-2); 8.28 (d, J = 7.6 Hz, 1H, H-7). ¹³C NMR (CDCl₃, 360 MHz) δ (ppm): 23.5; 27.1; 50.7; 53.5; 55.5; 58.3; 68.1; 95.4; 106.8; 111.3; 118.2; 121.0; 123.0; 129.4; 141.1; 141.4; 142.8; 152.4; 155.7.
C H N (%):C₂₂H₂₈N₄O₂ x 0.3H₂O; calculated.: C 68.48; H 7.47; N 14.52; found: C 68.51; H 7.71; N 14.38.

### Example E17:

### 5-[4-[4-(2- Chlorophenyl)piperazin-1-yl]butoxy]pyrazolo[1,5-a]pyridine

Synthesis worked according to the preparation of E15 when using 5-(4-bromobutoxy)pyrazolo[1,5-a]pyridine (C6a) and 1-(2-chlorophenyl)piperazine (C7c).
Yield: 619 mg (94 %) beige solid.
Mp.: 163°C. MS (EI): m/z 386 (M+1)⁺, 384 (M-1 )⁺. IR (NaCl) v (cm⁻¹): 3097; 2945; 2816; 1648; 1480; 1290; 1228; 1192; 750. ¹H NMR (CDCl₃, 600 MHz) δ (ppm): 1.71-1.76 (m, 2H, CH₂CH₂N); 1.85-1.90 (m, 2H, OCH₂CH₂); 2.50 (t, J = 7.6 Hz, 2H, CH₂N); 2.64-2.68 (m, 4H, Piperazine); 3.07-3.11 (m, 4H, Piperazine); 4.02 (t, J = 6.3 Hz, 2H, OCH₂); 6.29-6.30 (m, 1H, H-3); 6.44 (dd, J = 7.6 Hz, 2.6 Hz, 1H, H-6); 6.73 (d, J = 2.6 Hz, 1H, H-4); 6.96 (ddd, J = 7.8 Hz, 7.3 Hz, 1.3 Hz, 1H, Phenyl H4); 7.04 (dd, J = 8.0 Hz, 1.3 Hz, 1H, Phenyl H6); 7.21 (ddd, J = 8.0 Hz, 7.3 Hz, 1.2 Hz, 1H, Phenyl H5); 7.35 (dd, J = 7.8 Hz, 1.2 Hz, 1H, Phenyl H3); 7.85 (d, J = 2.0 Hz, 1H, H-2); 8.28 (d, J = 7.6 Hz, 1H, H-7). ¹³C NMR (CDCl₃, 360 MHz) δ (ppm): 23.5; 27.1; 51.3; 53.5; 58.2; 68.1; 95.4; 106.8; 120.5; 123.7; 127.7; 128.9 ; 129.4; 130.8; 141.1; 142.8; 149.4; 155.8.
C H N (%):C₂₁H₂₅N₄OCl x 0.25H₂O; calculated: C 64.77; H 6.60; N 14.39; found: C 65.02; H 6.69; N 14.28.

### Example E18:

### 5-[{4-[4-(4-Methoxyphenyl)piperazin-1-yl]butoxy]pyrazolo[1,5-a]pyridine

Synthesis worked according to the preparation of E15 when using 5-(4-bromobutoxy)pyrazolo[1,5-a]pyridine (C6a) and 1-(4-methoxyphenyl)piperazine (C7d). Yield: 210 mg (99 %) white solid.
Mp.: 111°C. MS (EI): m/z 380 (M)⁺. IR (NaCl) v (cm⁻¹): 3098; 2945; 2817; 1647; 1511; 1441; 1291; 1228; 1191; 822. ¹H NMR (CDCl₃, 600 MHz) δ (ppm): 1.70-1.75 (m, 2H, CH₂CH₂N); 1.85-1.89 (m, 2H, OCH₂CH₂); 2.48 (t, J = 7.6 Hz, 2H, CH₂N); 2.62-2.64 (m, 4H, Piperazine); 3.10-3.12 (m, 4H, Piperazine); 3.76 (s, 3H, OCH₃); 4.01 (t, J = 6.3 Hz, 2H, OCH₂); 6.29-6.30 (m, 1H, H-3); 6.43 (dd, J = 7.6 Hz, 2.6 Hz, 1H, H-6); 6.73 (d, J = 2.6 Hz, 1H, H-4); 6.83-6.84 (m, 2H, Phenyl H2, Phenyl H6); 6.89-6.91 (m, 2H, Phenyl H3, Phenyl H5); 7.84 (d, J = 2.1 Hz, 1H, H-2); 8.28 (d, J = 7.6 Hz, 1H, H-7). ¹³C NMR (CDCl₃, 360 MHz) δ (ppm): 23.5; 27.0; 50.7; 53.5; 55.7; 58.2; 68.1; 95.4; 106.8; 114.5; 118.2; 129.4; 141.1; 142.8; 145.8; 153.9; 155.7.
C H N (%):C₂₂H₂₈N₄O₂; calculated: C 69.45; H 7.42; N 14.72; found: C 69.28; H 7.51; N 14.78.

### Example E19:

### 5-[4-[4-(4-Chlorophenyl)piperazin-1-yl]butoxy]pyrazolo[1,5-a]pyridine

Synthesis worked according to the preparation of E15 when using 5-(4-bromobutoxy)pyrazolo[1,5-a]pyridine (C6a) and 1-(4-chlorophenyl)piperazine (C7e). Yield: 191 mg (89 %) white solid.
Mp.: 125°C. MS (EI): m/z 386 (M+1)⁺, 384 (M-1)⁺. IR (NaCl) v (cm⁻¹): 3067; 2946; 2815; 1647; 1485; 1289; 1227; 1191; 827; 775. ¹H NMR (CDCl₃, 600 MHz) δ (ppm): 1.70-1.75 (m, 2H, CH₂CH₂N); 1.85-1.90 (m, 2H, OCH₂CH₂); 2.47 (t, J = 7.6 Hz, 2H, CH₂N); 2.60-2.62 (m, 4H, Piperazine); 3.16-3.18 (m, 4H, Piperazine); 4.01 (t, J = 6.3 Hz, 2H, OCH₂); 6.29-6.30 (m, 1H, H-3); 6.43 (dd, J = 7.6 Hz, 2.6 Hz, 1H, H-6); 6.73 (d, J = 2.6 Hz, 1H, H-4); 6.82-6.84 (m, 2H; Phenyl H2, Phenyl H6); 7.19-7.20 (m, 2H, Phenyl H3, Phenyl H5); 7.85 (d, J = 2.0 Hz, 1H, H-2); 8.28 (d, J = 7.6 Hz, 1H, H-7). ¹³C NMR (CDCl₃, 360 MHz) δ (ppm): 23.5; 27.0; 49.3; 53.2; 58.2; 68.0; 95.4; 106.7; 117.3; 124.5; 129.0; 129.4, 141.1; 142.8; 150.0; 155.7.
C H N (%):C₂₁H₂₅N₄OCl x 0.2H₂O; calculated: C 64.92; H 6.59; N 14.42; found: C 65.07; H 6.68; N 14.48.

### Example E20:

### 5-[4-[4-(4-Hydroxyphenyl)piperazin-1-yl]butoxy]pyrazolo[1,5-a]pyridine

Synthesis worked according to the preparation of E15 when using 5-(4-bromobutoxy)pyrazolo[1,5-a]pyridine (C6a) and 1-(4-hydroxyphenyl)piperazine (C7f). Yield: 140 mg (63 %) beige solid.
Mp.: 124°C. MS (APCI): m/z 367 (M+1)⁺. IR (NaCl) v (cm⁻¹): 3136; 2941; 2817; 1648; 1513; 1444; 1289; 1229; 1192; 823. ¹H NMR (CDCl₃, 600 MHz) δ (ppm): 1.72-1.77 (m, 2H, CH₂CH₂N); 1.84-1.89 (m, 2H, OCH₂CH₂); 2.50 (t, J = 7.6 Hz, 2H, CH₂N); 2.65-2.66 (m, 4H, Piperazine); 3.09-3.11 (m, 4H, Piperazine); 4.00 (t, J = 6.3 Hz, 2H, OCH₂); 6.30-6.31 (m, 1H, H-3); 6.43 (dd, J = 7.6 Hz, 2.6 Hz, 1H, H-6); 6.73 (d, J = 2.6 Hz, 1H, H-4); 6.74-6.76 (m, 2H, Phenyl H2, Phenyl H6); 6.83-6.84 (m, 2H, Phenyl H3, Phenyl H5); 7.85 (d, J = 2.1 Hz, 1H, H-2); 8.28 (d, J = 7.6 Hz, 1H, H-7). ¹³C NMR (CDCl₃, 600 MHz) δ (ppm): 23.4; 27.1; 50.7; 53.4; 58.2; 68.0; 95.4; 95.5; 106.9; 116.0; 118.5; 129.3; 141.2; 142.6; 145.4; 150.2; 155.7.

### Example E1:

### 2-[4-[4-(2,3-Dichlorophenyl)piperazin-1-yl]butoxy]pyrazolo[1,5-a]pyridine

Synthesis worked according to the preparation of E15 when using 2-(4-bromobutoxy)pyrazolo[1,5-a]pyridine (C6b) and 1-(2,3-dichlorophenyl)piperazine (C7a). Yield: 291 mg (62 %) beige solid.
Mp.: 161°C. MS (EI): m/z 420 (M+1)⁺, 418 (M-1)⁺. IR (NaCl) v (cm⁻¹): 3074; 2947; 2819; 1635; 1578; 1242; 1144; 1043; 781. ¹H NMR (CDCl₃, 360 MHz) δ (ppm): 1.85-1.92 (m, 4H, OCH₂CH₂CH₂CH₂N); 2.69 (t, J = 6.8 Hz, 2H, CH₂N); 2.84-2.89 (m, 4H, Piperazine); 3.20-3.23 (m, 4H, Piperazine); 4.29 (t, J = 6.0 Hz, 2H, OCH₂); 5.83 (d, J = 0.6 Hz, 1H, H-3); 6.60 (ddd, J = 6.9 Hz, 6.9 Hz, 1.3 Hz, 1H, H-6); 6.97 (dd, J = 7.3 Hz, 2.3 Hz, 1H, Phenyl H6); 7.05 (ddd, J = 8.9 Hz, 6.9 Hz, 1.1 Hz, 1H, H-5); 7.13-7.19 (m, 2H, Phenyl H4, Phenyl H5); 7.29 (ddd, J = 8.9 Hz, 1.3 Hz, 0.6 Hz, 1H, H-4); 8.22 (dd, J = 6.9 Hz, 1.1 Hz, 1H, H-7). ¹³C NMR (CDCl₃, 360 MHz) δ (ppm): 22.7; 27.2; 50.4; 53.1; 58.1; 69.0; 79.8; 109.8; 116.5; 118.9; 124.0; 125.1; 127.6; 128.3; 134.2; 141.7; 150.6; 165.6.

### Example E2:

### 2-[4-[4-(2- Methoxyphenyl)piperazin-1-yl]butoxy]pyrazolo[1,5-a]pyridine

Synthesis worked according to the preparation of E15 when using 2-(4-bromobutoxy)pyrazolo[1,5-a]pyridine (C6b) and 1-(2-methoxyphenyl)piperazine (C7b). Yield: 262 mg (62 %) orange oil.
MS (APCI): m/z 381 (M+1)⁺. IR (NaCl) v (cm⁻¹): 3068; 2941; 2818; 1635; 1502; 1240; 752. ¹H NMR (CDCl₃, 360 MHz) δ (ppm): 1.87-1.92 (m, 4H, OCH₂CH₂CH₂CH₂N); 2.69 (t, J = 7.5 Hz, 2H, CH₂N); 2.86-2.91 (m, 4H, Piperazine); 3.23-3.26 (m, 4H, Piperazine); 3.86 (s, 3H, OCH₃); 4.29 (t, J = 5.7 Hz, 2H, OCH₂); 5.82 (d, J = 0.6 Hz, 1H, H-3); 6.59 (ddd, J = 6.9 Hz, 6.9 Hz, 1.3 Hz, 1H, H-6); 6.86 (dd, J = 7.9, 1.0 Hz, 1H, Phenyl H3); 6.91-6.95 (m, 2H, Phenyl H5, Phenyl H6); 6.99-7.07 (m, 2H, Phenyl H4, H-5); 7.29 (ddd, J = 8.9 Hz, 1.3 Hz, 0.6 Hz, 1H, H-4); 8.22 (dd, J = 6.9 Hz, 1.1 Hz, 1H, H-7). ¹³C NMR (CDCl₃, 360 MHz) δ (ppm): 22.7; 27.2; 49.6; 53.1; 55.4; 58.0; 69.0; 79.8; 109.8; 111.4; 116.4; 118.5; 121.2; 123.4; 124.0; 128.4; 140.8; 141.7; 152.3; 165.7.

### Example E3:

### 2-[4-[4-(2-Chlorophenyl)piperazin-1-yl]butoxy]pyrazolo[1,5-a]pyridine

Synthesis worked according to the preparation of E15 when using 2-(4-bromobutoxy)pyrazolo[1,5-a]pyridine (C6b) and 1-(2-chlorphenyl)piperazine (C7c).
Yield: 237 mg (55 %) yellow oil.
MS (EI): m/z 386 (M+1)⁺, 384 (M-1)⁺. IR (NaCl) v (cm⁻¹): 3059; 2945; 2818; 1635; 1504; 1259; 1228; 770. ¹H NMR (CDCl₃, 360 MHz) δ (ppm): 1.86-1.93 (m, 4H, OCH₂CH₂CH₂CH₂N); 2.70 (t, J = 7.0 Hz, 2H, CH₂N); 2.84-2.90 (m, 4H, Piperazine); 3.22-3.24 (m, 4H, Piperazine); 4.29 (t, J = 5.7 Hz, 2H, OCH₂); 5.83 (d, J = 0.6 Hz, 1H, H-3); 6.59 (ddd, J = 6.9 Hz, 6.9 Hz, 1.4 Hz, 1H, H-6); 6.98 (ddd, J = 7.8 Hz, 7.3 Hz, 1.3 Hz, 1H, Phenyl H4); 7.02-7.07 (m, 2H, Phenyl H6, H-5); 7.22 (ddd, J = 8.0 Hz, 7.3 Hz, 1.2 Hz, 1H, Phenyl H5); 7.29 (ddd, J = 8.9 Hz, 1.4 Hz, 0.6 Hz, 1H, H-4); 7.35 (dd, J = 7.8 Hz, 1.2 Hz, 1H, Phenyl H3); 8.22 (dd, J = 6.9 Hz, 1.1 Hz, 1H, H-7). ¹³C NMR (CDCl₃, 360 MHz) δ (ppm): 22.7; 27.2; 50.2; 53.1; 58.0; 69.0; 79.8; 109.8; 116.4; 120.6; 123.9; 124.1; 127.7; 128.3; 128.8; 130.6; 141.6; 148.7; 165.7.

### Example E7:

### 4-[4-[4-(2,3-Dichlorophenyl)piperazin-1-yl]butoxy]pyrazolo[1,5-a]pyridine

Synthesis worked according to the preparation of E15 when using 4-(4-bromobutoxy)pyrazolo[1,5-a]pyridine (C6c) and 1-(2,3-dichlorophenyl)piperazine (C7a). Yield: 741 mg (68 %) beige solid.
Mp.: 91 °C. MS (APCI): m/z 421 (M+2)⁺, 419 (M)⁺. IR (NaCl) v (cm⁻¹): 3062; 2946; 2817; 1578; 1548; 1277; 1241; 1093; 748. ¹H NMR (CDCl₃, 600 MHz) δ (ppm): 1.77-1.81 (m, 2H, CH₂CH₂N); 1.92-1.97 (m, 2H, OCH₂CH₂); 2.55 (t, J = 7.6 Hz, 2H, CH₂N); 2.67-2.71 (m, 4H, Piperazine); 3.07-3.11 (m, 4H, Piperazine); 4.14 (t, J = 6.3 Hz, 2H, OCH₂); 6.35 (d, J = 7.6 Hz, 1H, H-5); 6.63-6.65 (m, 2H, H-3, H-6); 6.95 (dd, J = 7.3 Hz, 2.4 Hz, 1H, Phenyl H6); 7.12-7.17 (m, 2H, Phenyl H4, Phenyl H5); 7.87 (d, J = 2.4 Hz, 1H, H-2); 8.12 (d, J = 6.9 Hz, 1H, H-7). ¹³C NMR (CDCl₃, 360 MHz) δ (ppm): 23.4; 27.0; 51.2; 53.3; 58.1; 68.2; 95.3; 99.7; 111.4; 118.6; 121.7; 124.7; 127.4; 127.5; 134.1; 135.4; 140.7; 150.4; 151.2. C H N (%):C₂₁H₂₄N₄OCl₂ x 0.5H₂O; calculated: C 58.88; H 5.88; N 13.08; found: C 58.81; H 5.61; N 12.96.

### Example E8:

### 4-[4-[4-(2-Methoxyphenyl)piperazin-1-yl]butoxy]pyrazolo[1,5-a]pyridine

Synthesis worked according to the preparation of E15 when using 4-(4-bromobutoxy)pyrazolo[1,5-a]pyridine (C6c) and 1-(2-methoxyphenyl)piperazine (C7b). Yield: 801 mg (81 %) beige solid.
Mp.: 74°C. MS (APCI): m/z 381 (M+1)⁺. IR (NaCl) v (cm⁻¹): 3061; 2942; 2814; 1548; 1500; 1276; 1241; 747. ¹H NMR (CDCl₃, 360 MHz) δ (ppm): 1.73-1.81 (m, 2H, CH₂CH₂N); 1.90-1.97 (m, 2H, OCH₂CH₂); 2.52 (t, J = 7.6 Hz, 2H, CH₂N); 2.65-2.70 (m, 4H, Piperazine); 3.09-3.13 (m, 4H, Piperazine); 3.86 (s, 3H, OCH₃); 4.13 (t, J = 6.3 Hz, 2H, OCH₂); 6.35 (d, J = 7.6 Hz, 1H, H-5); 6.61-6.65 (m, 2H, H-3, H-6); 6.86 (dd, J = 7.9 Hz, 1.0 Hz, 1H, Phenyl H3); 6.89-6.95 (m, 2H, Phenyl H5, Phenyl H6); 6.99 (ddd, J = 7.9 Hz, 7.0 Hz, 2.2 Hz, 1H, Phenyl H4); 7.87 (d, J = 2.4 Hz, 1H, H-2); 8.12 (d, J = 6.9 Hz, 1H, H-7). ¹³C NMR (CDCl₃, 600 MHz) δ (ppm): 23.5; 27.1; 50.7; 53.5; 55.4; 58.3; 68.3; 95.3; 99.7; 111.2; 111.4; 118.2; 121.0; 121.7; 122.9; 135.4; 140.7; 141.4; 150.4; 152.3.
C H N (%):C₂₂H₂₈N₄O₂ x 0.1H₂O; calculated: C 69.12; H 7.44; N 14.66; found: C 68.95; H 7.29; N 14.60.

### Example E9:

### 4-[4-[4-(2-Chlorophenyl)piperazin-1-yl]butoxy]pyrazolo[1,5-a]pyridine

Synthesis worked according to the preparation of E15 when using 4-(4-bromobutoxy)pyrazolo[1,5-a]pyridine (C6c) and 1-(2-chlorphenyl)piperazine (C7c).
Yield: 785 mg (78 %) orange solid.
Mp.: 88°C. MS (APCl): m/z 387 (M+2)⁺, 385 (M)⁺. IR (NaCl) v (cm⁻¹): 3061; 2945; 2815; 1548; 1517; 1479; 1276; 1231; 748. ¹H NMR (CDCl₃, 600 MHz) δ (ppm): 1.76-1.81 (m, 2H, CH₂CH₂N); 1.92-1.97 (m, 2H, OCH₂CH₂); 2.54 (t, J = 7.6 Hz, 2H, CH₂N); 2.64-2.72 (m, 4H, Piperazine); 3.07-3.13 (m, 4H, Piperazine); 4.14 (t, J = 6.3 Hz, 2H, OCH₂); 6.35 (d, J = 7.6 Hz, 1H, H-5); 6.62-6.65 (m, 2H, H-3, H-6); 6.96 (ddd, J = 7.8 Hz, 7.3 Hz, 1.3 Hz, 1H, Phenyl H4); 7.05 (dd, J = 8.0 Hz, 1.3 Hz, 1H, Phenyl H6); 7.22 (ddd, J = 8.0 Hz, 7.3 Hz, 1.2 Hz, 1H, Phenyl H5); 7.35 (dd, J = 7.8 Hz, 1.2 Hz, 1H, Phenyl H3); 7.87 (d, J = 2.4 Hz, 1H, H-2); 8.12 (d, J = 6.9 Hz, 1H, H-7). ¹³C NMR (CDCl₃, 600 MHz) δ (ppm): 23.5; 27.1; 51.2; 53.4; 58.3; 68.3; 95.3; 99.8; 111.4; 120.4; 121.7; 123.7; 127.6; 128.8; 130.7; 135.4; 140.7; 149.3; 150.4.
C H N (%):C₂₁H₂₅N₄OCl x 0.6H₂O; calculated: C 63.74; H 6.67; N 14.16; found: C 63.92; H 6.53; N 13.98.

### Example E51:

### 6-[4-[4-(2,3-Dichlorophenyl)piperazin-1-yl]butoxy]pyrazolo[1,5-a]pyridine

Synthesis worked according to the preparation of E15 when using 6-(4-bromobutoxy)pyrazolo[1,5-a]pyridine (C6d) and 1-(2,3-dichlorophenyl)piperazine (C7a). Yield: 43 mg (69 %) beige solid.
Mp.: 97°C. MS (APCI): m/z 421 (M+2)⁺, 419 (M)⁺. IR (NaCl) v (cm⁻¹): 3106; 2944; 2811; 1647; 1578; 1291; 1240; 1199; 1043; 781. ¹H NMR (CDCl₃, 360 MHz) δ (ppm): 1.72-1.81 (m, 2H, CH₂CH₂N); 1.85-1.93 (m, 2H, OCH₂CH₂); 2.54 (t, J = 7.6 Hz, 2H, CH₂N); 2.68-2.73 (m, 4H, Piperazine); 3.10-3.12 (m, 4H, Piperazine); 4.00 (t, J = 6.3 Hz, 2H, OCH₂); 6.44-6.45 (m, 1H, H-3); 6.92 (dd, J = 9.6 Hz, 2.2 Hz, 1H, H-5); 6.96 (dd, J = 6.9 Hz, 2.6 Hz, 1H, Phenyl H6); 7.14-7.16 (m, 2H, Phenyl H4, Phenyl H5); 7.41 (d, J = 9.6 Hz, 1H, H-4); 7.84 (d, J = 2.2 Hz, 1H, H-7); 8.07-8.08 (m, 1H, H-2). ¹³C NMR (CDCl₃, 360 MHz) δ (ppm): 23.2; 27.1; 51.1; 53.3; 58.1; 68.7; 96.7; 111.8; 117.9; 118.7; 119.1; 124.7; 127.5; 127.6; 134.1; 136.5; 140.8; 148.3; 151.2.
C H N (%):C₂₁H₂₄N₄OCl₂ x 0.6H₂O; calculated: C 58.64; H 5.91; N 13.03; found: C 58.86; H 5.81; N 12.78.

### Example E52:

### 6-[4-[4-(2-Methoxyphenyl)piperazin-1-yl]butoxy]pyrazolo[1,5-a]pyridine

Synthesis worked according to the preparation of E15 when using 6-(4-bromobutoxy)pyrazolo[1,5-a]pyridine (C6d) and 1-(2-methoxyphenyl)piperazine (C7b). Yield: 50 mg (89 %) beige oil.
MS (EI): m/z 380 (M)⁺. IR (NaCl) v (cm⁻¹): 3108; 2942; 2817; 1643; 1499; 1292; 1240; 1199; 748. ¹H NMR (CDCl₃, 600 MHz) δ (ppm): 1.80-1.85 (m, 2H, CH₂CH₂N); 1.87-1.91 (m, 2H, OCH₂CH₂); 2.59 (t, J = 7.6 Hz, 2H, CH₂N); 2.77-2.82 (m, 4H, Piperazine); 3.17-3.22 (m, 4H, Piperazine); 3.86 (s, 3H, OCH₃); 3.99 (t, J = 6.3 Hz, 2H, OCH₂); 6.45 (d, J = 2.1 Hz, 1H, H-3); 6.87 (dd, J = 7.9 Hz, 1.0 Hz, 1H, Phenyl H3); 6.91-6.93 (m, 2H, H-5, Phenyl H5); 6.95 (dd, J = 7.5 Hz, 2.0 Hz, 1H, Phenyl H6); 7.01 (ddd, J = 7.9 Hz, 7.0 Hz, 2.0 Hz, 1H, Phenyl H4); 7.41 (d, J = 9.6 Hz, 1H, H-4); 7.84 (d, J = 2.2 Hz, 1H, H-7); 8.06-8.07 (m, 1H, H-2). ¹³C NMR (CDCl₃, 600 MHz) δ (ppm): 23.1; 27.1; 50.2; 53.4; 55.4; 58.1; 68.7; 96.7; 111.2; 111.8; 117.9; 118.4; 119.0; 121.1; 123.2; 136.5; 140.8; 141.0; 148.3; 152.3.

### Example E53:

### 6-[4-[4-(2-Chlorophenyl)piperazin-1-yl]butoxy]pyrazolo[1,5-a]pyridine

Synthesis worked according to the preparation of E15 when using 6-(4-bromobutoxy)pyrazolo[1,5-a]pyridine (C6d) and 1-(2-chlorphenyl)piperazine (C7c).
Yield: 164 mg (81 %) beige solid.
Mp.: 86°C. MS (APCl): m/z 387 (M+2)⁺, 385 (M)⁺. IR (NaCl) v (cm⁻¹): 3088; 2943; 2818; 1643; 1525; 1479; 1291; 1233; 1199; 750. ¹H NMR (CDCl₃, 600 MHz) δ (ppm): 1.78-1.83 (m, 2H, CH₂CH₂N); 1.86-1.91 (m, 2H, OCH₂CH₂); 2.58 (t, J = 7.6 Hz, 2H, CH₂N); 2.74-2.78 (m, 4H, Piperazine); 3.14-3.18 (m, 4H, Piperazine); 3.99 (t, J = 6.3 Hz, 2H, OCH₂); 6.44-6.45 (m, 1H, H-3); 6.92 (dd, J = 9.6 Hz, 2.2 Hz, 1H, H-5); 6.97 (ddd, J = 7.8 Hz, 7.3 Hz, 1.3 Hz, 1H, Phenyl H4); 7.05 (dd, J = 8.0 Hz, 1.3 Hz, 1H, Phenyl H6); 7.22 (ddd, J = 8.0 Hz, 7.3 Hz, 1.2 Hz, 1H, Phenyl H5); 7.35 (dd, J = 7.8 Hz, 1.2 Hz, 1H, Phenyl H3); 7.41 (d, J = 9.6 Hz, 1H, H-4); 7.84 (d, J = 2.2 Hz, 1H, H-7); 8.07-8.08 (m, 1H, H-2). ¹³C NMR (CDCl₃, 600 MHz) δ (ppm): 23.1; 27.0; 50.8; 53.3; 58.1; 68.6; 96.7; 111.7; 117.9; 119.0; 120.5; 123.9; 127.7; 128.8; 130.7; 136.5; 140.8; 148.3; 149.1.

### Example E57:

### 7-[4-[4-(2,3-Dichlorophenyl)piperazin-1-yl]butoxy]pyrazolo[1,5-a]pyridine

Synthesis worked according to the preparation of E15 when using 7-(4-bromobutoxy)pyrazolo[1,5-a]pyridine (C6e) and 1-(2,3-dichlorophenyl)piperazine (C7a). Yield: 117 mg (75 %) light blue solid.
Mp.: 74°C. MS (APCI): m/z 421 (M+2)⁺, 419 (M)⁺. IR (NaCl) v (cm⁻¹): 3058; 2951; 1633; 1547; 1103; 781. ¹H NMR (CDCl₃, 600 MHz) δ (ppm): 2.03-2.06 (m, 2H, CH₂CH₂N); 2.08-2.11 (m, 2H, OCH₂CH₂); 2.97 (t, J = 7.6 Hz, 2H, CH₂N); 3.02-3.09 (m, 4H, Piperazine); 3.28-3.30 (m, 4H, Piperazine); 4.36 (t, J = 6.1 Hz, 2H, OCH₂); 6.10 (dd, J = 7.3 Hz, 0.6 Hz, 1H, H-6); 6.52 (d, J = 2.3 Hz, 1H, H-3); 6.98 (dd, J = 7.8 Hz, 1.5 Hz, 1H, Phenyl H6); 7.10 (dd, J = 8.4 Hz, 7.3 Hz, 1H, H-5); 7.17 (dd, J = 8.4 Hz, 0.6 Hz, 1H, H-4); 7.19-7.21 (m, 2H, Phenyl H4, Phenyl H5); 7.98 (d, J = 2.3 Hz, 1H, H-2). ¹³C NMR (CDCl₃, 600 MHz) δ (ppm): 22.4; 26.3; 46.9; 52.8; 57.6; 69.7; 89.4; 96.9; 110.2; 119.0; 124.6; 125.4; 127.6; 127.7; 134.1; 142.2; 150.3; 150.6.

### Example E58:

### 7-[4-[4-(2-Methoxyphenyl)piperazin-1-yl]butoxy]pyrazolo[1, 5-a]pyridine

Synthesis worked according to the preparation of E15 when using 7-(4-bromobutoxy)pyrazolo[1,5-a]pyridine (C6e) and 1-(2-methoxyphenyl)piperazine (C7b). Yield: 112 mg (79 %) light blue oil.
MS (APCI): m/z 381 (M+1)⁺. IR (NaCl) v (cm⁻¹): 2937; 1632; 1547; 1500; 1244; 1022; 756. ¹H NMR (CDCl₃, 360 MHz) δ (ppm): 2.10-2.17 (m, 2H, CH₂CH₂N); 2.34-2.42 (m, 2H, OCH₂CH₂); 3.36-3.45 (m, 6H, CH₂N, Piperazine); 3.56-3.61 (m, 4H, Piperazine); 3.87 (s, 3H, OCH₃); 4.39 (t, J = 5.7 Hz, 2H, OCH₂); 6.12 (dd, J = 7.3 Hz, 1.1 Hz, 1H, H-6); 6.53 (d, J = 2.2 Hz, 1H, H-3); 6.89 (dd, J = 7.9 Hz, 0.9 Hz, 1H, Phenyl H3); 6.93-6.96 (m, 2H, Phenyl H5, Phenyl H6); 7.07 (ddd, J = 7.9 Hz, 6.0 Hz, 2.7 Hz, 1H, Phenyl H4); 7.13 (dd, J = 8.5 Hz, 7.3 Hz, 1H, H-5); 7.22 (dd, J = 8.5 Hz, 1.1 Hz, 1H, H-4); 7.97 (d, J = 2.2 Hz, 1H, H-2). ¹³C NMR (CDCl₃, 360 MHz) δ (ppm): 21.3; 26.1; 47.1; 52.4; 55.5; 57.2; 69.3; 89.8; 97.1; 110.5; 111.5; 119.1; 121.3; 124.6; 138.8; 142.1; 150.3; 152.1.

### Example E59:

### 7-[4-[4-(2-Chlorophenyl)piperazin-1-yl]butoxy]pyrazolo[1,5-a]pyridine

Synthesis worked according to the preparation of E15 when using 7-(4-bromobutoxy)pyrazolo[1,5-a]pyridine (C6e) and 1-(2-chlorphenyl)piperazine (C7c).
Yield: 90 mg (63 %) light blue oil.
MS (APCl): m/z 387 (M+2)⁺, 385 (M)⁺. IR (NaCl) v (cm⁻¹): 3059; 2926; 1632; 1545; 1219; 1101; 771. ¹H NMR (CDCl₃, 360 MHz) δ (ppm): 2.10-2.17 (m, 2H, CH₂CH₂N); 2.29-2.38 (m, 2H, OCH₂CH₂); 3.37-3.48 (m, 6H, CH₂N, Piperazine); 3.52-3.58 (m, 4H, Piperazine); 4.39 (t, J = 5.7 Hz, 2H, OCH₂); 6.11 (dd, J = 7.3 Hz, 0.8 Hz, 1H, H-6); 6.53 (d, J = 2.2 Hz, 1H, H-3); 7.06 (ddd, J = 7.8 Hz, 7.3 Hz, 1.3 Hz, 1H, Phenyl H4); 7.10 (dd, J = 8.0 Hz, 1.3 Hz, 1H, Phenyl H6); 7.12 (dd, J = 8.9 Hz, 7.3 Hz, 1H, H-5); 7.18 (ddd, J = 8.0 Hz, 7.3 Hz, 1.2 Hz, 1H, Phenyl H5); 7.22 (dd, J = 8.9 Hz, 0.8 Hz, 1H, H-4); 7.38 (dd, J = 7.8 Hz, 1.2 Hz, 1H, Phenyl H3); 7.98 (d, J = 2.2 Hz, 1H, H-2). ¹³C NMR (CDCl₃, 360 MHz) δ (ppm): 21.6; 26.1; 48.1; 52.5; 57.4; 69.5; 89.7; 97.2; 110.5; 121.1; 124.6; 125.3; 128.1; 128.9; 130.7; 142.1; 147.1; 150.3.

### Example E13:

### 5-[3-[4-(2,3-Dichlorophenyl)piperazin-1-yl]propoxymethyl]pyrazolo[1,5-a]pyridine

Synthesis worked according to the preparation of E15 when using 5-(3-bromopropoxymethyl)pyrazolo[1,5-a]pyridine (C6f) and 1-(2,3-dichlorophenyl)piperazine (C7a).
Yield: 58 mg (99 %) yellow solid.
Mp.: 69°C. MS (EI): m/z 420 (M+1)⁺, 418 (M)⁺. IR (NaCl) v (cm⁻¹): 2945; 2819; 1647; 1578; 1241; 1044; 780. ¹H NMR (CDCl₃, 360 MHz) δ (ppm): 1.84-1.92 (m, 2H, CH₂CH₂N); 2.55 (t, J = 6.7 Hz, 2H, CH₂N); 2.63-2.68 (m, 4H, Piperazin); 3.05-3.08 (m, 4H, Piperazin); 3.59 (t, J = 6.4 Hz, 2H, OCH₂CH₂); 4.52-4.53 (m, 2H, CH₂O); 6.47 (dd, J = 2.4 Hz, 0.9 Hz, 1H, H-3); 6.73 (dd, J = 7.1 Hz, 1.8 Hz, 1H, H-6); 6.95 (dd, J = 6.7 Hz, 3.0 Hz, 1H, Phenyl H6); 7.13-7.15 (m, 2H, Phenyl H4, Phenyl H5); 7.48 (dd, J = 1.8 Hz, 0.9 Hz, 1H, H-4); 7.93 (d, J = 2.4 Hz, 1H, H-2); 8.43 (d, J = 7.1 Hz, 1H, H-7). ¹³C NMR (CDCl₃, 360 MHz) δ (ppm): 27.2; 51.4; 53.4; 55.5; 69.0; 71.9; 96.8; 111.4; 115.6; 118.6; 124.7; 127.4; 127.6; 128.5; 134.1; 134.5; 139.9; 142.2; 151.3.

### Example E27:

### 1-(5-[4-[4-(2,3-Dichlorophenyl)piperazin-1-yl]butoxy]pyrazolo[1,5-a]pyridine-3-yl)ethanone

Synthesis worked according to the preparation of E17 when using 1-[5-(4-brombutoxy)pyrazolo[1,5-a]pyridine-3-yl]ethanone (C6g) and 1-(2,3-dichlorophenyl)piperazine (C7a).
Yield: 12 mg (53 %) orange oil.
MS (APCl): m/z 463 (M+2)⁺, 461 (M)⁺. IR (NaCl) v (cm⁻¹): 3097; 2947; 2819; 1655; 1524; 1277; 1217; 1043; 783. ¹H NMR (CDCl₃, 600 MHz) δ (ppm): 1.72-1.77 (m, 2H, CH₂CH₂N); 1.89-1.94 (m, 2H, OCH₂CH₂); 2.50-2.53 (m, 5H, CH₂N, COCH₃); 2.64-2.70 (m, 4H, Piperazine); 3.06-3.11 (m, 4H, Piperazine); 4.15 (t, J = 6.4 Hz, 2H, OCH₂); 6.66 (dd, J = 7.6 Hz, 2.6 Hz, 1H, H-6); 6.97 (dd, J = 7.1 Hz, 2.2 Hz, 1H, Phenyl H6); 7.12-7.17 (m, 2H, Phenyl H4, Phenyl H5); 7.68 (d, J = 2.6 Hz, 1H, H-4); 8.24 (s, 1H, H-2); 8.33 (d, J = 7.6 Hz, 1H, H-7). ¹³C NMR (CDCl₃, 360 MHz) δ (ppm): 23.3; 26.9; 27.8; 51.3; 53.4; 58.1; 68.8; 98.0; 109.1; 112.4; 118.7; 124.6; 127.5; 127.6; 129.8; 134.1; 142.1; 145.7; 151.3; 160.1; 191.7.

### Example E28:

### 1-(5-[4-[4-(2-Methoxyphenyl)piperazin-1-yl]butoxy]pyrazolo[1,5-a]pyridine-3-yl)ethanone

Synthesis worked according to the preparation of E17 when using 1-[5-(4-brombutoxy)pyrazolo[1 ,5-a]pyridine-3-yl]ethanone (C6g) and 1-(2-methoxyphenyl)piperazine (C7b).
Yield: 17 mg (78 %) orange oil.
MS (APCI): m/z 423 (M+1)⁺. IR (NaCl) v (cm⁻¹): 3064; 2943; 2812; 1653; 1524; 1275; 1240; 1217; 750. ¹H NMR (CDCl₃, 600 MHz) δ (ppm): 1.78-1.83 (m, 2H, CH₂CH₂N); 1.89-1.94 (m, 2H, OCH₂CH₂); 2.52 (s, 3H, COCH₃); 2.58 (t, J = 7.6 Hz, 2H, CH₂N); 2.73-2.81 (m, 4H, Piperazine); 3.14-3.21 (m, 4H, Piperazine); 3.87 (s, 3H, OCH₃); 4.15 (t, J = 6.3 Hz, 2H, OCH₂); 6.67 (dd, J = 7.6 Hz, 2.6 Hz, 1H, H-6); 6.86 (dd, J = 7.9 Hz, 1.0 Hz, 1H, Phenyl H3); 6.92 (ddd, J = 7.5 Hz, 7.0 Hz, 1.0 Hz, 1H, Phenyl H5); 6.95 (dd, J = 7.5 Hz, 2.0 Hz, 1H, Phenyl H6); 7.01 (ddd, J = 7.9 Hz, 7.0 Hz, 2.0 Hz, 1H, Phenyl H4); 7.68 (d, J = 2.6 Hz, 1H, H-4); 8.24 (s, 1H, H-2); 8.33 (d, J = 7.6 Hz, 1H, H-7). ¹³C NMR (CDCl₃, 360 MHz) δ (ppm): 23.1; 26.8; 27.7; 50.3; 53.4; 55.4; 58.1; 68.6; 98.0; 109.0; 111.3; 112.4; 118.4; 121.1; 123.1; 129.8; 141.1; 142.1; 145.6; 152.3; 160.2; 191.7.

### Example E29:

### 1-(5-[4-[4-(2-Chlorophenyl)piperazin-1-yl]butoxy]pyrazolo[1,5-a]pyridine-3-yl)ethanone

Synthesis worked according to the preparation of E17 when using 1-[5-(4-brombutoxy)pyrazolo[1,5-a]pyridine-3-yl]ethanone (C6g) and 1-(2-chlorophenyl)piperazine (C7c).
Yield: 13 mg (51 %) orange oil.
MS (APCl): m/z 429 (M+2)⁺, 427 (M)⁺. IR (NaCl) v (cm⁻¹): 3064; 2941; 2816; 1655; 1525; 1277; 1219; 1039; 760. ¹H NMR (CDCl₃, 600 MHz) δ (ppm): 1.74-1.79 (m, 2H, CH₂CH₂N); 1.89-1.94 (m, 2H, OCH₂CH₂); 2.52 (s, 3H, COCH₃); 2.54 (t, J = 7.6 Hz, 2H, CH₂N); 2.66-2.74 (m, 4H, Piperazine); 3.10-3.15 (m, 4H, Piperazine); 4.15 (t, J = 6.3 Hz, 2H, OCH₂); 6.66 (dd, J = 7.6 Hz, 2.6 Hz, 1H, H-6); 6.97 (ddd, J = 7.8 Hz, 7.3 Hz, 1.3 Hz, 1H, Phenyl H4); 7.06 (dd, J = 8.0 Hz, 1.3 Hz, 1H, Phenyl H6); 7.22 (ddd, J = 8.0 Hz, 7.3 Hz, 1.5 Hz, 1H, Phenyl H5); 7.35 (dd, J = 7.8 Hz, 1.5 Hz, 1H, Phenyl H3); 7.68 (d, J = 2.6 Hz, 1H, H-4); 8.24 (s, 1H, H-2); 8.33 (d, J = 7.6 Hz, 1H, H-7). ¹³C NMR (CDCl₃, 600 MHz) δ (ppm): 23.3; 26.9; 27.8; 51.1; 53.3; 58.1; 68.7; 98.0; 109.1; 112.4; 120.5; 123.7; 127.6; 128.8; 129.8; 130.6; 142.1; 145.7; 149.2; 160.0; 191.8.

### Example E39:

### Methyl 5-[4-[4-(2,3-dichlorophenyl)piperazin-1-yl]butoxy]pyrazolo[1,5-a]pyridine-3-carboxylate

Synthesis worked according to the preparation of E17 when using methyl 5-(4-bromobutoxy)pyrazolo[1,5-a]pyridine-3-carboxylate (C6h) and 1-(2,3-dichlorophenyl)piperazine (C7a).
Yield: 42 mg (58 %) beige solid.
Mp.: 73°C. MS (APCI): m/z 479 (M+2)⁺, 477 (M)⁺. IR (NaCl) v (cm⁻¹): 3070; 2945; 2816; 1699; 1647; 1275; 1213; 1047; 777. ¹H NMR (CDCl₃, 600 MHz) δ (ppm): 1.72-1.77 (m, 2H, CH₂CH₂N); 1.89-1.94 (m, 2H, OCH₂CH₂); 2.52 (t, J = 7.5 Hz, 2H, CH₂N); 2.64-2.70 (m, 4H, Piperazine); 3.06-3.11 (m, 4H, Piperazine); 3.89 (s, 3H, COOCH₃); 4.13 (t, J = 6.5 Hz, 2H, OCH₂); 6.61 (dd, J = 7.6 Hz, 2.6 Hz, 1H, H-6); 6.96 (dd, J = 7.3 Hz, 2.2 Hz, 1H, Phenyl H6); 7.13-7.16 (m, 2H, Phenyl H4, Phenyl H5); 7.41 (d, J = 2.6 Hz, 1H, H-4); 8.27 (s, 1H, H-2); 8.32 (d, J = 7.6 Hz, 1H, H-7). ¹³C NMR (CDCl₃, 360 MHz) δ (ppm): 23.4; 26.9; 51.0; 51.4; 53.3; 58.1; 68.6; 96.8; 102.3; 108.4; 118.7; 124.6; 127.4; 127.5; 130.1; 134.1; 142.9; 145.3; 151.3; 159.0; 164.1.

### Example E40:

### Methyl 5-[4-[4-(2-methoxyphenyl)piperazin-1-yl]butoxy]pyrazolo[1,5-a]pyridine-3-carboxylate

Synthesis worked according to the preparation of E17 when using methyl 5-(4-bromobutoxy)pyrazolo[1,5-a]pyridine-3-carboxylate (C6h) and 1-(2-methoxyphenyl)piperazine (C7b).
Yield: 58 mg (87 %) beige solid.
Mp.: 75°C. MS (APCI): m/z 439 (M+1)⁺. IR (NaCl) v (cm⁻¹): 3064; 2945; 2816; 1699; 1647; 1539; 1275; 1242; 1213; 748. ¹H NMR (CDCl₃, 360 MHz) δ (ppm): 1.72-1.80 (m, 2H, CH₂CH₂N); 1.87-1.95 (m, 2H, OCH₂CH₂); 2.53 (t, J = 7.6 Hz, 2H, CH₂N); 2.68-2.74 (m, 4H, Piperazine); 3.10-3.17 (m, 4H, Piperazine); 3.86 (s, 3H, OCH₃); 3.89 (s, 3H, COOCH₃); 4.13 (t, J = 6.2 Hz, 2H, OCH₂); 6.60 (dd, J = 7.6 Hz, 2.6 Hz, 1H, H-6); 6.86 (dd, J = 8.0 Hz, 1.2 Hz, 1H, Phenyl H3); 6.89-6.96 (m, 2H, Phenyl H5, Phenyl H6); 7.00 (ddd, J = 8.0 Hz, 6.8 Hz, 2.4 Hz, 1H. Phenyl H4); 7.40 (d, J = 2.6 Hz, 1H, H-4); 8.27 (s, 1H, H-2); 8.31 (d, J = 7.6 Hz, 1H, H-7). ¹³C NMR (CDCl₃, 360 MHz) δ (ppm): 23.3; 26.9; 50.5; 51.0; 53.5; 55.4; 58.2; 68.6; 96.8; 102.3; 108.4; 111.3; 118.3; 121.0; 123.0; 130.0; 141.3; 142.9; 145.3; 152.3; 159.0; 164.1.

### Example E41:

### Meth yl 5-[4-[4-(2-chlorophenyl)piperazin-1-yl]butoxy]pyrazolo[1,5-a]pyridine-3-carboxylate

Synthesis worked according to the preparation of E17 when using methyl 5-(4-bromobutoxy)pyrazolo[1 ,5-a]pyridine-3-carboxylate (C60h) and 1-(2-chlorophenyl)piperazine (C7c).
Yield: 49 mg (72 %) orange oil.
MS (APCI): m/z 445 (M+2)⁺, 443 (M)⁺. IR (NaCl) v (cm⁻¹): 3064; 2948; 2817; 1699; 1648; 1539; 1276; 1213; 1048; 751. ¹H NMR (CDCl₃, 600 MHz) δ (ppm): 1.79-1.84 (m, 2H, CH₂CH₂N); 1.90-1.94 (m, 2H, OCH₂CH₂); 2.60 (t, J = 7.6 Hz, 2H, CH₂N); 2.73-2.81 (m, 4H, Piperazine); 3.14-3.20 (m, 4H, Piperazine); 3.89 (s, 3H, COOCH₃); 4.13 (t, J = 6.2 Hz, 2H, OCH₂); 6.61 (dd, J = 7.6 Hz, 2.6 Hz, 1H, H-6); 6.98 (ddd, J = 7.8 Hz, 7.3 Hz, 1.3 Hz, 1H, Phenyl H4); 7.06 (dd, J = 8.0 Hz, 1.3 Hz, 1H, Phenyl H6); 7.22 (ddd, J = 8.0 Hz, 7.3 Hz, 1.5 Hz, 1H, Phenyl H5); 7.36 (dd, J = 7.8 Hz, 1.5 Hz, 1H, Phenyl H3); 7.41 (d, J = 2.6 Hz, 1H, H-4); 8.28 (s, 1H, H-2); 8.32 (d, J = 7.6 Hz, 1H, H-7). ¹³C NMR (CDCl₃, 600 MHz) δ (ppm): 23.1; 26.8; 50.8; 51.0; 53.3; 58.0; 68.4; 96.8; 102.3; 108.4; 120.5; 123.9; 127.7; 128.8; 130.0; 130.7; 142.8; 145.3; 149.0; 159.0; 164.1.

### Example E63:

### 7-[4-[4-(2,3-Dichlorophenyl)piperazin-1-yl]butoxy]tetrazolo[1,5-a]pyridine

Synthesis worked according to the preparation of E15 when using 7-(4-bromobutoxy)tetrazolo[1,5-a]pyridine (C6i) and 1-(2,3-dichlorophenyl)piperazine (C7a). Yield: 15 mg (62 %) beige solid.
Mp.: 138°C. MS (APCI) m/z 423 (M+2)⁺, 421 (M)⁺. IR (NaCl) v (cm⁻¹): 3066; 2947; 2819; 1643; 1578; 1242; 1203; 779. ¹H NMR (CDCl₃, 360 MHz) δ (ppm): 1.71-1.80 (m, 2H, CH₂CH₂N); 1.91-1.99 (m, 2H, OCH₂CH₂); 2.52 (t, J = 7.4 Hz, 2H, CH₂N); 2.64-2.69 (m, 4H, Piperazine); 3.07-3.10 (m, 4H, Piperazine); 4.16 (t, J = 6.4 Hz, 2H, OCH₂); 6.86 (dd, J = 7.5 Hz, 2.4 Hz, 1H, H-6); 6.96 (dd, J = 6.4 Hz, 3.1 Hz, 1H, Phenyl H6); 7.14-7.17 (m, 3H, H-8, Phenyl H4, Phenyl H5); 8.60 (dd, J = 7.5 Hz, 0.7 Hz, 1H, H-5). ¹³C NMR (CDCl₃, 360 MHz) δ (ppm): 23.2; 26.6; 51.4; 53.4; 58.0; 69.3; 92.3; 112.2; 118.7; 124.7; 125.4; 127.5; 127.6; 134.1; 150.5; 151.2; 161.7.

### Example E97:

### 7-[4-[4-(2-Methoxyphenyl)piperazin-1-yl]butoxy]tetrazolo[1,5-a]pyridine

Synthesis worked according to the preparation of E15 when using 5-(4-bromobutoxy)tetrazolo[1,5-a]pyridine (C6i) and 1-(2-methoxyphenyl)piperazine (C7b). Yield: 19 mg (85 %) beige solid.
Mp.: 124°C. MS (APCI) m/z 383 (M+1)⁺. IR (NaCl) v (cm⁻¹): 3062; 2943; 2816; 1643; 1500; 1242; 1203; 742. ¹H NMR (CDCl₃, 360 MHz) δ (ppm): 1.77-1.85 (m, 2H, CH₂CH₂N); 1.91-1.99 (m, 2H, OCH₂CH₂); 2.57 (t, J = 7.6 Hz, 2H, CH₂N); 2.72-2.78 (m, 4H, Piperazine); 3.13-3.19 (m, 4H, Piperazine); 3.86 (s, 3H, OCH₃); 4.16 (t, J = 6.4 Hz, 2H, OCH₂); 6.85-6.88 (m, 2H, Phenyl H3, H-6); 6.91-6.96 (m, 2H, Phenyl H5, Phenyl H6); 7.01 (ddd, J = 9.1 Hz, 6.6 Hz, 2.6 Hz, 1H, Phenyl H4); 7.15 (dd, J = 2.4 Hz, 0.6 Hz, 1H, H-8); 8.61 (dd, J = 7.5 Hz, 0.6 Hz, 1H, H-5). ¹³C NMR (CDCl₃, 600 MHz) δ (ppm): 23.1; 26.6; 50.4; 53.4; 55.4; 58.0; 69.2; 92.2; 111.2; 112.2; 118.3; 121.0; 123.1; 125.4; 141.1; 150.4; 152.3; 161.6.

### Example E98:

### 7-[4-[4-(2-Chloroyphenyl)piperazin-1-yl]butoxy]tetrazolo[1,5-a]pyridine

Synthesis worked according to the preparation of E15 when using 5-(4-bromobutoxy)tetrazolo[1,5-a]pyridine (C6i) and 1-(2-chlorophenyl)piperazine (C7c).
Yield: 12 mg (63 %) beige solid.
Mp.: 101°C. MS (APCl) m/z 389 (M+2)⁺, 387 (M)⁺. IR (NaCl) v (cm⁻¹): 3070; 2943; 2816; 1647; 1477; 1203; 768. ¹H NMR (CDCl₃, 360 MHz) δ (ppm): 1.71-1.80 (m, 2H, CH₂CH₂N); 1.91-1.99 (m, 2H, OCH₂CH₂); 2.52 (t, J = 7.3 Hz, 2H, CH₂N); 2.64-2.70 (m, 4H, Piperazine); 3.09-3.11 (m, 4H, Piperazine); 4.16 (t, J = 6.4 Hz, 2H, OCH₂); 6.86 (dd, J = 7.5 Hz, 2.3 Hz, 1H, H-6); 6.97 (ddd, J = 7.8 Hz, 7.3 Hz, 1.5 Hz, 1H, Phenyl H4); 7.05 (dd, J = 8.1 Hz, 1.5 Hz, 1H, Phenyl H6); 7.16 (dd, J = 2.3 Hz, 0.6 Hz, 1H, H-7); 7.22 (ddd, J = 8.1 Hz, 7.3 Hz, 1.6 Hz, 1H, Phenyl H5); 7.35 (dd, J = 7.8 Hz, 1.6 Hz, 1H, Phenyl H3); 8.60 (dd, J = 7.5 Hz, 0.6 Hz, 1H, H-7). ¹³C NMR (CDCl₃, 360 MHz) δ (ppm): 23.3; 26.7; 51.3; 53.5; 58.1; 69.3; 92.3; 112.2; 120.5; 123.8; 125.4; 127.7 ; 128.8; 130.7; 149.3; 150.5; 161.7.

### 5. Synthesis of Example compounds according to formula XVI

### 5a) Compounds substituted with a formyl group in position 3

The synthesis of compounds according formula XVIa was achieved by formylation of the pyrazolo[1,5-a]pyridine scaffold in position 3.

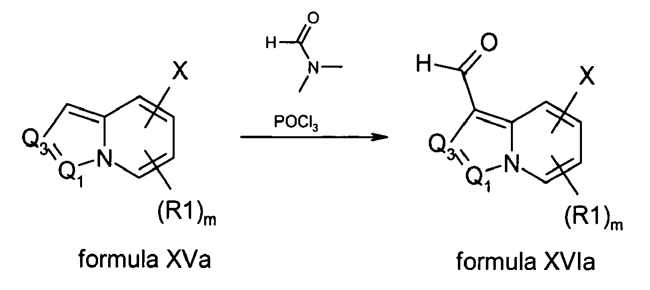

### Example E21:

### 5-[4-[4-(2,3-Dichlorophenyl)piperazin-1-yl]butoxy]pyrazolo[1,5-a]pyridine-3-carbaldehyde

For the synthesis 360 mg (0.86 mmol) 5-[4-[4-(2,3-dichlorophenyl)piperazin-1-yl]butoxy]pyrazolo[1,5-a]pyridine (E15), 2.75 ml dry dimethylformamide and 0.25 ml (2.66 mmol) phosphoroxychloride were stirred at room temperature for one hr. Then, water was added and the mixture was adjusted to basic conditions with 5N NaOH. After extraction with chloroform, the organic layer was dried with Na₂SO₄ and evaporated. The crude product was purified by flash-chromatography (CH₂Cl₂/MeOH 98:2 and subsequently with 95:5).
Yield: 361 mg (94 %) beige solid.
Mp.: 107°C. MS (EI): m/z 448 (M+1)⁺, 446 (M)⁺. IR (NaCl) v (cm⁻¹): 2946; 2818; 1663; 1633; 1577; 1527; 1275; 1241; 1194; 1044; 774. ¹H NMR (CDCl₃, 360 MHz) δ (ppm): 1.70-1.78 (m, 2H, CH₂CH₂N); 1.88-1.96 (m, 2H, OCH₂CH₂); 2.51 (t, J = 7.6 Hz, 2H, CH₂N); 2.64-2.68 (m, 4H, Piperazine); 3.07-3.09 (m, 4H, Piperazine); 4.16 (t, J = 6.5 Hz, 2H, OCH₂); 6.71 (dd, J = 7.6 Hz, 2.6 Hz, 1H, H-6); 6.96 (dd, J = 6.5 Hz, 3.0 Hz, 1H, Phenyl H6); 7.14-7.15 (m, 2H, Phenyl H4, Phenyl H5); 7.58 (d, J = 2.6 Hz, 1H, H-4); 8.26 (s, 1H, H-2); 8.37 (d, J = 7.6 Hz, 1H, H-7); 9.95 (s, 1H, CHO). ¹³C NMR (CDCl₃, 360 MHz) δ (ppm): 23.3; 26.9; 51.4; 53.4; 58.0; 69.0; 97.8; 109.6; 113.3; 118.7; 124.6; 127.4; 127.5; 130.1; 134.1; 141.6; 147.6; 151.4; 160.7; 183.2.
C H N (%):C₂₂H₂₄N₄O₂Cl₂ x 0.3H₂O; calculated: C 58.36; H 5.48; N 12.37; found: C 58.34; H 5.43; N 12.27.

### Example E22:

### 5-[4-[4-(2-Methoxyphenyl)piperazin-1-yl]butoxy]pyrazolo[1,5-a]pyridine-3-carbaldehyde

Synthesis worked according to the preparation of E21 when using 5-[4-[4-(2-methoxyphenyl)piperazin-1-yl]butoxy]pyrazolo[1,5-a]pyridine (E16).
Yield: 846 mg (99 %) beige solid.
Mp.: 90°C. MS (EI): m/z 408 (M)⁺. IR (NaCl) v (cm⁻¹): 2941; 2816; 1663; 1633; 1527; 1275; 1241; 1194; 772. ¹H NMR (CDCl₃, 600 MHz) δ (ppm): 1.72-1.77 (m, 2H, CH₂CH₂N); 1.89-1.93 (m, 2H, OCH₂CH₂); 2.50 (t, J = 7.6 Hz, 2H, CH₂N); 2.67-2.70 (m, 4H, Piperazine); 3.09-3.13 (m, 4H, Piperazine); 3.86 (s, 3H, OCH₃); 4.15 (t, J = 6.3 Hz, 2H, OCH₂); 6.71 (dd, J = 7.6 Hz, 2.6 Hz, 1H, H-6); 6.86 (dd, J = 7.9 Hz, 1.0 Hz, 1H, Phenyl H3); 6.92 (ddd, J = 7.5 Hz, 7.0 Hz, 1.0 Hz, 1H, Phenyl H5); 6.95 (dd, J = 7.5 Hz, 1.7 Hz, 1H, Phenyl H6); 6.99 (ddd, J = 7.9 Hz, 7.0 Hz, 1.7 Hz, 1H, Phenyl H4); 7.57 (d, J = 2.6 Hz, 1H, H-4); 8.26 (s, 1H, H-2); 8.37 (d, J = 7.6 Hz, 1H, H-7); 9.94 (s, 1H, COH). ¹³C NMR (CDCl₃, 360 MHz) δ (ppm): 23.4; 26.9; 50.7; 53.5; 55.4; 58.2; 69.0; 97.9; 109.7; 111.3; 113.3; 118.3; 121.1; 123.0; 130.2; 141.4; 141.6; 147.5; 152.3; 160.7; 183.1.
C H N (%):C₂₃H₂₈N₄O₃ x 0.3 H₂O; calculated: C 66.74; H 6.97; N 13.54; found: C 66.62; H 6.79; N 13.52.

### Example E23:

### 5-[4-[4-(2-Chlorophenyl)piperazin-1-yl]butoxy]pyrazolo[1,5-a]pyridine-3-carbaldehyde

Synthesis worked according to the preparation of E21 when using 5-[4-[4-(2-chlorophenyl)piperazin-1-yl]butoxy]pyrazolo[1,5-a]pyridine (E17).
Yield: 285 mg (80 %) white solid.
Mp.: 116°C. MS (EI): m/z 414 (M+1)⁺, 412 (M-1)⁺. IR (NaCl) v (cm⁻¹): 2942; 2816; 1663; 1633; 1527; 1274; 1220; 1194; 772. ¹H NMR (CDCl₃, 600 MHz) δ (ppm): 1.72-1.77 (m, 2H, CH₂CH₂N); 1.90-1.94 (m, 2H, OCH₂CH₂); 2.51 (t, J = 7.6 Hz, 2H, CH₂N); 2.64-2.69 (m, 4H, Piperazine); 3.07-3.12 (m, 4H, Piperazine); 4.15 (t, J = 6.3 Hz, 2H, OCH₂); 6.71 (dd, J = 7.6 Hz, 2.6 Hz, 1H, H-6); 6.96 (ddd, J = 7.8 Hz, 7.3 Hz, 1.3 Hz, 1H, Phenyl H4); 7.05 (dd, J = 8.0 Hz, 1.3 Hz, 1H, Phenyl H6); 7.21 (ddd, J = 8.0 Hz, 7.3 Hz, 1.2 Hz, 1H, Phenyl H5); 7.35 (dd, J = 7.8 Hz, 1.2 Hz, 1H, Phenyl H3); 7.58 (d, J = 2.6 Hz, 1H, H-4); 8.26 (s, 1H, H-2); 8.37 (d, J = 7.6 Hz, 1H, H-7); 9.94 (s, 1H, COH). ¹³C NMR (CDCl₃, 360 MHz) δ (ppm): 23.4; 26.9; 51.3; 53.4; 58.1; 69.0; 97.7; 109.6; 113.2; 120.4; 123.7; 127.6; 128.8; 130.1; 130.7; 141.6; 147.5; 149.3; 160.7; 183.1.
C H N (%):C₂₂H₂₅N₄O₂Cl x 0.1H₂O; calculated: C 63.72; H 6.13; N 13.51; found: C 63.84; H 6.42; N 13.69.

### Example E24:

### 5-[4-[4-(4-Methoxyphenyl)piperazin-1-yl]butoxy]pyrazolo[1,5-a]pyridine-3-carbaldehyde

Synthesis worked according to the preparation of E21 when using 5-[4-[4-(4-methoxyphenyl)piperazin-1-yl]butoxy]pyrazolo[1,5-a]pyridine (E18).
Yield: 44 mg (85 %) yellow solid.
Mp.: 114°C. MS (EI): m/z 408 (M)⁺. IR (NaCl) v (cm⁻¹): 3065; 2945; 2817; 1663; 1633; 1528; 1511; 1276; 1243; 1194; 751. ¹H NMR (CDCl₃, 600 MHz) δ (ppm): 1.71-1.76 (m, 2H, CH₂CH₂N); 1.89-1.93 (m, 2H, OCH₂CH₂); 2.48 (t, J = 7.6 Hz, 2H, CH₂N); 2.62-2.64 (m, 4H, Piperazine); 3.10-3.12 (m, 4H, Piperazine); 3.76 (s, 3H, OCH₃); 4.15 (t, J = 6.3 Hz, 2H, OCH₂); 6.70 (dd, J = 7.6 Hz, 2.6 Hz, 1H, H-6); 6.83-6.84 (m, 2H, Phenyl H2, Phenyl H6); 6.90-6.91 (m, 2H, Phenyl H3, Phenyl H5); 7.57 (d, J = 2.6 Hz, 1H, H-4); 8.26 (s, 1H, H-2); 8.37 (d, J = 7.6 Hz, 1H, H-7); 9.94 (s, 1H, COH). ¹³C NMR (CDCl₃, 360 MHz) δ (ppm): 23.4; 26.9; 50.7; 53.5; 55.6; 58.1; 69.0; 97.8; 109.6; 113.3; 114.5; 118.2; 130.2; 141,6; 145.8; 147.5; 153.9; 160.7; 183.2.
C H N (%):C₂₃H₂₈N₄O₃ x 0.7 H₂O; calculated: C 65.60; H 7.04; N 13.31; found: C 65.39; H 6.68; N 13.18.

### Example E25:

### 5-[4-[4-(4-Chlorophenyl)piperazin-1-yl]butoxy]pyrazolo[1,5-a]pyridine-3-carbaldehyde

Synthesis worked according to the preparation of E21 when using 5-[4-[4-(4-chlorophenyl)piperazin-1-yl]butoxy]pyrazolo[1,5-a]pyridine (E19).
Yield: 36 mg (68 %) light yellow solid.
Mp.: 113°C. MS (APCl): m/z 415 (M+2)⁺, 413 (M)⁺. IR (NaCl) v (cm⁻¹): 2944; 2817; 1663; 1633; 1527; 1274; 1239; 1194; 772. ¹H NMR (CDCl₃, 600 MHz) δ (ppm): 1.71-1.76 (m, 2H, CH₂CH₂N); 1.89-1.94 (m, 2H, OCH₂CH₂); 2.48 (t, J = 7.6 Hz, 2H, CH₂N); 2.61-2.62 (m, 4H, Piperazine); 3.16-3.18 (m, 4H, Piperazine); 4.15 (t, J = 6.3 Hz, 2H, OCH₂); 6.70 (dd, J = 7.6 Hz, 2.6 Hz, 1H, H-6); 6.83-6.84 (m, 2H, Phenyl H2, Phenyl H6); 7.19-7.20 (m, 2H, Phenyl H3, Phenyl H5); 7.57 (d, J = 2.6 Hz, 1H, H-4); 8.26 (s, 1H, H-2); 8.37 (d, J = 7.6 Hz, 1H, H-7); 9.94 (s, 1H, COH). ¹³C NMR (CDCl₃, 360 MHz) δ (ppm): 23.4; 26.9; 49.2; 53.2; 58.0; 69.0; 97.8; 109.6; 113.2; 117.2; 124.6; 129.0; 130.2; 141.6; 147.6; 150.0; 160.7; 183.2.
C H N (%):C₂₂H₂₅N₄O₂Cl x 0.3H₂O; calculated: C 63.17; H 6.17; N 13.39; found: C 63.12; H 6.13; N 13.18.

### Example E26:

### 5-[4-[4-(4-Hydroxyphenyl)piperazin-1-yl]butoxy]pyrazolo[1,5-a]pyridine-3-carbaldehyde

Synthesis worked according to the preparation of E21 when using 5-[4-[4-(4-hydroxyphenyl)piperazin-1-yl]butoxy]pyrazolo[1,5-a]pyridine (E20).
Yield: 38 mg (89 %) beige solid.
Mp.: 164°C. MS (APCI): m/z 395 (M+1)⁺. IR (NaCl) v (cm⁻¹): 3306; 2927; 2819; 1661; 1633; 1528; 1514; 1276; 1241; 1194; 771. ¹H NMR (CDCl₃, 360 MHz) δ (ppm): 1.70-1.78 (m, 2H, CH₂CH₂N); 1.86-1.93 (m, 2H, OCH₂CH₂); 2.49 (t, J = 7.6 Hz, 2H, CH₂N); 2.64-2.66 (m, 4H, Piperazine); 3.08-3.11 (m, 4H, Piperazine); 4.13 (t, J = 6.3 Hz, 2H, OCH₂); 6.69 (dd, J = 7.6 Hz, 2.6 Hz, 1H, H-6); 6.74-6.76 (m, 2H, Phenyl H2, Phenyl H6); 6.82-6.85 (m, 2H, Phenyl H3, Phenyl H5); 7.57 (d, J = 2.6 Hz, 1H, H-4); 8.26 (s, 1H, H-2); 8.36 (d, J = 7.6 Hz, 1H, H-7); 9.93 (s, 1H, COH). ¹³C NMR (CDCl₃, 360 MHz) δ (ppm): 23.2; 26.9; 50.7; 53.4; 58.0; 68.9; 97.9; 109.7; 113.2; 116.0; 118.6; 130.2; 141.7; 145.6; 147.6; 150.3; 160.8; 183.3.

### Example E4:

### 2-[4-[4-(2,3-Dichlorophenyl)piperazin-1-yl]butoxy]pyrazolo[1,5-a]pyridine-3-carbaldehyde

Synthesis worked according to the preparation of E21 when using 2-[4-[4-(2,3-dichlorophenyl)piperazin-1-yl]butoxy]pyrazolo[1,5-a]pyridine (E1).
Yield: 75 mg (70 %) yellow solid.
Mp.: 100°C. MS (APCI): m/z 449 (M+2)⁺, 447 (M)⁺. IR (NaCl) v (cm⁻¹): 3107; 2947; 2816; 1659; 1625; 1529; 1242; 1142; 1063; 756. ¹H NMR (CDCl₃, 600 MHz) δ (ppm): 1.72-1.77 (m, 2H, CH₂CH₂N); 1.91-1.96 (m, 2H, OCH₂CH₂); 2.52 (t, J = 7.8 Hz, 2H, CH₂N); 2.63-2.68 (m, 4H, Piperazine); 3.05-3.10 (m, 4H, Piperazine); 4.47 (t, J = 6.5 Hz, 2H, OCH₂); 6.93-6.96 (m, 2H, H-6, Phenyl H6); 7.12-7.16 (m, 2H, Phenyl H4, Phenyl H5); 7.46 (ddd, J = 8.8 Hz, 7.0 Hz, 1.2 Hz, 2H, H-5); 8.14 (dd, J = 8.8 Hz, 1.4 Hz, 1 H, H-4); 8.31 (dd, J = 6.7 Hz, 1.2 Hz, 1H, H-7); 9.93 (s, 1 H, COH). ¹³C NMR (CDCl₃, 600 MHz) δ (ppm): 23.4; 27.2; 51.4; 53.4; 58.2; 69.6; 98.8; 114.0; 118.3; 118.6; 124.5; 127.4; 127.5; 129.0; 129.6; 134.1; 141.2; 151.4; 167.4; 182.1.
C H N (%):C₂₂H₂₄N₄O₂Cl₂ x 0.2H₂O; calculated: C 58.59; H 5.45; N 12.42; found: C 58.50; H 5.48; N 12.19.

### Example E5:

### 2-[4-[4-(2-Methoxyphenyl)piperazin-1-yl]butoxy]pyrazolo[1, 5-a]pyridine-3-carbaldehyde

Synthesis worked according to the preparation of E21 when using 2-[4-[4-(2-methoxyphenyl)piperazin-1-yl]butoxy]pyrazolo[1,5-a]pyridine (E2).
Yield: 72 mg (67 %) light yellow solid.
Mp.: 101°C. MS (APCI): m/z 409 (M+1)⁺. IR (NaCl) v (cm⁻¹): 2941; 3068; 2943; 2814; 1656; 1626; 1527; 1240; 1061; 754. ¹H NMR (CDCl₃, 360 MHz) δ (ppm): 1.71-1.79 (m, 2H, CH₂CH₂N); 1.89-1.97 (m, 2H,OCH₂CH₂); 2.51 (t, J = 7.5 Hz, 2H, CH₂N); 2.66-2.69 (m, 4H, Piperazine); 3.09-3.12 (m, 4H, Piperazine); 3.86 (s, 3H, OCH₃); 4.47 (t, J = 6.7 Hz, 2H, OCH₂); 6.86 (dd, J = 7.9, 1.0 Hz, 1 H, Phenyl H3); 6.91-6.95 (m, 3H, Phenyl H5, Phenyl H6, H-6); 6.99 (ddd, J = 7.9 Hz, 6.7 Hz, 2.6 Hz, 1 H, Phenyl H4); 7.45 (ddd, J = 8.8 Hz, 6.9 Hz, 1.2 Hz, 1H, H-5); 8.14 (dd, J = 8.8 Hz, 1.4 Hz, 1 H, H-4); 8.31 (dd, J = 6.7 Hz, 1.2 Hz, 1 H, H-7); 9.93 (s, 1H, COH). ¹³C NMR (CDCl₃, 360 MHz) δ (ppm): 23.4; 27.2; 50.7; 53.5; 55.4; 58.3; 69.7; 98.9; 111.3; 114.0; 118.2; 118.3; 121.0; 122.9; 129.0; 129.6; 141.2; 141.4; 152.3; 167.4; 182.2.
C H N (%):C₂₃H₂₈N₄O₃ x 0.6H₂O; calculated: C 65.88; H 7.02; N 13.36; found: C 65.67; H 6.79; N 13.12.

### Example E6:

### 2-[4-[4-(2-Chlorophenyl)piperazin-1-yl]butoxy]pyrazolo[1,5-a]pyridine-3-carbaldehyde

Synthesis worked according to the preparation of E21 when using 2-[4-[4-(2-chlorophenyl)piperazin-1-yl]butoxy]pyrazolo[1,5-a]pyridine (E3).
Yield: 69 mg (64 %) light yellow solid.
Mp.: 104°C. MS (APCI): m/z 415 (M+2)⁺, 413 (M)⁺. IR (NaCl) v (cm⁻¹): 3062; 2947; 2814; 1659; 1626; 1527; 1230; 1063; 756. ¹H NMR (CDCl₃, 600 MHz) δ (ppm): 1.72-1.78 (m, 2H, CH₂CH₂N); 1.91-1.96 (m, 2H, OCH₂CH₂); 2.52 (t, J = 7.8 Hz, 2H, CH₂N); 2.63-2.69 (m, 4H, Piperazine); 3.07-3.12 (m, 4H, Piperazine); 4.47 (t, J = 6.4 Hz, 2H, OCH₂); 6.94 (ddd, J = 7.0 Hz, 6.8 Hz, 1.4 Hz, 1 H, H-6); 6.96 (ddd, J = 7.8 Hz, 7.3 Hz, 1.3 Hz, 1 H, Phenyl H4); 7.04 (dd, J = 8.0 Hz, 1.3 Hz, 1H, Phenyl H6); 7.21 (ddd, J = 8.0 Hz, 7.3 Hz, 1.1 Hz, 1H, Phenyl H5); 7.35 (dd, J = 7.8 Hz, 1.1 Hz, 1 H, Phenyl H3); 7.46 (ddd, J = 8.9 Hz, 7.0 Hz, 1.2 Hz, 1H, H-5); 8.14 (dd, J = 8.9 Hz, 1.4 Hz, 1 H, H-4); 8.31 (dd, J = 6.8 Hz, 1.2 Hz, 1 H, H-7); 9.93 (s, 1 H, COH). ¹³C NMR (CDCl₃, 360 MHz) δ (ppm): 23.7; 27.4; 51.2; 53.4; 58.2; 69.6; 98.8; 113.9; 118.3; 120.6; 123.6; 127.6; 128.8; 129.0; 129.6; 130.7; 141.2; 149.4; 167.4; 182.2.
C H N (%):C₂₂H₂₅N₄O₂Cl x 0.2H₂O calculated: C 63.44; H 6.15; N 13.45; found: C 63.17; H 5.77; N 13.63.

### Example E10:

### 4-[4-[4-(2,3-Dichlorophenyl)piperazin-1-yl]butoxy]pyrazolo[1,5-a]pyridine-3-carbaldehyde

Synthesis worked according to the preparation of E21 when using 4-[4-[4-(2,3-dichlorophenyl)piperazin-1-yl]butoxy]pyrazolo[1,5-a]pyridine (E7).
Yield: 152 mg (85 %) beige solid.
Mp.: 128°C. MS (APCl): m/z 449 (M+2)⁺, 447 (M)⁺. IR (NaCl) v (cm⁻¹): 3065; 2947; 2818; 1661; 1518; 1278; 1072; 779. ¹H NMR (CDCl₃, 600 MHz) δ (ppm): 1.74-1.79 (m, 2H, CH₂CH₂N); 1.97-2.02 (m, 2H, OCH₂CH₂); 2.51 (t, J = 7.6 Hz, 2H, CH₂N); 2.63-2.68 (m, 4H, Piperazine); 3.05-3.09 (m, 4H, Piperazine); 4.24 (t, J = 6.3 Hz, 2H, OCH₂); 6.77 (d, J = 7.6 Hz, 1 H, H-5); 6.88 (dd, J = 7.6 Hz, 6.9 Hz, 1 H, H-6); 6.96 (dd, J = 7.3 Hz, 2.4 Hz, 1H, Phenyl H6); 7.13-7.16 (m, 2H, Phenyl H4, Phenyl H5); 8.22 (d, J = 6.9 Hz, 1H, H-7); 8.46 (s, 1 H, H-2); 10.40 (s, 1 H, COH). ¹³C NMR (CDCl₃, 360 MHz) δ (ppm): 23.5; 27.0; 51.3; 53.4; 57.9; 69.0; 105.4; 113.6; 115.8; 118.6; 122.7; 124.6; 127.4; 127.5; 134.0; 135.2; 142.3; 151.3; 151.5; 184.7.
C H N (%):C₂₂H₂₄N₄O₂Cl₂; calculated: C 59.07; H 5.41; N 12.52; found: C 59.13; H 5.27; N 12.46.

### Example E11:

### 4-[4-[4-(2-Methoxyphenyl)piperazin-1-yl]butoxy]pyrazolo[1,5-a]pyridine-3-carbaldehyde

Synthesis worked according to the preparation of E21 when using 4-[4-[4-(2-methoxyphenyl)piperazin-1-yl]butoxy]pyrazolo[1,5-a]pyridine (E8).
Yield: 135 mg (84 %) light yellow solid.
Mp.: 139°C. MS (APCI): m/z 409 (M+1)⁺. IR (NaCl) v (cm⁻¹): 3062; 2944; 2814; 1663; 1519; 1278; 1240; 749. ¹H NMR (CDCl₃, 600 MHz) δ (ppm): 1.74-1.79 (m, 2H, CH₂CH₂N), 1.97-2.02 (m, 2H, OCH₂CH₂), 2.51 (t, J = 7.6 Hz, 2H, CH₂N), 2.65-2.68 (m, 4H, Piperazine), 3.08-3.12 (m, 4H, Piperazine), 3.86 (s, 3H, OCH₃), 4.24 (t, J = 6.3 Hz, 2H, OCH₂), 6.77 (d, J = 7.6 Hz, 1H, H-5), 6.85-6.88 (m, 2H, H-6, Phenyl H3), 6.92 (ddd, J = 7.5 Hz, 7.0 Hz, 1.0 Hz, 1 H, Phenyl H5), 6.94 (dd, J = 7.5 Hz, 2.2 Hz, 1H, Phenyl H6), 6.99 (ddd, J = 7.9 Hz, 7.0 Hz, 2.2 Hz, 1 H, Phenyl H4), 8.22 (d, J = 6.9 Hz, 1 H, H-7), 8.46 (s, 1 H, H-2), 10.40 (s, 1 H, COH). ¹³C NMR (CDCl₃, 360 MHz) δ (ppm): 23.5; 27.1; 50.7; 53.5; 55.4; 58.1; 69.1; 105.5; 111.4; 113.6; 115.8; 118.3; 121.1; 122.7; 122.9; 135.3; 141.4; 142.3; 151.5; 152.4; 184.7.
C H N (%):C₂₃H₂₈N₄O₃ x 0.3H₂O; calculated: C 66.74; H 6.97; N 13.54; found: C 66.58; H 6.80; N 13.56.

### Example E12:

### 4-[4-[4-(2-Chlorophenyl)piperazin-1-yl]butoxy]pyrazolo[1,5-a]pyridine-3-carbaldehyde

Synthesis worked according to the preparation of E21 when using 4-[4-[4-(2-chlorophenyl)piperazin-1-yl]butoxy]pyrazolo[1,5-a]pyridine (E9).
Yield: 149 mg (90 %) light yellow solid.
Mp.: 125°C. MS (APCl): m/z 415 (M+2)⁺, 413 (M)⁺. IR (NaCl) v (cm⁻¹): 3069; 2945; 2817; 1662; 1518; 1278; 1226; 765. ¹H NMR (CDCl₃, 600 MHz) δ (ppm): 1.74-1.79 (m, 2H, CH₂CH₂N); 1.97-2.02 (m, 2H, OCH₂CH₂); 2.51 (t, J = 7.6 Hz, 2H, CH₂N); 2.63-2.67 (m, 4H, Piperazine); 3.07-3.10 (m, 4H, Piperazine); 4.24 (t, J = 6.3 Hz, 2H, OCH₂); 6.77 (d, J = 7.6 Hz, 1 H, H-5); 6.88 (dd, J = 7.6 Hz, 6.9 Hz, 1 H, H-6); 6.96 (ddd, J = 7.8 Hz, 7.3 Hz, 1.3 Hz, 1 H, Phenyl H4); 7.05 (dd, J = 8.0 Hz, 1.3 Hz, 1 H, Phenyl H6); 7.22 (ddd, J = 8.0 Hz, 7.3 Hz, 1.5 Hz, 1 H, Phenyl H5); 7.35 (dd, J = 7.8 Hz, 1.5 Hz, 1 H, Phenyl H3); 8.22 (d, J = 6.9 Hz, 1 H, H-7); 8.46 (s, 1 H, H-2); 10.40 (s, 1 H, COH). ¹³C NMR (CDCl₃, 600 MHz) δ (ppm): 23.4; 27.0; 51.2; 53.4; 58.0; 69.0; 105.4; 113.5; 115.7; 120.3; 122.7; 123.6; 127.5; 128.8; 130.6; 135.2; 142.3; 149.3; 151.5; 184.7.
C H N (%):C₂₂H₂₅N₄O₂Cl; calculated: C 63.99; H 6.10; N 13.57; found: C 63.97; H 6.11; N 13.56.

### Example E54:

### 6-[4-[4-(2,3-Dichlorophenyl)piperazin-1-yl]butoxy]pyrazolo[1,5-a]pyridine-3-carbaldehyde

Synthesis worked according to the preparation of E21 when using 6-[4-[4-(2,3-dichlorophenyl)piperazin-1-yl]butoxy]pyrazolo[1,5-a]pyridine (E51).
Yield: 40 mg (63 %) beige solid.
Mp.: 99°C. MS (APCI): m/z 449 (M+2)⁺, 447 (M)⁺. IR (NaCl) v (cm⁻¹): 3096; 2946; 2818; 1663; 1578; 1519; 1278; 1242; 1185; 1044; 780. ¹H NMR (CDCl₃, 360 MHz) δ (ppm): 1.71-1.79 (m, 2H, CH₂CH₂N); 1.88-1.95 (m, 2H, OCH₂CH₂); 2.51 (t, J = 7.6 Hz, 2H, CH₂N); 2.64-2.70 (m, 4H, Piperazine); 3.06-3.11 (m, 4H, Piperazine); 4.05 (t, J = 6.4 Hz, 2H, OCH₂); 6.96 (dd, J = 6.6 Hz, 3.0 Hz, 1 H, Phenyl H6); 7.14-7.15 (m, 2H, Phenyl H4, Phenyl H5); 7.30 (dd, J = 9.7 Hz, 2.2 Hz, 1 H, H-5); 8.16-8.18 (m, 2H, H-4, H-7); 8.29 (s, 1 H, H-2); 9.98 (s, 1H, COH). ¹³C NMR (CDCl₃, 360 MHz) δ (ppm): 23.3; 27.0; 51.4; 53.4; 58.1; 69.1; 112.7; 113.8; 118.6; 119.1; 123.7; 124.6; 127.4; 127.6; 134.1; 135.5; 146.2; 150.6; 151.3; 183.1.
C H N (%):C₂₂H₂₄N₄O₂Cl₂; calculated: C 59.07; H 5.41; N 12.52; found: C 58.68; H 5.43; N 12.16.

### Example E55:

### 6-[4-[4-(2- Methoxyphenyl)piperazin-1-yl]butoxy]pyrazolo[1,5-a]pyridine-3-carbaldehyde

Synthesis worked according to the preparation of E21 when using 6-[4-[4-(2-methoxyphenyl)piperazin-1-yl]butoxy]pyrazolo[1,5-a]pyridine (E52).
Yield: 135 mg (79 %) orange solid.
Mp.: 87°C. MS (APCI): m/z 409 (M+1)⁺. IR (NaCl) v (cm⁻¹): 3099; 2943; 2816; 1663; 1519; 1278; 1241; 1185; 748. ¹H NMR (CDCl₃, 600 MHz) δ (ppm): 1.71-1.79 (m, 2H, CH₂CH₂N); 1.87-1.95 (m, 2H, OCH₂CH₂); 2.50 (t, J = 7.6 Hz, 2H, CH₂N); 2.66-2.70 (m, 4H, Piperazine); 3.09-3.14 (m, 4H, Piperazine); 3.86 (s, 3H, OCH₃); 4.05 (t, J = 6.4 Hz, 2H, OCH₂); 6.86 (dd, J = 8.0 Hz, 1.3 Hz, 1 H, Phenyl H3); 6.89-6.96 (m, 2H, Phenyl H5, Phenyl H6); 7.00 (ddd, J = 8.0 Hz, 6.6 Hz, 2.5 Hz, 1H, Phenyl H4); 7.30 (dd, J = 9.7 Hz, 2.2 Hz, 1 H, H-5); 8.15-8.18 (m, 2H, H-4, H-7); 8.28 (s, 1 H, H-2); 9.98 (s, 1 H, COH). ¹³C NMR (CDCl₃, 360 MHz) δ (ppm): 23.3; 27.1; 50.7; 53.6; 55.4; 58.2; 69.1; 111.3; 112.7; 113.8; 118.2; 119.0; 121.0; 122.9; 123.7; 135.5; 141.4; 146.2; 150.6; 152.3; 183.1. C H N (%):C₂₃H₂₈N₄O₃ x 0.3H₂O; calculated: C 66.74; H 6.97; N 13.54; found: C 66.39; H 6.76; N 13.89.

### Example E56:

### 6-[4-[4-(2-Chlorophenyl)piperazin-1-yl]butoxy]pyrazolo[1,5-a]pyridine-3-carbaldehyde

Synthesis worked according to the preparation of E21 when using 6-[4-[4-(2-chlorophenyl)piperazin-1-yl]butoxy]pyrazolo[1,5-a]pyridine (E53).
Yield: 19 mg (32 %) light pink solid.
Mp.: 71°C. MS (APCI): m/z 415 (M+2)⁺, 413 (M)⁺. IR (NaCl) v (cm⁻¹): 3094; 2945; 2817; 1663; 1520; 1278; 1231; 1185; 765. ¹H NMR (CDCl₃, 360 MHz) δ (ppm): 1.71-1.79 (m, 2H, CH₂CH₂N); 1.88-1.95 (m, 2H, OCH₂CH₂); 2.51 (t, J = 7.6 Hz, 2H, CH₂N); 2.65-2.70 (m, 4H, Piperazine); 3.08-3.13 (m, 4H, Piperazine); 4.05 (t, J = 6.3 Hz, 2H, OCH₂); 6.97 (ddd, J = 7.8 Hz, 7.3 Hz, 1.3 Hz, 1 H, Phenyl H4); 7.05 (dd, J = 8.0 Hz, 1.3 Hz, 1 H, Phenyl H6); 7.22 (ddd, J = 8.0 Hz, 7.3 Hz, 1.5 Hz, 1 H, Phenyl H5); 7.30 (dd, J = 9.7 Hz, 2.2 Hz, 1 H, H-5); 7.35 (dd, J = 7.8 Hz, 1.5 Hz, 1 H, Phenyl H3); 8.15-8.18 (m, 2H, H-4, H-7); 8.29 (s, 1 H, H-2); 9.98 (s, 1 H, COH). ¹³C NMR (CDCl₃, 360 MHz) δ (ppm): 23.4; 27.0; 51.3; 53.5; 58.1; 69.1; 112.8; 113.9; 119.1; 120.5; 123.8; 127.6; 128.8; 130.7; 135.5; 146.2; 149.3; 150.6; 183.1.
C H N (%):C₂₂H₂₅N₄O₂Cl; calculated: C 63.99; H 6.10; N 13.57; found: C 63.60; H 6.05; N 13.34.

### Example E60:

### 7-[4-[4-(2,3-Dichlorophenyl)piperazin-1-yl]butoxy]pyrazolo[1,5-a]pyridine-3-carbaldehyde

Synthesis worked according to the preparation of E21 when using 7-[4-[4-(2,3-dichlorophenyl)piperazin-1-yl]butoxy]pyrazolo[1,5-a]pyridine (E57).
Yield: 22 mg (40 %) light green oil.
MS (APCI): m/z 449 (M+2)⁺, 447 (M)⁺. IR (NaCl) v (cm⁻¹): 3088; 2927; 2818; 1660; 1520; 1219; 1138; 773. ¹H NMR (CDCl₃, 600 MHz) δ (ppm): 1.78-1.83 (m, 2H, CH₂CH₂N); 2.07-2.11 (m, 2H, OCH₂CH₂); 2.54 (t, J = 7.2 Hz, 2H, CH₂N); 2.63-2.69 (m, 4H, Piperazine); 3.03-3.07 (m, 4H, Piperazine); 4.41 (t, J = 6.6 Hz, 2H, OCH₂); 6.43 (dd, J = 7.6 Hz, 0.8 Hz, 1 H, H-6); 6.94 (dd, J = 7.1 Hz, 2.4 Hz, 1H, Phenyl H6); 7.12-7.15 (m, 2H, Phenyl H4, Phenyl H5); 7.52 (dd, J = 8.6 Hz, 7.6 Hz, 1 H, H-5); 7.91 (dd, J = 8.6 Hz, 0.8 Hz, 1 H, H-4); 8.42 (s, 1H, H-2); 10.04 (s, 1 H, COH). ¹³C NMR (CDCl₃, 600 MHz) δ (ppm): 23.1; 26.7; 51.3; 53.3; 57.8; 70.5; 93.8; 110.7; 113.7; 118.6; 124.6; 127.5; 127.6; 131.2; 134.0; 141.6; 146.7; 150.9; 151.2; 183.4.

### Example E61:

### 7-[4-[4-(2-Methoxyphenyl)piperazin-1-yl]butoxy]pyrazolo[1,5-a]pyridine-3-carbaldehyde

Synthesis worked according to the preparation of E21 when using 7-[4-[4-(2-methoxyphenyl)piperazin-1-yl]butoxy]pyrazolo[1,5-a]pyridine (E58).
Yield: 14 mg (23 %) light yellow oil.
MS (APCI): m/z 409 (M+1)⁺. IR (NaCl) v (cm⁻¹): 3078; 2947; 2819; 1660; 1522; 1242; 1157; 783. ¹H NMR (CDCl₃, 600 MHz) δ (ppm): 1.79-1.85 (m, 2H, CH₂CH₂N); 2.07-2.11 (m, 2H, OCH₂CH₂); 2.56 (t, J = 7.3 Hz, 2H, CH₂N); 2.68-2.73 (m, 4H, Piperazine); 3.07-3.12 (m, 4H, Piperazine); 3.86 (s, 3H, OCH₃); 4.41 (t, J = 6.8 Hz, 2H, OCH₂); 6.43 (dd, J = 7.7 Hz, 0.9 Hz, 1 H, H-6); 6.89 (dd, J = 8.0 Hz, 1.1 Hz, 1 H, Phenyl H3); 6.92-6.95 (m, 2H, Phenyl H5, Phenyl H6); 7.09 (ddd, J = 8.0 Hz, 7.2 Hz, 1.5 Hz, 1H, Phenyl H4); 7.52 (dd, J = 8.6 Hz, 7.7 Hz, 1 H, H-5); 7.91 (dd, J = 8.6 Hz, 0.9 Hz, 1H, H-4); 8.41 (s, 1H, H-2); 10.04 (s, 1 H, COH). ¹³C NMR (CDCl₃, 600 MHz) δ (ppm): 23.1; 26.8; 51.3; 53.5; 55.4; 57.9; 70.5; 93.8; 110.7; 111.3; 113.8; 118.2; 121.0; 123.0; 131.2; 141.2; 141.5; 146.7; 150.8; 152.3; 183.3.

### Example E62:

### 7-[4-[4-(2-Chlorophenyl)piperazin-1-yl]butoxy]pyrazolo[1,5-a]pyridine-3-carbaldehyde

Synthesis worked according to the preparation of E21 when using 7-[4-[4-(2-chlorophenyl)piperazin-1-yl]butoxy]pyrazolo[1,5-a]pyridine (E59).
Yield: 13 mg (26 %) light yellow oil.
MS (APCI): m/z 415 (M+2)⁺, 413 (M)⁺. IR (NaCl) v (cm⁻¹): 3091; 2943; 2818; 1662; 1520; 1296; 1041; 785. ¹H NMR (CDCl₃, 600 MHz) δ (ppm): 1.78-1.83 (m, 2H, CH₂CH₂N); 2.07-2.12 (m, 2H, OCH₂CH₂); 2.54 (t, J = 7.6 Hz, 2H, CH₂N); 2.64-2.70 (m, 4H, Piperazine); 3.04-3.10 (m, 4H, Piperazine); 4.41 (t, J = 6.6 Hz, 2H, OCH₂); 6.43 (dd, J = 7.9 Hz, 0.9 Hz, 1 H, H-6); 6.97 (ddd, J = 7.8 Hz, 7.3 Hz, 1.3 Hz, 1H, Phenyl H4); 7.03 (dd, J = 8.0 Hz, 1.3 Hz, 1 H, Phenyl H6); 7.22 (ddd, J = 8.0 Hz, 7.3 Hz, 1.2 Hz, 1 H, Phenyl H5); 7.35 (dd, J = 7.8 Hz, 1.2 Hz, 1 H, Phenyl H3); 7.52 (dd, J = 8.6 Hz, 7.9 Hz, 1 H, H-5); 7.91 (dd, J = 8.6 Hz, 0.9 Hz, 1 H, H-4); 8.42 (s, 1 H, H-2); 10.04 (s, 1 H, COH). ¹³C NMR (CDCl₃, 600 MHz) δ (ppm): 23.1; 26.8; 51.2; 53.5; 57.8; 70.5; 93.8; 110.7; 113.8; 120.4; 123.8; 127.6; 128.8; 130.7; 131.1; 141.6; 146.7; 149.2; 150.9; 183.4.

### Example E14:

### 5-[3-[4-(2,3-Dichlorophenyl)piperazin-1-yl]propoxymethyl]pyrazolo[1,5-a]pyridine-3-carbaldehyde

Synthesis worked according to the preparation of E21 when using 5-[3-[4-(2,3-dichlorophenyl)piperazin-1-yl]propoxymethyl]pyrazolo[1,5-a]pyridine (E13).
Yield: 58 mg (92 %) yellow solid.
Mp.: 90°C. MS (EI): m/z 448 (M+1)⁺, 446 (M-1)⁺. IR (NaCl) v (cm⁻¹): 2922; 2819; 1666; 1638; 1522; 1241; 1199; 775. ¹H NMR (CDCl₃, 360 MHz) δ (ppm): 1.86-1.93 (m, 2H, CH₂CH₂N); 2.56 (t, J = 6.7 Hz, 2H, CH₂N); 2.64-2.67 (m, 4H, Piperazin); 3.05-3.08 (m, 4H, Piperazin); 3.62 (t, J = 6.3 Hz, 2H, OCH₂CH₂); 4.63 (s, 2H, CH₂O); 6.95 (dd, J = 6.7 Hz, 3.0 Hz, 1H, Phenyl H6); 7.11 (dd, J = 7.1 Hz, 1.9 Hz, 1H, H-6); 7.13-7.15 (m, 2H, Phenyl H4, Phenyl H5); 8.23 (dd, J = 1.9 Hz, 0.8 Hz, 1H, H-4); 8.37 (s, 1 H, H-2); 8.54 (dd, J = 7.1 Hz, 0.8 Hz, 1 H, H-7); 10.03 (s, 1 H, COH). ¹³C NMR (CDCl₃, 360 MHz) δ (ppm): 27.1; 51.3; 53.4; 55.3; 69.4; 71.4; 113.7; 114.7; 116.7; 118.6; 124.6; 127.4; 127.6; 129.1; 134.1; 139.7; 141.5; 147.0; 151.3; 183.3.

### 5b) Compounds substituted with a halogene group in position 3

The synthesis of compounds according to formula XVI bearing a halogene group at position 3 of the pyrazolo[1,5-a]scaffold was done as follows:

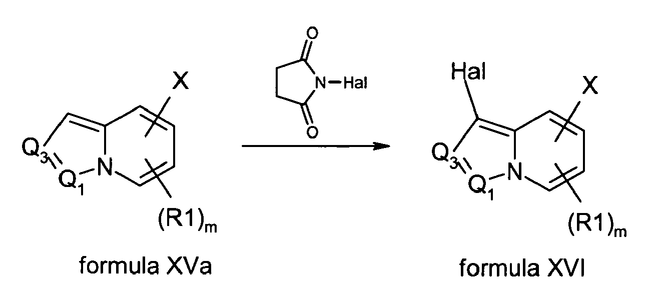

### Example E42:

### 3-Bromo-5-[4-(4-(2,3-dichlorophenyl)piperazin-1-yl]butoxy]pyrazolo[1,5-a]pyridine

For the synthesis 13.7 mg (0.033 mmol) 5-[4-[4-(2,3-dichlorophenyl)piperazin-1-yl]butoxy] pyrazolo[1,5-a]pyridine (E15), 1.16 ml dry chloroform and 7.0 mg (0.039 mmol) *N-*bromosuccinimide were stirred at room temperature for 3 days. Then the solvent was evaporated and the crude product was purified by flash-chromatography (CH₂Cl₂/MeOH 98:2).
Yield: 13 mg (80 %) white solid.
Mp.: 132°C. MS (APCI): m/z 501 (M+3)⁺, 499 (M+1)⁺, 497 (M-1)⁺. IR (NaCl) v (cm⁻¹): 3080; 2953; 2818; 1648; 1558; 1238; 1192; 1045. ¹H NMR (CDCl₃, 360 MHz) δ (ppm): 1.71-1.79 (m, 2H, CH₂CH₂N); 1.88-1.94 (m, 2H, OCH₂CH₂); 2.52 (t, J = 7.6 Hz, 2H, CH₂N); 2.66-2.70 (m, 4H, Piperazine); 3.07-3.10 (m, 4H, Piperazine); 4.08 (t, J = 6.3 Hz, 2H, OCH₂); 6.48 (dd, J = 7.6 Hz, 2.6 Hz, 1 H, H-6); 6.65 (d, J = 2.6 Hz, 1 H, H-4); 6.96 (dd, J = 6.5 Hz, 3.0 Hz, 1 H, Phenyl H6); 7.13-7.16 (m, 2H, Phenyl H4, Phenyl H5); 7.82 (s, 1 H, H-2); 8.25 (d, J = 7.6 Hz, 1H, H-7). ¹³C NMR (CDCl₃, 360 MHz) δ (ppm): 23.4; 27.0; 51.2; 53.3; 58.1; 68.4; 81.9; 93.8; 107.7; 118.6; 124.6; 127.4; 127.5; 129.8; 134.1; 139.2; 142.5; 151.3; 156.7.

### Example E43:

### 3-Bromo-5-[4-[4-(2-methoxyphenyl)piperazin-1-yl]butoxy]pyrazolo[1,5-a]pyridine

Synthesis worked according to the preparation of E42 when using 5-[4-[4-(2-methoxyphenyl)piperazin-1-yl]butoxy]pyrazolo[1,5-a]pyridine (E16).
Yield: 11 mg (63 %) colourless oil.
MS (APCI): m/z 462 (M+2)⁺, 460 (M)⁺. IR (NaCl) v (cm⁻¹): 2941; 2814; 1649; 1539; 1238; 1205; 1026; 748. ¹H NMR (CDCl₃, 360 MHz) δ (ppm): 1.71-1.79 (m, 2H, CH₂CH₂N); 1.86-1.94 (m, 2H, OCH₂CH₂); 2.51 (t, J = 7.6 Hz, 2H, CH₂N); 2.67-2.70 (m, 4H, Piperazine); 3.10-3.13 (m, 4H, Piperazine); 3.87 (s, 3H, OCH₃); 4.08 (t, J = 6.3 Hz, 2H, OCH₂); 6.48 (dd, J = 7.6 Hz, 2.6 Hz, 1 H, H-6); 6.65 (d, J = 2.6 Hz, 1 H, H-4); 6.86 (dd, J = 7.9 Hz, 1.0 Hz, 1 H, Phenyl H3); 6.91-6.96 (m, 2H, Phenyl H5, Phenyl H6); 7.00 (ddd, J = 7.9 Hz, 6.8 Hz, 2.2 Hz, 1 H, Phenyl H4); 7.81 (s, 1H, H-2); 8.23 (d, J = 7.6 Hz, 1 H, H-7). ¹³C NMR (CDCl₃, 360 MHz) δ (ppm): 23.5; 27.0; 50.8; 53.6; 55.4; 58.3; 68.5; 81.8; 93.7; 107.6; 111.3; 118.3; 121.1; 123.0; 129.8; 139.2; 141.4; 142.5; 152.4; 156.7.

### Example E44:

### 3-Bromo-5-[4-[4-(2-chlorophenyl)piperazin-1-yl]butoxy]pyrazolo[1,5-a]pyridine

Synthesis worked according to the preparation of E42 when using 5-[4-[4-(2-chlorophenyl)piperazin-1-yl]butoxy]pyrazolo[1,5-a]pyridine (E17).
Yield: 13 mg (91 %) white solid.
Mp.: 84°C. MS (APCI): m/z 467 (M+3)⁺, 465 (M+1)⁺, 463 (M-1)⁺. IR (NaCl) v (cm⁻¹): 2933; 2814; 1649; 1537; 1230; 1207; 1039; 754. ¹H NMR (CDCl₃, 360 MHz) δ (ppm): 1.71-1.81 (m, 2H, CH₂CH₂N); 1.87-1.95 (m, 2H, OCH₂CH₂); 2.54 (t, J = 7.6 Hz, 2H, CH₂N); 2.68-2.73 (m, 4H, Piperazine); 3.11-3.13 (m, 4H, Piperazine); 4.08 (t, J = 6.3 Hz, 2H, OCH₂); 6.48 (dd, J = 7.6 Hz, 2.6 Hz, 1 H, H-6); 6.66 (d, J = 2.6 Hz, 1 H, H-4); 6.98 (ddd, J = 7.8 Hz, 7.3 Hz, 1.3 Hz, 1 H, Phenyl H4); 7.05 (dd, J = 8.0 Hz, 1.3 Hz, 1 H, Phenyl H6); 7.22 (ddd, J = 8.0 Hz, 7.3 Hz, 1.5 Hz, 1H, Phenyl H5); 7.36 (dd, J = 7.8 Hz, 1.5 Hz, 1 H, Phenyl H3); 7.82 (s, 1H, H-2); 8.23 (d, J = 7.6 Hz, 1H, H-7). ¹³C NMR (CDCl₃, 360 MHz) δ (ppm): 23.3; 26.9; 51.1; 53.4; 58.1; 68.4; 81.9; 93.8; 107.7; 120.4; 123.8; 127.6; 128.8; 129.8; 130.7; 139.2; 142.5; 149.2; 156.6.

### Example E45:

### 3-Chloro-5-[4-[4-(2,3-dichlorophenyl)piperazin-1-yl]butoxy]pyrazolo[1,5-a]pyridine

Synthesis worked according to the preparation of E42 when using 5-[4-[4-(2,3-dichlorophenyl)piperazin-1-yl]butoxy]pyrazolo[1,5-a]pyridine (E15) and *N-*chlorosuccinimide and the reaction took 27 hrs.
Yield: 11 mg (78 %) white solid.
Mp.: 133°C. MS (APCI): m/z 457 (M+3)⁺, 455 (M+1)⁺, 453 (M-1)⁺. IR (NaCl) v (cm⁻¹): 2943; 2816; 1649; 1578; 1234; 1043; 773. ¹H NMR (CDCl₃, 600 MHz) δ (ppm): 1.72-1.78 (m, 2H, CH₂CH₂N); 1.88-1.93 (m, 2H, OCH₂CH₂); 2.52 (t, J = 7.6 Hz, 2H, CH₂N); 2.65-2.70 (m, 4H, Piperazine); 3.07-3.11 (m, 4H, Piperazine); 4.08 (t, J = 6.3 Hz, 2H, OCH₂); 6.47 (dd, J = 7.6 Hz, 2.6 Hz, 1 H, H-6); 6.67 (d, J = 2.6 Hz, 1 H, H-4); 6.96 (dd, J = 7.0 Hz, 2.4 Hz, 1 H, Phenyl H6); 7.13-7.17 (m, 2H, Phenyl H4, Phenyl H5); 7.79 (s, 1 H, H-2); 8.20 (d, J = 7.6 Hz, 1 H, H-7). ¹³C NMR (CDCl₃, 600 MHz) δ (ppm): 23.3; 26.8; 51.3; 53.3; 58.0; 68.3; 93.1; 98.0; 107.6; 118.6; 124.7; 127.4; 127.5; 129.8; 134.1; 137.7; 140.4; 151.3; 156.3.

### Example E46:

### 3-Chloro-5-[4-[4-(2-methoxyphenyl)piperazin-1-yl]butoxy]pyrazolo[1,5-a]pyridine

Synthesis worked according to the preparation of E45 when using 5-[4-[4-(2-methoxyphenyl)piperazin-1-yl]butoxy]pyrazolo[1,5-a]pyridine (E16).
Yield: 20 mg (96 %) white solid.
Mp.: 68°C. MS (APCI): m/z 417 (M+2)⁺, 415 (M)⁺. IR (NaCl) v (cm⁻¹): 3064; 2941; 2819; 1649; 1539; 1238; 1020; 750. ¹H NMR (CDCl₃, 600 MHz) δ (ppm): 1.72-1.78 (m, 2H, CH₂CH₂N); 1.87-1.92 (m, 2H, OCH₂CH₂); 2.52 (t, J = 7.6 Hz, 2H, CH₂N); 2.69-2.72 (m, 4H, Piperazine); 3.10-3.14 (m, 4H, Piperazine); 3.86 (s, 3H, OCH₃); 4.07 (t, J = 6.3 Hz, 2H, OCH₂); 6.47 (dd, J = 7.6 Hz, 2.6 Hz, 1 H, H-6); 6.67 (d, J = 2.6 Hz, 1 H, H-4); 6.86 (dd, J = 7.9 Hz, 1.0 Hz, 1 H, Phenyl H3); 6.92 (ddd, J = 7.5 Hz, 7.0 Hz, 1.0 Hz, 1 H, Phenyl H5); 6.95 (dd, J = 7.5 Hz, 2.0 Hz, 1 H, Phenyl H6); 7.00 (ddd, J = 7.9 Hz, 7.0 Hz, 2.0 Hz, 1 H, Phenyl H4); 7.80 (s, 1 H, H-2); 8.21 (d, J = 7.6 Hz, 1 H, H-7). ¹³C NMR (CDCl₃, 600 MHz) δ (ppm): 23.3; 27.0; 50.6; 53.4; 55.4; 58.2; 68.4; 93.1; 98.1; 107.6; 111.2; 118.2; 121.0; 123.0; 129.8; 137.8; 140.5; 141.3; 152.3; 156.4.
C H N (%):C₂₂H₂₇N₄O₂Cl x 0.3H₂O; calculated: C 62.86; H 6.62; N 13.33; found: C 63.06; H 6.54; N 13.07.

### Example E47:

### 3-Chloro-5-[4-[4-(2-chlorophenyl)piperazin-1-yl]butoxy]pyrazolo[1,5-a]pyridine

Synthesis worked according to the preparation of E45 when using 5-[4-[4-(2-chlorophenyl)piperazin-1-yl]butoxy]pyrazolo[1,5-a]pyridine (E17).
Yield: 14 mg (68 %) beige solid.
Mp.: 63°C. MS (APCI): m/z 421 (M+2)⁺, 419 (M)⁺. IR (NaCl) v (cm⁻¹): 3062; 2943; 2818; 1649; 1539; 1232; 1211; 1039; 770. ¹H NMR (CDCl₃, 600 MHz) δ (ppm): 1.74-1.79 (m, 2H, CH₂CH₂N); 1.88-1.93 (m, 2H, OCH₂CH₂); 2.55 (t, J = 7.6 Hz, 2H, CH₂N); 2.68-2.73 (m, 4H, Piperazine); 3.10-3.14 (m, 4H, Piperazine); 4.08 (t, J = 6.3 Hz, 2H, OCH₂); 6.48 (dd, J = 7.6 Hz, 2.6 Hz, 1 H, H-6); 6.67 (d, J = 2.6 Hz, 1 H, H-4); 6.97 (ddd, J = 7.8 Hz, 7.3 Hz, 1.3 Hz, 1 H, Phenyl H4); 7.05 (dd, J = 8.0 Hz, 1.3 Hz, 1 H, Phenyl H6); 7.22 (ddd, J = 8.0 Hz, 7.3 Hz, 1.5 Hz, 1 H, Phenyl H5); 7.36 (dd, J = 7.8 Hz, 1.5 Hz, 1 H, Phenyl H3); 7.80 (s, 1 H, H-2); 8.21 (d, J = 7.6 Hz, 1H, H-7). ¹³C NMR (CDCl₃, 600 MHz) δ (ppm): 23.3; 26.9; 51.1; 53.4; 58.1; 68.3; 93.1; 98.1; 107.6; 120.4; 123.8; 127.7; 128.8; 129.8; 130.7; 137.8; 140.5; 149.2; 156.4.
C H N (%):C₂₁H₂₄N₄OCl₂ x 0.5H₂O; calculated: C 58.88; H 5.88; N 13.08; found: C 58.90; H 5.76; N 12.91.

### Example E48:

### 5-[4-[4-(2,3-Dichlorophenyl)piperazin-1-yl]butoxy]3-iodo-pyrazolo[1,5-a]pyridine

Synthesis worked according to the preparation of E42 when using 5-[4-[4-(2,3-dichlorophenyl)piperazin-1-yl]butoxy]pyrazolo[1,5-a]pyridine (E15) and *N-*iodosuccinimide and the reaction took 2 hrs.
Yield: 16 mg (98 %) beige solid.
Mp.: 110°C. MS (APCI): m/z 546 (M+1)⁺. IR (NaCl) v (cm⁻¹): 3082; 2947; 2818; 1647; 1578; 1228; 1194; 1045; 775. ¹H NMR (CDCl₃, 600 MHz) δ (ppm): 1.74-1.79 (m, 2H, CH₂CH₂N); 1.88-1.93 (m, 2H, OCH₂CH₂); 2.54 (t, J = 7.6 Hz, 2H, CH₂N); 2.67-2.72 (m, 4H, Piperazine); 3.08-3.12 (m, 4H, Piperazine); 4.09 (t, J = 6.3 Hz, 2H, OCH₂); 6.48 (dd, J = 7.6 Hz, 2.6 Hz, 1 H, H-6); 6.62 (d, J = 2.6 Hz, 1 H, H-4); 6.96 (dd, J = 7.0 Hz, 2.4 Hz, 1 H, Phenyl H6); 7.12-7.17 (m, 2H, Phenyl H4, Phenyl H5); 7.85 (s, 1 H, H-2); 8.27 (d, J = 7.6 Hz, 1 H, H-7). ¹³C NMR (CDCl₃, 600 MHz) δ (ppm): 23.3; 27.0; 45.5; 51.2; 53.2; 58.0; 68.3; 95.2; 107.7; 118.7; 124.7; 127.4; 127.5; 129.9; 134.0; 142.0; 146.8; 151.2; 157.2.
C H N (%):C₂₁H₂₃N₄OCl₂l; calculated: C 46.26; H 4.25; N 10.28; found: C 46.19; H 4.44; N 10.38.

### Example E49:

### 3-Iodo-5-[4-[4-(2-methoxyphenyl)piperazin-1-yl]butoxy]pyrazolo[1,5-a]pyridine

Synthesis worked according to the preparation of E48 when using 5-[4-[4-(2-methoxyphenyl)piperazin-1-yl]butoxy]pyrazolo[1,5-a]pyridine (E16).
Yield: 18 mg (91 %) light yellow solid.
Mp.: 61°C. MS (EI): m/z 506 (M)⁺. IR (NaCl) v (cm⁻¹): 3064; 2941; 2819; 1647; 1537; 1240; 1026; 750. ¹H NMR (CDCl₃, 600 MHz) δ (ppm): 1.74-1.79 (m, 2H, CH₂CH₂N); 1.87-1.93 (m, 2H, OCH₂CH₂); 2.53 (t, J = 7.6 Hz, 2H, CH₂N); 2.69-2.73 (m, 4H, Piperazine); 3.11-3.16 (m, 4H, Piperazine); 3.86 (s, 3H, OCH₃); 4.09 (t, J = 6.3 Hz, 2H, OCH₂); 6.47 (dd, J = 7.6 Hz, 2.6 Hz, 1 H, H-6); 6.61 (d, J = 2.6 Hz, 1 H, H-4); 6.86 (dd, J = 7.9 Hz, 1.0 Hz, 1H, Phenyl H3); 6.92 (ddd, J = 7.5 Hz, 7.0 Hz, 1.0 Hz, 1 H, Phenyl H5); 6.96 (dd, J = 7.5 Hz, 2.0 Hz, 1H, Phenyl H6); 7.00 (ddd, J = 7.9 Hz, 7.0 Hz, 2.0 Hz, 1 H, Phenyl H4); 7.85 (s, 1 H, H-2); 8.27 (d, J = 7.6 Hz, 1 H, H-7). ¹³C NMR (CDCl₃, 600 MHz) δ (ppm): 23.4; 27.0; 45.4; 50.6; 53.4; 55.4; 58.2; 68.4; 95.2; 107.7; 111.2; 118.3; 121.1; 123.1; 129.9; 141.3; 142.1; 146.9; 152.4; 157.2.

### Example E50:

### 5-[4-[4-(2-Chlorophenyl)piperazin-1-yl]butoxy]-3-iodo-pyrazolo[1,5-a]pyridine

Synthesis worked according to the preparation of E48 when using 5-[4-[4-(2-chlorophenyl)piperazin-1-yl]butoxy]pyrazolo[1,5-a]pyridine (E17).
Yield: 10 mg (99 %) light yellow solid.
Mp.: 94°C. MS (APCI): m/z 512 (M+1)⁺. IR (NaCl) v (cm⁻¹): 3062; 2941; 2818; 1647; 1537; 1228; 1200; 1039; 752. ¹H NMR (CDCl₃, 360 MHz) δ (ppm): 1.73-1.81 (m, 2H, CH₂CH₂N); 1.87-1.95 (m, 2H, OCH₂CH₂); 2.55 (t, J = 7.6 Hz, 2H, CH₂N); 2.69-2.73 (m, 4H, Piperazine); 3.11-3.13 (m, 4H, Piperazine); 4.09 (t, J = 6.3 Hz, 2H, OCH₂); 6.48 (dd, J = 7.6 Hz, 2.6 Hz, 1 H, H-6); 6.62 (d, J = 2.6 Hz, 1 H, H-4); 6.97 (ddd, J = 7.8 Hz, 7.3 Hz, 1.3 Hz, 1 H, Phenyl H4); 7.05 (dd, J = 8.0 Hz, 1.3 Hz, 1H, Phenyl H6); 7.22 (ddd, J = 8.0 Hz, 7.3 Hz, 1.5 Hz, 1 H, Phenyl H5); 7.35 (dd, J = 7.8 Hz, 1.5 Hz, 1H, Phenyl H3); 7.85 (s, 1 H, H-2); 8.26 (d, J = 7.6 Hz, 1H, H-7). ¹³C NMR (CDCl₃, 360 MHz) δ (ppm): 23.3; 27.1; 45.5; 51.1; 53.4; 58.1; 68.4; 95.2; 107.7; 120.4; 123.8; 127.6; 128.8; 129.9; 130.7; 142.0; 146.9; 149.2; 157.1.

### 6. Synthesis of examplary compounds according to formula XVII

The synthesis of compounds according to formula XVII started from formyl derivatives of formula XVIa, which were aminated with hydroxylamine to get the Example compounds E30 to E35 (formula XVII, R1" = HCNOH) and subsequently dehydratised to obtain the Example compounds E36 to E38 (formula XVII, R1" = CN):

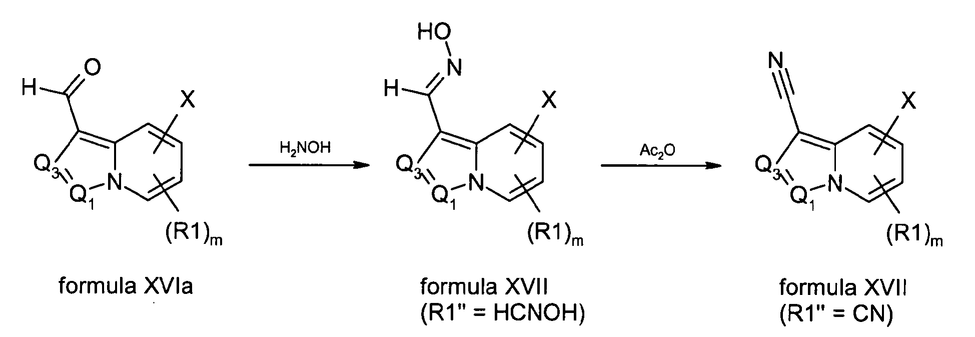

### Example E30:

### (s-trans) 5-[4-[4-(2,3-Dichlorophenyl)piperazin-1-yl]butoxy]pyrazolo[1,5-a]pyridine-3-carbaldehyde oxime

A solution of 18.0 mg (0.26 mmol) hydroxylamine hydrochloride in 0.74 ml water and 0.07 ml (0.13 mmol) 2N NaOH were cooled to 0°C and adjusted to pH = 5 with 2N HCl. Then, 57.8 mg (0.13 mmol) 5-[4-[4-(2,3-dichlorophenyl)piperazin-1-yl]butoxy]pyrazolo[1,5-a]pyridine-3-carbaldehyde (E21) solved in 6.45 ml ethanol were added dropwise and refluxed for 2 hrs. After cooling to room temperature, saturated NaHCO₃-solution was added and the mixture was extracted with dichloromethane. The organic layer was dried with Na₂SO₄, evaporated and purified by flash-chromatography (CH₂Cl₂/MeOH 98:2). Two isomeres were isolated with the first one representing the *s-trans* isomer.
Yield: 25 mg (42 %) white solid.
Mp.: 189°C. MS (APCl): m/z 464 (M+2)⁺, 462 (M)⁺. IR (KBr) v (cm⁻¹): 3446; 3078; 2956; 2831; 1647; 1578; 1535; 1267; 1244; 1038; 789. ¹H NMR (DMSO-d₆, 360 MHz) δ (ppm): 1.60-1.68 (m, 2H, CH₂CH₂N); 1.79-1.86 (m, 2H, OCH₂CH₂); 2.44 (br t, J = 7.6 Hz, 2H, CH₂N); 2.52-2.60 (m, 4H, Piperazine); 2.97-3.00 (m, 4H, Piperazine); 4.09 (t, J = 6.4 Hz, 2H, OCH₂); 6.68 (dd, J = 7.6 Hz, 2.6 Hz, 1H, H-6); 7.11 (dd, J = 6.5 Hz, 3.0 Hz, 1 H, Phenyl H6); 7.26-7.30 (m, 3H, Phenyl H4, Phenyl H5, H-4); 8.08 (s, 1H, H-2); 8.29 (s, 1 H, CHNOH); 8.58 (d, J = 7.6 Hz, 1 H, H-7); 10.63 (s, 1 H, OH). ¹³C NMR (DMSO-d₆, 360 MHz) δ (ppm): 23.2; 26.9; 51.4; 53.3; 57.8; 68.6; 97.0; 104.4; 108.0; 120.0; 124.9; 126.6; 129.0; 130.0; 133.2; 138.6; 142.5; 143.1; 151.6; 157.5.
C H N (%): C₂₂H₂₅N₅O₂Cl₂ x 0.4H₂O; calculated: C 56.27; H 5.54; N 14.91; found: C 56.48; H 5.47; N 14.69.

### Example E33:

### (s-cis) 5-[4-[4-(2,3-Dichlorophenyl)piperazin-1-yl]butoxy]pyrazolo[1,5-a]pyridine-3-carbaldehyde oxime

Synthesis worked identical with the preparation of E30. Flash-chromatographie yielded two isomers with the second one representing the s-cis isomer.
Yield: 24 mg (39 %) white solid.
Mp.: 165°C. MS (EI): m/z 463 (M+1)⁺, 461 (M)⁺. IR (NaCl) v (cm⁻¹): 3425; 2846; 1684; 1649; 1542; 1204; 1051; 764. ¹H NMR (DMSO-d₆, 360 MHz) δ (ppm): 1.60-1.68 (m, 2H, CH₂CH₂N); 1.79-1.86 (m, 2H, OCH₂CH₂); 2.43 (t, J = 7.6 Hz, 2H, CH₂N); 2.52-2.58 (m, 4H, Piperazine); 2.97-3.00 (m, 4H, Piperazine); 4.14 (t, J = 6.3 Hz, 2H, OCH₂); 6.68 (dd, J = 7.6 Hz, 2.6 Hz, 1 H, H-6); 7.11 (dd, J = 6.5 Hz, 3.0 Hz, 1H, Phenyl H6); 7.27-7.30 (m, 2H, Phenyl H4, Phenyl H5); 7.45 (d, J = 2.6 Hz, 1 H, H-4); 7.75 (s, 1 H, H-2); 8.56-8.59 (m, 2H, CHNOH, H-7); 11.06 (s, 1H, OH). ¹³C NMR (DMSO-d₆, 360 MHz) δ (ppm): 23.2; 26.8; 51.5; 53.3; 57.7; 68.8; 95.6; 103.1; 108.0; 120.0; 124.8; 126.5; 128.9; 130.6; 133.1; 137.0; 140.5; 145.6; 151.7; 157.4.
C H N (%): C₂₂H₂₅N₅O₂Cl₂ x 0.5H₂O; calculated: C 56.06; H 5.56; N 14.86; found: C 55.91; H 5.40; N 14.73.

### Example E31:

### (s-trans) 5-[4-[4-(2-Methoxyphenyl)piperazin-1-yl]butoxy]pyrazolo[1,5-a]pyridine-3-carbaldehyde oxime

Synthesis worked according to the preparation of E30 when using 5-[4-[4-(2-methoxyphenyl)piperazin-1-yl]butoxy]pyrazolo[1,5-a]pyridine-3-carbaldehyde (E22).
Yield: 42 mg (46 %) white solid.
Mp.: 77°C. MS (EI): m/z 423 (M)⁺. IR (NaCl) v (cm⁻¹): 3257; 3064; 2945; 2823; 1647; 1541; 1269; 1242; 1026; 750. ¹H NMR (CDCl₃, 360 MHz) δ (ppm): 1.78-1.83 (m, 2H, CH₂CH₂N); 1.86-1.90 (m, 2H, OCH₂CH₂); 2.55 (t, J = 7.6 Hz, 2H, CH₂N); 2.71-2.77 (m, 4H, Piperazine); 3.14-3.20 (m, 4H, Piperazine); 3.87 (s, 3H, OCH₃); 4.05 (t, J = 6.3 Hz, 2H, OCH₂); 6.45 (dd, J = 7.6 Hz, 2.6 Hz, 1 H, H-6); 6.86 (dd, J = 7.9 Hz, 1.0 Hz, 1 H, Phenyl H3); 6.91 (ddd, J = 7.5 Hz, 7.0 Hz, 1.0 Hz, 1 H, Phenyl H5); 6.96 (dd, J = 7.5 Hz, 2.0 Hz, 1 H, Phenyl H6); 7.00 (ddd, J = 7.9 Hz, 7.0 Hz, 2.0 Hz, 1H, Phenyl H4); 7.20 (d, J = 2.6 Hz, 1 H, H-4); 7.93 (s, 1 H, H-2); 8.22 (d, J = 7.6 Hz, 1H, H-7); 8.27 (s, 1 H, CHNOH). ¹³C NMR (CDCl₃, 360 MHz) δ (ppm): 23.3; 27.1; 50.5; 53.5; 55.5; 58.3; 68.3; 97.2; 103.8; 107.9; 111.3; 118.4; 121.1; 123.1; 129.6; 138.6; 141.1; 143.3; 143.6; 152.3; 157.6.
C H N (%): C₂₃H₂₉N₅O₃ x 0.5H₂O; calculated: C 63.87; H 6.99; N 16.19; found: C 63.88; H 6.88; N 16.02.

### Example E34:

### (s-cis) 5-[4-[4-(2-Methoxyphenyl)piperazin-1-yl]butoxy]pyrazolo[1,5-a]pyridine-3-carbaldehyde oxime

Synthesis worked identical with the preparation of E31. Flash-chromatographie yielded two isomers with the second one representing the s-cis isomer.
Yield: 39 mg (43 %) white solid.
Mp.: 138°C. MS (EI): m/z 423 (M)⁺. IR (NaCl) v (cm⁻¹): 3224; 2945; 2821; 1647; 1541; 1238; 1026; 750. ¹H NMR (CDCl₃, 360 MHz) δ (ppm): 1.74-1.82 (m, 2H, CH₂CH₂N); 1.87-1.94 (m, 2H, OCH₂CH₂); 2.54 (t, J = 7.6 Hz, 2H, CH₂N); 2.70-2.75 (m, 4H, Piperazine); 3.12-3.18 (m, 4H, Piperazine); 3.87 (s, 3H, OCH₃); 4.08 (t, J = 6.3 Hz, 2H, OCH₂); 6.54 (dd, J = 7.6 Hz, 2.6 Hz, 1 H, H-6); 6.86 (dd, J = 7.9 Hz, 1.0 Hz, 1H, Phenyl H3); 6.88-7.02 (m, 4H, Phenyl H4, Phenyl H5, Phenyl H6, H-4); 7.50 (s, 1 H, H-2); 8.30 (d, J = 7.6 Hz, 1 H, H-7); 8.67 (s, 1 H, CHNOH). ¹³C NMR (CDCl₃, 360 MHz) δ (ppm): 23.3; 26.8; 50.5; 53.5; 55.4; 58.1; 68.3; 94.4; 102.4; 107.7; 111.2; 118.3; 121.1; 123.0; 129.9; 137.9; 140.5; 141.3; 146.2; 152.3; 157.6.
C H N (%): C₂₃H₂₉N₅O₃ x 0.7H₂O; calculated: C 63.34; H 7.03; N 16.06; found: C 63.21; H 6.77; N 15.90.

### Example E32:

### (s-trans) 5-[4-[4-(2-Chlorophenyl)piperazin-1-yl]butoxy[pyrazolo[1,5-a]pyridine-3-carbaldehyde oxime

Synthesis worked according to the preparation of E30 when using 5-[4-[4-(2-chlorophenyl)piperazin-1-yl]butoxy]pyrazolo[1,5-a]pyridine-3-carbaldehyde (E23).
Yield: 69 mg (44 %) white solid.
Mp.: 174°C. MS (APCI): m/z 430 (M+2)⁺, 428 (M)⁺. IR (KBr) v (cm⁻¹): 3440; 3066; 2943; 2825; 1645; 1539; 1273; 1238; 1036; 756. ¹H NMR (DMSO-d₆, 360 MHz) δ (ppm): 1.61-1.66 (m, 2H, CH₂CH₂N); 1.80-1.84 (m, 2H, OCH₂CH₂); 2.43 (t, J = 7.6 Hz, 2H, CH₂N); 2.52-2.59 (m, 4H, Piperazine); 2.94-3.00 (m, 4H, Piperazine); 4.09 (t, J = 6.3 Hz, 2H, OCH₂); 6.69 (dd, J = 7.6 Hz, 2.6 Hz, 1 H, H-6); 7.03 (ddd, J = 7.8 Hz, 7.3 Hz, 1.3 Hz, 1H. Phenyl H4); 7.12 (dd, J = 8.0 Hz, 1.3 Hz, 1H. Phenyl H6); 7.27 (ddd, J = 8.0 Hz, 7.3 Hz, 1.5 Hz, 1 H, Phenyl H5); 7.29 (d, J = 2.6 Hz, 1 H, H-4); 7.39 (dd, J = 7.8 Hz, 1.5 Hz, 1 H, Phenyl H3); 8.08 (s, 1 H, H-2); 8.29 (s, 1 H, CHNOH); 8.59 (d, J = 7.6 Hz, 1 H, H-7); 10.66 (s, 1 H, OH). ¹³C NMR (DMSO-d₆, 360 MHz) δ (ppm): 23.1; 26.8; 51.3; 53.3; 57.8; 68.6; 96.9; 104.3; 108.0; 121.3; 124.3; 128.1; 128.5; 130.9; 138.5; 142.5; 143.0; 149.5; 157.4.
C H N (%):C₂₂H₂₆N₅O₂Cl x 0.1 H₂O; calculated: C 61.49; H 6.15; N 16.29; found: C 61.51; H 6.24; N 15.99.

### Example E35:

### (s-cis) 5-[4-[4-(2-Chlorophenyl)piperazin-1-yl]butoxy[pyrazolo[1,5-a]pyridine-3-carbaldehyde oxime

Synthesis worked identical with the preparation of E32. Flash-chromatographie yielded two isomers with the second one representing the s-cis isomer.
Yield: 82 mg (52 %) white solid.
Mp.: 160°C. MS (APCI): m/z 430 (M+2)⁺, 428 (M)⁺. IR (KBr) v (cm⁻¹): 3444; 3055; 2951; 2823; 1647; 1529; 1232; 1038; 752. ¹H NMR (DMSO-d₆, 600 MHz) δ (ppm): 1.62-1.67 (m, 2H, CH₂CH₂N); 1.80-1.85 (m, 2H, OCH₂CH₂); 2.43 (t, J = 7.6 Hz, 2H, CH₂N); 2.53-2.58 (m, 4H, Piperazine); 2.95-2.99 (m, 4H, Piperazine); 4.14 (t, J = 6.3 Hz, 2H, OCH₂); 6.69 (dd, J = 7.6 Hz, 2.6 Hz, 1 H, H-6); 7.03 (ddd, J = 7.8 Hz, 7.3 Hz, 1.3 Hz, 1 H, Phenyl H4); 7.13 (dd, J = 8.0 Hz, 1.3 Hz, 1 H, Phenyl H6); 7.28 (ddd, J = 8.0 Hz, 7.3 Hz, 1.5 Hz, 1 H, Phenyl H5); 7.39 (dd, J = 7.8 Hz, 1.5 Hz, 1 H, Phenyl H3); 7.46 (d, J = 2.6 Hz, 1 H, H-4); 7.76 (s, 1 H, H-2); 8.56-8.59 (m, 2H, CHNOH, H-7); 11.08 (s, 1 H, OH). ¹³C NMR (DMSO-d₆, 360 MHz) δ (ppm): 23.2; 26.7; 51.4; 53.4; 57.8; 68.8; 95.7; 103.1; 108.1; 121.3; 124.3; 128.1; 128.5; 130.6; 130.8; 137.1; 140.5; 145.6; 149.6; 157.4.
C H N (%): C₂₂H₂₆N₅O₂Cl x 0.7 H₂O; calculated: C 59.98; H 6.27; N 15.90; found: C 60.23; H 6.11; N 15.63.

### Example E36:

### 5-[4-[4-(2,3-Dichlorophenyl)piperazin-1-yl]butoxy]pyrazolo[1,5-a]pyridine-3-carbonitrile

For the synthesis 126 mg (0.272 mmol) 5-[4-[4-(2,3-dichlorophenyl)piperazin-1-yl]butoxy] pyrazolo[1,5-a]pyridine-3-carbaldehyde oxime (E30 or E33) and 1.35 ml acetic anhydride were refluxed for 8 hrs. After adding ice the mixture was extracted with chloroform and the organic layer was dried with Na₂SO₄, evaporated and purified by flash-chromatography (CH₂Cl₂/MeOH 98:2).
Yield: 86 mg (72 %) beige solid.
Mp.: 118°C. MS (APCI): m/z 446 (M+2)⁺, 444 (M)⁺. IR (NaCl) v (cm⁻¹): 3065; 2948; 2817; 2219; 1648; 1578; 1542; 1243; 1064; 781. ¹H NMR (CDCl₃, 600 MHz) δ (ppm): 1.73-1.78 (m, 2H, CH₂CH₂N); 1.90-1.95 (m, 2H, OCH₂CH₂); 2.53 (t, J = 7.6 Hz, 2H, CH₂N); 2.67-2.72 (m, 4H, Piperazine); 3.07-3.12 (m, 4H, Piperazine); 4.11 (t, J = 6.4 Hz, 2H, OCH₂); 6.66 (dd, J = 7.6 Hz, 2.6 Hz, 1 H, H-6); 6.94 (d, J = 2.6 Hz, 1 H, H-4); 6.96 (dd, J = 7.2 Hz, 2.2 Hz, 1 H, Phenyl H6); 7.13-7.17 (m, 2H, Phenyl H4, Phenyl H5); 8.11 (s, 1H, H-2); 8.35 (d, J = 7.6 Hz, 1 H, H-7). ¹³C NMR (CDCl₃, 600 MHz) δ (ppm): 23.2; 26.8; 51.3; 53.3; 58.0; 68.9; 80.9; 95.0; 109.3; 114.5; 118.6; 124.7; 127.4; 127.5; 130.5; 134.0; 144.4; 145.6; 151.2; 159.2.

### Example E37:

### 5-[4-[4-(2-Methoxyphenyl)piperazin-1-yl]butoxy]pyrazolo[1,5-a]pyridine-3-carbonitrile

Synthesis worked according to the preparation of E36 when using 5-[4-[4-(2-methoxyphenyl)piperazin-1-yl]butoxy]pyrazolo[1,5-a]pyridine-3-carbaldehyde oxime (E31 or E34).
Yield: 30 mg (79 %) white solid.
Mp.: 92°C. MS (EI): m/z 405 (M)⁺. IR (NaCl) v (cm⁻¹): 3070; 2937; 2816; 2219; 1648; 1542; 1240; 1028; 749. ¹H NMR (CDCl₃, 360 MHz) δ (ppm): 1.71-1.80 (m, 2H, CH₂CH₂N); 1.88-1.96 (m, 2H, OCH₂CH₂); 2.52 (t, J = 8.0 Hz, 2H, CH₂N); 2.68-2.72 (m, 4H, Piperazine); 3.10-3.14 (m, 4H, Piperazine); 3.86 (s, 3H, OCH₃); 4.11 (t, J = 6.1 Hz, 2H, OCH₂); 6.66 (dd, J = 7.6 Hz, 2.6 Hz, 1 H, H-6); 6.86 (dd, J = 7.8 Hz, 1.4 Hz, 1H, Phenyl H3); 6.91-6.96 (m, 3H, Phenyl H5, Phenyl H6, H-4); 7.02 (ddd, J = 7.8 Hz, 6.8 Hz, 2.5 Hz, 1 H, Phenyl H4); 8.10 (s, 1 H, H-2); 8.34 (d, J = 7.6 Hz, 1 H, H-7). ¹³C NMR (CDCl₃, 360 MHz) δ (ppm): 23.3; 26.9; 50.7; 53.6; 55.5; 58.1; 69.0; 80.9; 95.0; 109.3; 111.3; 114.4; 118.2; 121.0; 122.9; 130.5; 141.4; 144.4; 145.6; 152.4; 159.3.

### Example E38:

### 5-[4-[4-(2-Chlorophenyl)piperazin-1-yl]butoxy]pyrazolo[1,5-a]pyridine-3-carbonitrile

Synthesis worked according to the preparation of E36 when using 5-[4-[4-(2-chlorophenyl)piperazin-1-yl]butoxy]pyrazolo[1,5-a]pyridine-3-carbaldehyde oxime (E32 or E35).
Yield: 44 mg (66 %) light yellow solid.
Mp.: 119°C. MS (EI): m/z 411 (M+1)⁺, 409 (M-1)⁺. IR (NaCl) v (cm⁻¹): 3061; 2944; 2815; 2220; 1648; 1543; 1244; 1038; 750. ¹H NMR (CDCl₃, 360 MHz) δ (ppm): 1.71-1.79 (m, 2H, CH₂CH₂N); 1.89-1.96 (m, 2H, OCH₂CH₂); 2.52 (t, J = 7.6 Hz, 2H, CH₂N); 2.65-2.70 (m, 4H, Piperazine); 3.07-3.12 (m, 4H, Piperazine); 4.11 (t, J = 6.4 Hz, 2H, OCH₂); 6.66 (dd, J = 7.6 Hz, 2.6 Hz, 1 H, H-6); 6.92-6.99 (m, 2H, H-4, Phenyl H4); 7.05 (dd, J = 8.1 Hz, 1.5 Hz, 1 H, Phenyl H6); 7.22 (ddd, J = 8.1 Hz, 7.3 Hz, 1.2 Hz, 1H, Phenyl H5); 7.35 (dd, J = 7.8 Hz, 1.2 Hz, 1 H, Phenyl H3); 8.11 (s, 1H, H-2); 8.34 (d, J = 7.6 Hz, 1 H, H-7). ¹³C NMR (CDCl₃, 360 MHz) δ (ppm): 23.4; 26.9; 51.3; 53.4; 58.1; 69.0; 80.9; 95.0; 109.2; 114.5; 120.4; 123.7; 127.6; 128.8; 130.5; 130.6; 144.4; 145.7; 149.4; 159.3.

### 7. Synthesis of exemplary compounds

### 7a) Heteroarene components according to formula XI 7-Hydroxyindolizine (C4j), 6-hydroxyimidazo[1,2-a]pyridine (C4k), 6-hydroxyimidazo[1,2-a]pyridine (C4l),

Heteroarene components according to formula XI are available by suppliers of chemical substances:

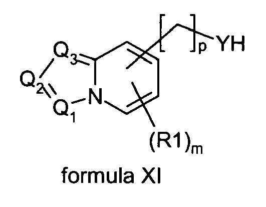

For example, the following precursors can be purchased:
*7-Hydroxyindolizine (C4j)*
from *Chemstep, Carbon Blanc (France)* (Q1, Q2, Q3 = CH; Y = O; m, p = 0 - [Order number: 40238])
*6-Hydroxyimidazo[1,2-a]pyridine* (C4k)
from *Anichem LLC, Monmouth Junction (NJ)* (Q1, Q2 = CH; Q3 = N; Y = O; m, p = 0 - [Order number: A82110])
*8-Hydroxyimidazo[1,2-a]pyridine* (C4l)
from *ChemPur, Karlsruhe (Germany)* (Q1, Q2 = CH; Q3 = N; Y = O; m, p = 0 - [Order number: 8X-0841 ])
or from *Chemstep, Carbon Blanc (France)* (Q1, Q2 = CH; Q3 = N; Y = O; m, p = 0 - [Order number: 40412]).

### 7b) Synthesis of the heteroarene components according to formula XIII 7-(4-Bromobutoxy)indolizine (C6j), 6-(4-bromobutoxy)imidazo[1,2-a]pyridine (C6k), 8-(4-bromobutoxy)imidazo[1,2-a]pyridine (C6l),

### 7-(4-Bromobutoxy)indolizine (C6j)

Synthesis can be achieved according to the preparation of C6a when using 7-hydroxyindolizine (C4j).

### 6-(4-Bromobutoxy)imidazo[1,2-a]pyridine (C6k)

Synthesis can be achieved according to the preparation of C6a when using 6-hydroxyimidazo[1,2-a]pyridine (C4k).

### 8-(4-Bromobutoxy)imidazo[1,2-a]pyridine (C6l)

Synthesis can be achieved according to the preparation of C6a when using 8-hydroxyimidazo[1,2-a]pyridine (C4I).

### 7c) Phenylpiperazine derivatives according to formula XIV

*1-(3-Trifluoromethylphenyl)piperazine* (C7g), *1-(3-Nitrophenyl)piperazine* (C7h),

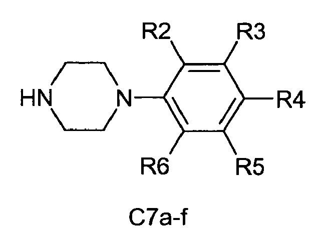

Derivatives according to formula C7g-h can be purchased:
*1-(3-Trifluoromethylphenyl)piperazine* (C7g)
from *Alfa Aesar, Karlsruhe (Germany)* (R3 = CF₃; R2, R4-R6 = H - [Order number: L05333])
*1-(3-Nitrophenyl)piperazine* (C7h)
from *ChemPur, Karlsruhe (Germany)* (R3 = NO₂; R2, R4-R6 = H - [Order number: fI19978])

### 7d) Synthesis of phenylpiperazine derivatives according to formula XIV 1-(2,3-Dihydrobenzofuran-7-yl)piperazine (C7i), 1-(8-chroman-8-yl)piperazine (C7j), 1-(2,3-dichlorophenyl)-1,4-diazepane (C7k), 1-(2-methoxyphenyl)-1,4-diazepane (C7l), 1-(2-chlorophenyl)-1,4-diazepane (C7m), 1-(2,3-dihydrobenzofuran-7-yl)-1,4-diazepane (C7n), 1-(8-chroman-8-yl)-1,4-diazepane (C7o)

For the general synthesis of annulated phenylpiperazine derivatives according to formula C7 ring closure reaction of a halogene substituted phenole C8a with a bishalogenated alkylene gives the annulated compound C8b which can be substituted with piperazine to the products C7.

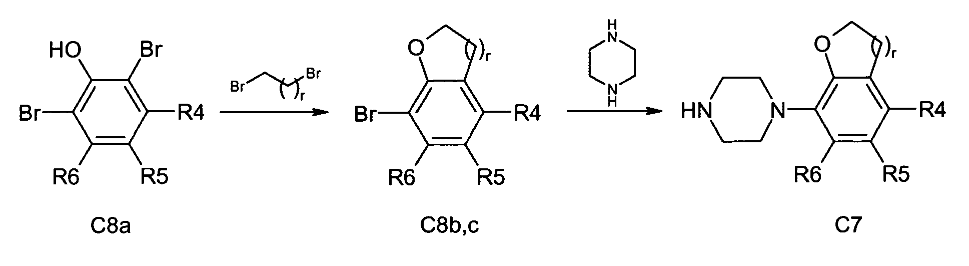

### 1-(2,3-Dihydrobenzofuran-7-yl)piperazine (C7i)

In detail, for the synthesis of compound C7i 2,6-dibromophenole (29 mmol) (C8a; R4-R6 = H) is mixed with 1,2-dibromoethane (29 mmol) (r = 1) unter basic aqueous conditions (NaOH) and heated at reflux for 18 hrs. The resulting monoalkylation product can be solved in THF/hexane (4/1), cooled to -80 °C and then, a solution of 2,5 M butyllithium (18mmol) in hexane is added dropwise (17.1 mmol). The cyclisation product 7-bromo-2,3-dihydrobenzofurane (4 mmol) (C8b; r = 1) can be suspended together with NaOtBu (20 mmol), Pd₂(dba)₃ (2 mol%), BINAP (2 mol%) and piperazine (8 mmol) in 5 ml dry toluene and heated at 117°C for 6 hrs to get the product.

### 1-(Chroman-8-yl)piperazine (C7j)

Synthesis can be achieved according to the preparation of C7i when using 1,3-dibromopropane (r = 2) to get the intermediate C8c (r = 2) which is reacted with piperazine to C7j.

For the general synthesis of phenyl substituted cyclic diamine derivatives according to formula XIV the bromo phenyl derivative C9a can be substituted with the cyclic diamine C8b to achive the compounds of formula XIV.

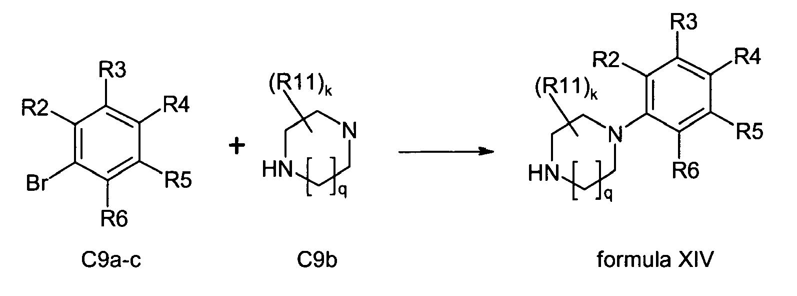

### 1-(2,3-Dichlorophenyl)-1,4-diazepane (C7k)

Synthesis can be achieved according to the last step of the preparation of C7i when using 1-bromo-2,3-dichlorobenzene (C9a: R2, R3 = Cl; R4-R6 = H) and 1,4-diazeipane (C9b: q = 2; k = 0).

### 1-(2-Methoxyphenyl)-1,4-diazepane (C7l)

Synthesis can be achieved according to the last step of the preparation of C7i when using 1-bromo-2-methoxybenzene (C9a: R2 = OMe; R3-R6 = H) and 1,4-diazepane (C9b: q = 2; k = 0).

### 1-(2-Chlorophenyl)-1,4-diazepane (C7m)

Synthesis can be achieved according to the last step of the preparation of C7i when using 1-bromo-2-chlorobenzene (C9a: R2 = Cl; R3-R6 = H) and 1,4-diazepane (C9b: q = 2; k = 0).

### 1-(2,3-Dihydrobenzofuran-7-yl)-1,4-diazepane (C7n)

Synthesis can be achieved according to the last step of the preparation of C7i when using 7-bromo-2,3-dihydrobenzofuran (C8b: R4-R6 = H; r = 1) and 1,4-diazepane (C9b: q = 2; k = 0).

### 1-(Chroman-8-yl)-1,4-diazepane (C7o)

Synthesis can be achieved according to the last step of the preparation of C7i when using 8-bromochroman (C8c: R4-R6 = H; r = 2) and 1,4-diazepane (C9b: q = 2; k = 0).

### 7e) Synthesis of example compounds according to formula XV

### Example E64:

### 5-[4-[4-(3-Trifluoromethylphenyl)piperazin-1-yl]butoxy]pyrazolo[1,5-a]pyridine

Synthesis works according to the preparation of E15 when using 5-(4-bromobutoxy)pyrazolo[1,5-a]pyridine (C6a) and 1-(3-trifluoromethylphenyl)piperazine (C7g).

### Example E65:

### 5-[4-[4-(3-Nitrophenyl)piperazin-1-yl]butoxy]pyrazolo[1,5-a]pyridine

Synthesis works according to the preparation of E15 when using 5-(4-bromobutoxy)pyrazolo[1,5-a]pyridine (C6a) and 1-(3-nitrophenyl)piperazine (C7h).

### Example E66:

### 5-[4-[4-(3-Aminophenyl)piperazin-1-yl]butoxy]pyrazolo[1,5-a]pyridine

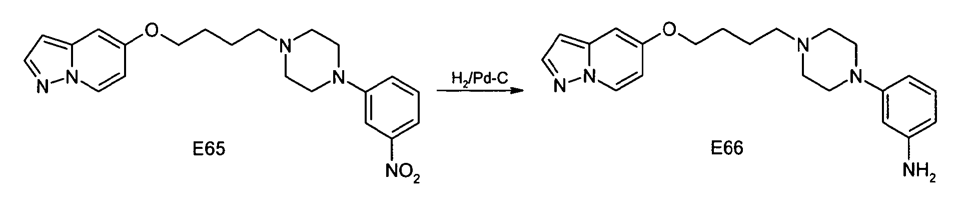

Synthesis works when reducing the nitro derivative E65 with hydrogene at palladium catalyst on charcoal for 3 hrs at room temperature in ethanol to get E66.

### Example E67:

### N-[3-[4-[4-(Pyrazolo[1,5-a]pyridin-5-yloxy)butyl]piperazin-1-yl]phenyl]acetamide

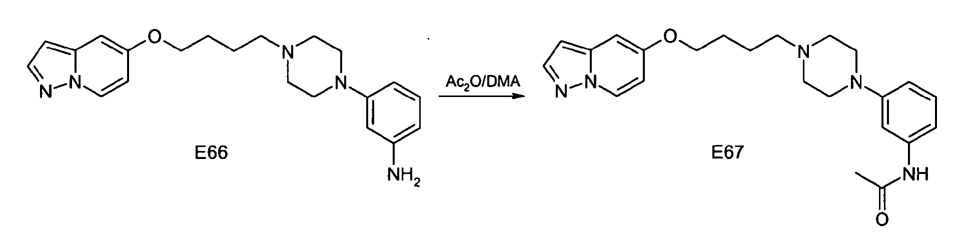

Synthesis works when acetylating the amino derivative E66 under the catalytic action of dimethylaminopyridine (DMA) with acetanhydride for 3 hrs at room temperature to achieve E67.

### Example E83:

### 5-[4-(4-(2,3-Dihydrobenzofuran-7-yl)piperazin-1-yl)butoxy]pyrazolo[1,5-a]pyridine

Synthesis works according to the preparation of E15 when using 5-(4-bromobutoxy)pyrazolo[1,5-a]pyridine (C6a) and 1-(2,3-dihydrobenzofuran-7-yl)piperazine (C7i).

### Example E84:

### 5-[4-(4-(Chroman-8-yl)piperazin-1-yl)butoxy]pyrazolo[1,5-a]pyridine

Synthesis works according to the preparation of E15 when using 5-(4-bromobutoxy)pyrazolo[1,5-a]pyridine (C6a) and 1-(chroman-8-yl)piperazine (C7j).

### Example E85:

### 5-[4-(4-(2,3-Dichlorophenyl)-1,4-diazepan-1-yl)butoxy]pyrazolo[1,5-a]pyridine

Synthesis works according to the preparation of E15 when using 5-(4-bromobutoxy)pyrazolo[1,5-a]pyridine (C6a) and 1-(2,3-dichlorophenyl)-1,4-diazepane (C7k).

### Example E86:

### 5-[4-(4-(2-Methoxyphenyl)-1,4-diazepan-1-yl)butoxy]pyrazolo[1,5-a]pyridine

Synthesis works according to the preparation of E15 when using 5-(4-bromobutoxy)pyrazolo[1,5-a]pyridine (C6a) and 1-(2-methoxyphenyl)-1,4-diazepane (C7I).

### Example E87:

### 5-[4-(4-(2-Chlorophenyl)-1,4-diazepan-1-yl)butoxy]pyrazolo[1,5-a]pyridine

Synthesis works according to the preparation of E15 when using 5-(4-bromobutoxy)pyrazolo[1,5-a]pyridine (C6a) and 1-(2-chlorophenyl)-1,4-diazepane (C7m).

### Example E88:

*5-[4-(4-(2,3-Dihydrobenzofuran-7-yl)-1,4-diazepan-1-yl)butoxy]pyrazolo[1,5-a]pyridine 5-[4-(4-(Chroman-8-yl)-1,4-diazepan-1-yl)butoxy]pyrazolo[1,5-a]pyridine*

Synthesis works according to the preparation of E15 when using 5-(4-bromobutoxy)pyrazolo[1,5-a]pyridine (C6a) and 1-(2,3-dihydrobenzofuran-7-yl)-1,4-diazepane (C7n).

### Example E89:

### 5-[4-(4-(Chroman-8-yl)-1,4-diazepan-1-yl)butoxy]pyrazolo[1,5-a]pyridine

Synthesis works according to the preparation of E15 when using 5-(4-bromobutoxy)pyrazolo[1,5-a]pyridine (C6a) and 1-(chroman-8-yl)-1,4-diazepane (C7o).

### Example E99:

### 7-[4-[4-(2,3-Dichlorophenyl)piperazin-1-yl]butoxy]indolizine

Synthesis works according to the preparation of E15 when using 7-(4-bromobutoxy)indolizine (C6j) and 1-(2,3-dichlorophenyl)piperazine (C7a).

### Example E100:

### 7-[4-[4-(2-Methoxyphenyl)piperazin-1-yl]butoxy]indolizine

Synthesis works according to the preparation of E15 when using 7-(4-bromobutoxy)indolizine (C6j) and 1-(2-methoxyphenyl)piperazine (C7b).

### Example E101:

### 7-[4-[4-(2-Chlorophenyl)piperazin-1-yl]butoxy]indolizine

Synthesis works according to the preparation of E15 when using 7-(4-bromobutoxy)indolizine (C6j) and 1-(2-chlorophenyl)piperazine (C7c).

### Example E102:

### 6-[4-[4-(2,3-Dichlorophenyl)piperazin-1-yl]butoxy]imidazo[1,2-a]pyridine

Synthesis works according to the preparation of E15 when using 6-(4-bromobutoxy)imidazo[1,2-a]pyridine (C6k) and 1-(2,3-dichlorophenyl)piperazine (C7a).

### Example E103:

### 6-[4-[4-(2-Methoxyphenyl)piperazin-1-yl]butoxy]imidazo[1,2-a]pyridine

Synthesis works according to the preparation of E15 when using 6-(4-bromobutoxy)imidazo[1,2-a]pyridine (C6k) and 1-(2-methoxyphenyl)piperazine (C7b).

### Example E104:

### 6-[4-[4-(2-Chlorophenyl)piperazin-1-yl]butoxy]imidazo[1,2-a]pyridine

Synthesis works according to the preparation of E15 when using 6-(4-bromobutoxy)imidazo[1,2-a]pyridine (C6k) and 1-(2-chlorophenyl)piperazine (C7c).

### Example E105:

### 8-[4-[4-(2,3-Dichlorophenyl)piperazin-1-yl]butoxy]imidazo[1,2-a]pyridine

Synthesis works according to the preparation of E15 when using 8-(4-bromobutoxy)imidazo[1,2-a]pyridine (C6l) and 1-(2,3-dichlorophenyl)piperazine (C7a).

### Example E106:

### 8-[4-[4-(2-Methoxyphenyl)piperazin-1-yl]butoxy]imidazo[1,2-a]pyridine

Synthesis works according to the preparation of E15 when using 8-(4-bromobutoxy)imidazo[1,2-a]pyridine (C6l) and 1-(2-methoxyphenyl)piperazine (C7b).

### Example E107:

### 8-[4-[4-(2-Chlorophenyl)piperazin-1-yl]butoxy]imidazo[1,2-a]pyridine

Synthesis works according to the preparation of E15 when using 8-(4-bromobutoxy)imidazo[1,2-a]pyridine (C6l) and 1-(2-chlorophenyl)piperazine (C7c).

### 7f) Synthesis of example compounds according to formula XVI

### Example E68:

### 5-[4-[4-(3-Trifluoromethylphenyl)piperazin-1-yl]butoxy]pyrazolo[1,5-a]pyridin-3-carbaldehyde

Synthesis works according to the preparation of E21 when using 5-[4-[4-(3-trifluoromethylphenyl)piperazin-1-yl]butoxy]pyrazolo[1,5-a]pyridine (E64).

### Example E69:

### 5-[4-[4-(3-Nitrophenyl)piperazin-1-yl]butoxy]pyrazolo[1,5-a]pyridin-3-carbaldehyde

Synthesis works according to the preparation of E21 when using 5-[4-[4-(3-nitrophenyl)piperazin-1-yl]butoxy]pyrazolo[1,5-a]pyridine (E65).

### Example E70:

### N-[3-[4-[4-(3-Formylpyrazolo[1,5-a]pyridin-5-yloxy)butyl]piperazin-1-yl]phenyl]acetamide

Synthesis works according to the preparation of E21 when using N-[3-[4-[4-(pyrazolo(1,5-a]pyridin-5-yloxy)butyl]piperazin-1-yl]phenyl]acetamide (E67).

### Example E90:

### 5-[4-(4-(2,3-Dihydrobenzofuran-7-yl)piperazin-1-yl)butoxy]pyrazolo[1,5-a]pyridin-3-carbaldehyde

Synthesis works according to the preparation of E21 when using 5-[4-(4-(2,3-dihydrobenzofuran-7-yl)piperazin-1-yl)butoxy]pyrazolo[1,5-a]pyridine (E83).

### Example E91:

### 5-[4-(4-(Chroman-8-yl)piperazin-1-yl)butoxy]pyrazolo[1,5-a]pyridin-3-carbaldehyde

Synthesis works according to the preparation of E21 when using 5-[4-(4-(chroman-8-yl)piperazin-1-yl)butoxy]pyrazolo[1,5-a]pyridine (E84).

### Example E92:

### 5-[4-(4-(2,3-Dichlorophenyl)-1,4-diazepan-1-yl)butoxy]pyrazolo[1,5-a]pyridin-3-carbaldehyde

Synthesis works according to the preparation of E21 when using 5-[4-(4-(2,3-dichlorophenyl)-1,4-diazepan-1-yl)butoxy]pyrazolo[1,5-a]pyridine (E85).

### Example E93:

### 5-[4-(4-(2-Methoxyphenyl)-1,4-diazepan-1-yl)butoxy]pyrazolo[1,5-a]pyridin-3-carbaldehyde

Synthesis works according to the preparation of E21 when using 5-[4-(4-(2-methoxyphenyl)-1,4-diazepan-1-yl)butoxy]pyrazolo[1,5-a]pyridine (E86).

### Example E94:

### 5-[4-(4-(2-Chlorophenyl)-1,4-diazepan-1-yl)butoxy]pyrazolo[1,5-a]pyridin-3-carbaldehyde

Synthesis works according to the preparation of E21 when using 5-[4-(4-(2-chlorophenyl)-1,4-diazepan-1-yl)butoxy]pyrazolo[1,5-a]pyridine (E87).

### Example E95:

### 5-[4-(4-(2,3-Dihydrobenzofuran-7-yl)-1, 4-diazepan-1-yl)butoxy]pyrazolo[1,5-a]pyridin-3-carbaldehyde

Synthesis works according to the preparation of E21 when using 5-[4-(4-(2,3-dihydrobenzofuran-7-yl)-1,4-diazepan-1-yl)butoxy]pyrazolo[1,5-a]pyridine (E88).

### Example E96:

### 5-[4-(4-(Chroman-8-yl)-1,4-diazepan-1-yl)butoxy]pyrazolo[1,5-a]pyridin-3-carbaldehyde

Synthesis works according to the preparation of E21 when using 5-[4-(4-(chroman-8-yl)-1,4-diazepan-1-yl)butoxy]pyrazolo[1,5-a]pyridine (E89).

### 7g) Synthesis of example compounds according to formula XVII

The synthesis of compounds according to formula XVII starts from formyl derivatives of formula XVIa, which can be hydrogenated to get the Example compounds E71 to E73 (formula XVII, R1" = CH₂OH) and subsequently reduced to obtain the Example compounds E74 to E76 (formula XVII, R1" = CH₃):

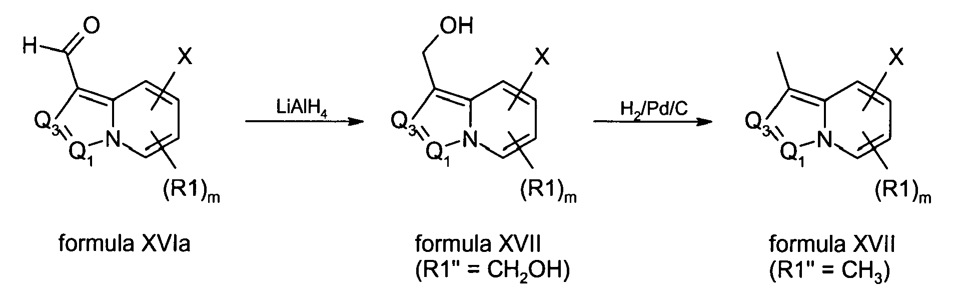

### Example E71:

### 5-[4-[4-(2,3-Dichlorophenyl)piperazin-1-yl]butoxy]-3-hydroxymethylpyrazolo[1,5-a]pyridine

Synthesis of the hydroxymethyl derivative E71 is achived by reduction of 5-[4-[4-(2,3-dichlorophenyl)piperazin-1-yl]butoxy]pyrazolo[1,5-a]pyridine-3-carbaldehyde (E21) with LiAIH₄ in refluxing THF for 1 hr.

### Example E72:

### 3-Hydroxymethyl-5-[4-[4-(2-methoxyphenyl)piperazin-1-yl]butoxy]methylpyrazolo[1,5-a]pyridine

Synthesis works according to the preparation of E71 when using 5-[4-[4-(2-methoxyphenyl)piperazin-1-yl]butoxy]pyrazolo[1,5-a]pyridine-3-carbaldehyde (E22).

### Example E73:

### 5-[4-[4-(2-Chlorophenyl)piperazin-1-yl]butoxy]-3-hydroxymethylpyrazolo[1,5-a]pyridine

Synthesis works according to the preparation of E71 when using 5-[4-[4-(2-chlorophenyl)piperazin-1-yl]butoxy]pyrazolo[1,5-a]pyridine-3-carbaldehyde (E23).

### Example E74:

### 5-[4-[4-(2,3-Dichlorophenyl)piperazin-1-yl]butoxy]-3-methylpyrazolo[1,5-a]pyridine

Synthesis of the methyl derivative E74 is done by reduction of 5-[4-[4-(2,3-dichlorophenyl)piperazin-1-yl]butoxy]-3-hydroxymethylpyrazolo[1,5-a]pyridine (E71) with hydrogene catalaysed by palladium on charcoal in acetic acid and under a pressure of 3 atm for 14 hrs.

### Example E75:

### 5-[4-[4-(2-Methoxyphenyl)piperazin-1-yl]butoxy]-3-methylpyrazolo[1,5-a]pyridine

Synthesis works according to the preparation of E74 when using 3-hydroxymethyl-5-[4-[4-(2-methoxyphenyl)piperazin-1-yl]butoxy]methylpyrazolo[1,5-a]pyridine (E72).

### Example E76:

### 5-[4-[4-(2-Chlorophenyl)piperazin-1-yl]butoxy]-3-methylpyrazolo[1,5-a]pyridine

Synthesis works according to the preparation of E71 when using 5-[4-[4-(2-chlorophenyl)piperazin-1-yl]butoxy]-3-hydroxymethylpyrazolo[1,5-a]pyridine (E73).

Further compounds according to formula XVII can be synthesised starting from cyano derivatives of formula XVII, which are hydrogenated to get the Example compounds E77 to E79 (formula XVII, R1" = CH₂NH₂). These aminomethyl derivatives can be acetylated reduced to obtain the Example compounds E80 to E82 (formula XVII, R1" = CH₂NHCOCH₃):

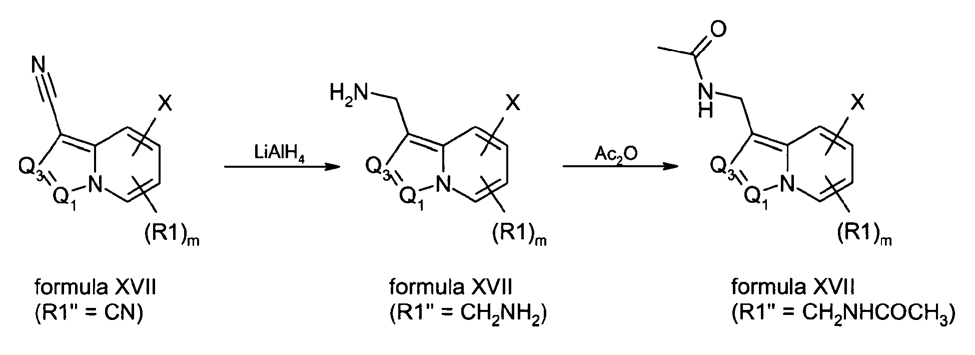

### Example E77:

### 3-Aminomethyl-5-[4-[4-(2,3-dichlorophenyl)piperazin-1-yl]butoxy]pyrazolo[1,5-a]pyridine

Synthesis of the aminomethyl derivative E77 is achived by reduction of 5-[4-[4-(2,3-dichlorophenyl)piperazin-1-yl]butoxy]pyrazolo[1,5-a]pyridine-3-carbonitrile (E36) with LiAlH₄ in refluxing THF for 16 hrs.

### Example E78:

### 3-Aminomethyl-5-[4-[4-(2-methoxyphenyl)piperazin-1-yl]butoxy]pyrazolo[1,5-a]pyridine

Synthesis works according to the preparation of E77 when using 5-[4-[4-(2-methoxyphenyl)piperazin-1-yl]butoxy]pyrazolo[1,5-a]pyridine-3-carbonitrile (E37).

### Example E79:

### 3-Aminomethyl-5-[4-[4-(2-chlorophenyl)piperazin-1-yl]butoxy]pyrazolo[1,5-a]pyridine

Synthesis works according to the preparation of E77 when using 5-[4-[4-(2-chlorophenyl)piperazin-1-yl]butoxy]pyrazolo[1,5-a]pyridine-3-carbonitrile (E38).

### Example E80:

### N-[5-[4-[4-(2,3-Dichlorophenyl)piperazin-1-yl]butoxy]pyrazolo[1,5-a]pyridin-3-ylmethyl]acetamide

Synthesis of the acetylaminomethyl derivative E80 is achived by acetylation of 3-aminomethyl-5-[4-[4-(2,3-dichlorophenyl)piperazin-1-yl]butoxy]pyrazolo[1,5-a]pyridine (E77) under standard conditions (acetanhydride, pyridine, room temperature, 2 hrs.).

### Example E81:

### N-[5-[4-[4-(2-Methoxyphenyl)piperazin-1-yl]butoxy]pyrazolo[1,5-a]pyridin-3-ylmethyl]acetamide

Synthesis works according to the preparation of E80 when using 3-aminomethyl-5-[4-[4-(2-methoxyphenyl)piperazin-1-yl]butoxy]pyrazolo[1,5-a]pyridine (E78).

### Example E82:

### N-[5-[4-[4-(2-Chlorophenyl)piperazin-1-yl]butoxy]pyrazolo[1,5-a]pyridin-3-ylmethyl]acetamide

Synthesis works according to the preparation of E80 when using 3-aminomethyl-5-[4-[4-(2-methoxyphenyl)piperazin-1-yl]butoxy]pyrazolo[1,5-a]pyridine (E78).

### (b) Biological Activity

Biological activity of the Example compounds has been determined in radioligand binding assys. These experiments have been done according to methods which are described in literature (Hübner, H.; Haubmann, C.; Utz, W.; Gmeiner, P. et al. J. Med. Chem. (2000), 43, 756-762). For the determination of binding affinity to the receptors of the dopamine D2 family membranes were established carrying the human D2long, D2short, D3 or D4.4 receptor subtype expressed in Chinese hamster ovary cell lines. In principle, the binding experiments started when incubating an appropriate amount of memebrane with the radioligand [³H]spiperone and the particular test compounds diluted in a wide range of different concentrations from subnanomolar up to micromolar. In the same way binding affinities of the test compounds to the dopamine D1 receptor were determined when using native membrane preparations from porcine striatum and the D1 selective radioligand [³H]SCH 23390.

The investigation of receptor binding to the serotonin and adrenergic receptors was done according to a published procedure (Heindl, C.; hübner, H.; Gmeiner, P. Tetrahedron: Asymmetry (2003), 14, 3141-3152). In detail, for the serotonin receptors membrane preparations from porcine cortical material was incubated with the 5-HT1A selective radioligands [³H]8-OH-DPAT and [³H]WAY 100635 and the 5-HT2 selective compound [³H]ketanserin. For the determination of alpha receptor binding cortical membranes and the radioligands [³H]prazosin (for α1) and [³H]RX821002 were established in identical manner.

The results of the binding experiments are summarized in Table 1 as mean values of 2 to 4 individual experiments each done in triplicate and expressed as Ki values in nM. Table 1 shows that with two exceptions all presently disclosed compounds have a Ki value at the D2s and D2l receptors of 300 nM or less. 32 of the 61 tested compounds even have a Ki value at the D2 receptors of 10 nm or less.

**Table 1: Results of binding assays with dopaminergic and serotonergic receptors Ki-values given: A ≤30nM; B = 30-300 nM; C = 301-3000 nM; D ≥3µM**

| compound | R1 | R2 | R3 | R4 | D21o | D2sh | D3 | D4.4 | 5-HT1a | 5-HT2 |
|---|---|---|---|---|---|---|---|---|---|---|
| aripiprazole | - | - | - | - | A | A | A | B | B | C |
| 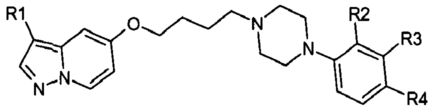 | | | | | | | | | | |
| E15E15 | H | Cl | Cl | H | B | B | A | B | B | C |
| E16 | H | OMe | H | H | A | A | A | A | A | C |
| E17 | H | Cl | H | H | A | A | A | B | B | C |
| E18 | H | H | H | OMe | D | C | B | C | B | C |
| E19 | H | H | H | Cl | B | B | B | B | B | C |
| E20 | H | H | H | OH | B | B | B | C | D | C |
| E21 | CHO | Cl | Cl | H | A | A | A | B | B | B |
| E22 | CHO | OMe | H | H | A | A | A | A | A | C |
| E23 | CHO | Cl | H | H | A | A | A | A | A | C |
| E24 | CHO | H | H | OMe | B | B | C | C | B | D |
| E25 | CHO | H | H | Cl | A | A | B | B | B | C |
| E26 | CHO | H | H | OH | B | A | B | B | B | C |
| E27 | MeCO | Cl | Cl | H | A | A | A | A | A | B |
| E28 | MeCO | OMe | H | H | A | A | A | A | A | C |
| E29 | MeCO | Cl | H | H | A | A | A | A | A | B |
| E30 | oxime s-trans | Cl | Cl | H | A | A | A | A | A | B |
| E31 | oxime s-trans | OMe | H | H | A | A | A | A | A | C |
| E32 | oxime s-trans | Cl | H | H | A | A | A | A | A | B |
| E33 | oxime s-cis | Cl | Cl | H | A | A | A | A | B | B |
| E34 | oxime s-cis | OMe | H | H | A | A | A | A | A | C |
| E35 | oxime s-cis | Cl | H | H | A | A | A | A | A | B |
| E36 | cyano | Cl | Cl | H | A | A | A | A | B | C |
| E37 | cyano | OMe | H | H | A | A | A | A | A | C |
| E38 | cyano | Cl | H | H | A | A | A | A | A | B |
| E39 | MeOCO | Cl | Cl | H | A | A | A | A | A | B |
| E40 | MeOCO | OMe | H | H | A | A | A | A | A | B |
| E41 | MeOCO | Cl | H | H | A | A | A | A | A | C |
| E45 | Cl | Cl | Cl | H | B | B | A | B | B | C |
| E46 | Cl | OMe | H | H | A | A | A | A | A | C |
| E47 | Cl | Cl | H | H | B | A | A | B | B | C |
| E42 | Br | Cl | Cl | H | B | B | A | C | C | D |
| E43 | Br | OMe | H | H | A | A | A | A | A | C |
| E48 | I | Cl | Cl | H | B | A | A | B | C | C |
| E49 | I | OMe | H | H | A | A | A | A | A | C |
| E50 | I | Cl | H | H | B | B | A | B | B | C |

| compound | R1 | R2 | R3 | R4 | D2lo | D2sh | D3 | D4.4 | 5-HT1 a | 5-HT2 |
|---|---|---|---|---|---|---|---|---|---|---|
| 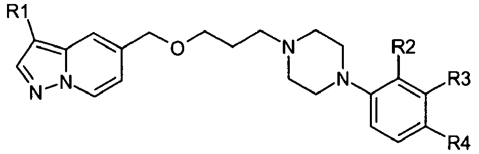 | | | | | | | | | | |
| E13 | H | Cl | Cl | H | A | A | A | B | B | B |
| E14 | CHO | Cl | Cl | H | A | A | A | B | A | B |
| 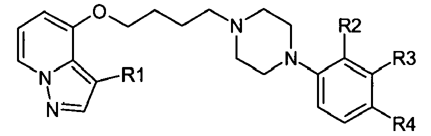 | | | | | | | | | | |
| E7 | H | Cl | Cl | H | A | A | A | A | B | C |
| E8 | H | OMe | H | H | A | A | A | A | A | C |
| E9 | H | Cl | H | H | A | A | A | A | A | C |
| E10 | CHO | Cl | Cl | H | A | A | A | B | B | B |
| E11 | CHO | OMe | H | H | A | A | B | A | A | C |
| E12 | CHO | Cl | H | H | A | A | B | A | A | C |
| 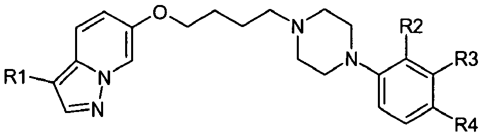 | | | | | | | | | | |
| E51 | H | Cl | Cl | H | A | A | A | A | B | B |
| E52 | H | OMe | H | H | A | A | A | A | A | C |
| E53 | H | Cl | H | H | A | A | A | A | A | C |
| E54 | CHO | Cl | Cl | H | A | A | A | A | A | B |
| E55 | CHO | OMe | H | H | A | A | A | A | A | C |
| E56 | CHO | Cl | H | H | A | A | A | A | A | C |
| 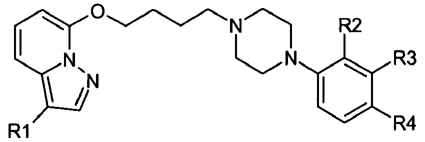 | | | | | | | | | | |
| E57 | H | Cl | Cl | H | B | A | A | B | B | B |
| E58 | H | OMe | H | H | B | B | B | A | A | C |
| E59 | H | Cl | H | H | B | B | A | A | B | B |
| E50 | CHO | Cl | Cl | H | B | A | A | B | B | B |
| E61 | CHO | OMe | H | H | C | B | C | C | B | D |
| E62 | CHO | Cl | H | H | B | A | B | B | A | B |

| compound | R1 | R2 | R3 | R4 | D2lo | D2sh | D3 | D4.4 | 5-HT1a | 5-HT2 |
|---|---|---|---|---|---|---|---|---|---|---|
| 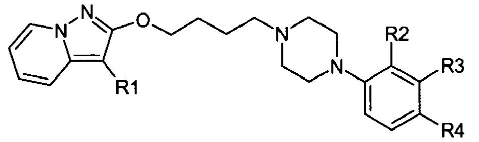 | | | | | | | | | | |
| E1 | H | Cl | Cl | H | A | A | A | B | A | B |
| E2 | H | OMe | H | H | A | A | A | A | A | C |
| E3 | H | Cl | H | H | A | A | A | B | A | C |
| E4 | CHO | Cl | Cl | H | A | A | A | A | A | B |
| E5 | CHO | OMe | H | H | A | A | A | A | A | C |
| E6 | CHO | Cl | H | H | A | B | A | B | A | C |
| 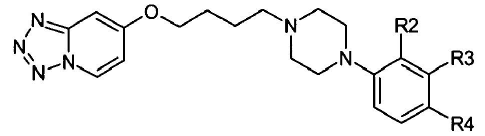 | | | | | | | | | | |
| E63 | H | Cl | Cl | H | A | A | A | A | B | B |
| E97 | H | OMe | H | H | A | A | A | A | A | D |
| E98 | H | Cl | H | H | A | A | A | A | A | C |

Moreover, most of the compounds of the present invention exhibit a remarkable affinity to at least one of the tested serotonin receptors. While aripiprazole shows a pronounced selectivity for the D2 receptors over the D4.4 receptor as well as over the 5-HT1 a (about 40 fold) and the 5-HT2A receptor (about 330 fold), many of the presently disclosed compounds are less selective compared to D4.4 and the serotonin receptors and tend to have a more "balanced" dopamine/serotonin and D2/D4.4 ratio, which may be associated with a reduced likelihood of extrapyramidal symptoms and an increased potency to alleviate negative symptoms of schizophrenia.

The investigation of functional activity was carried out by a mitogenesis assay indicating the stimulation of receptor by incorporation of [³H]thymidine into growing cells as described in literature (Hübner, H.; Kraxner, J.; Gmeiner, P. J. Med. Chem. (2000), 43, 4563-4569). Furthermore, a second method was established when measuring the binding of [³⁵S]GTPγS to membranes after stimulation of the appropriate receptor which is published in literature (Schlotter, K.; Boeckler, F.; Hübner, H.; Gmeiner, P. J. Med. Chem. (2005), 48, 3696-3699).

Table 2 shows the functional (intrinsic) activity of the compounds according to the present disclosure at the dopamine D2short receptor as a representative target molecule. In both, the mitogenesis and the GTPγS-incorporation assay the intrinsic activity is given based on % activity of quinpirol: A=Full antagonist (<15%); B=weak partial agonist (15-40%) C=strong partial agonist (40-75%) D=Full agonist (>75%).

**Table 2: Intrinsic activities of the resently disclaimed compounds at the dopamine D2short receptor determined by mitogenesis assay and binding of [³⁵S]GTPγS**

| compound | R1 | R2 | R3 | R4 | D2short: [³⁵S]GTPγS |
|---|---|---|---|---|---|
| aripiprazole | - | - | - | - | B |
| 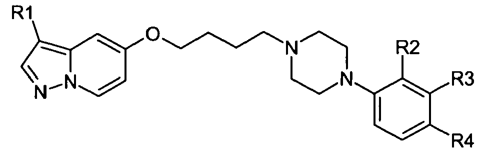 | | | | | |
| E15E15 | H | Cl | Cl | H | B |
| E16 | H | OMe | H | H | A |
| E17 | H | Cl | H | H | B |
| E18 | H | H | H | OMe | A |
| E19 | H | H | H | Cl | B |
| E20 | H | H | H | OH | B |
| E21 | CHO | Cl | Cl | H | B |
| E22 | CHO | OMe | H | H | B |
| E23 | CHO | Cl | H | H | B |
| E24 | CHO | H | H | OMe | A |
| E25 | CHO | H | H | Cl | B |
| E26 | CHO | H | H | OH | C |
| E27 | MeCO | Cl | Cl | H | A |
| E28 | MeCO | OMe | H | H | B |
| E29 | MeCO | Cl | H | H | A |
| E30 | oxime s-trans | Cl | Cl | H | A |
| E31 | oxime s-trans | OMe | H | H | B |
| E32 | oxime s-trans | Cl | H | H | B |
| E33 | oxime s-cis | Cl | Cl | H | A |
| E34 | oxime s-cis | OMe | H | H | A |
| E35 | oxime s-cis | Cl | H | H | A |
| E36 | cyano | Cl | Cl | H | A |
| E37 | cyano | OMe | H | H | A |
| E38 | cyano | Cl | H | H | B |
| E39 | MeOCO | Cl | Cl | H | A |
| E40 | MeOCO | OMe | H | H | B |
| E41 | MeOCO | Cl | H | H | B |
| E45 | Cl | Cl | Cl | H | B |
| E46 | Cl | OMe | H | H | A |
| E47 | Cl | Cl | H | H | A |
| E42 | Br | Cl | Cl | H | B |
| E43 | Br | OMe | H | H | B |
| E44 | Br | Cl | H | H | B |
| E48 | I | Cl | Cl | H | A |
| E49 | I | OMe | H | H | A |
| E50 | I | Cl | H | H | A |
| 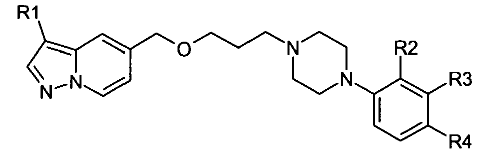 | | | | | |
| E13 | H | Cl | Cl | H | A |
| E14 | CHO | Cl | Cl | H | B |
| 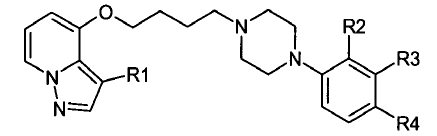 | | | | | |
| E7 | H | Cl | Cl | H | A |
| E8 | H | OMe | H | H | A |
| E9 | H | Cl | H | H | A |
| E10 | CHO | Cl | Cl | H | B |
| E11 | CHO | OMe | H | H | A |
| E12 | CHO | Cl | H | H | B |
| 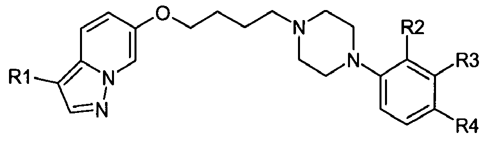 | | | | | |
| E51 | H | Cl | Cl | H | A |
| E52 | H | OMe | H | H | B |
| E53 | H | Cl | H | H | A |
| E54 | CHO | Cl | Cl | H | A |
| E55 | CHO | OMe | H | H | A |
| E56 | CHO | Cl | H | H | B |
| 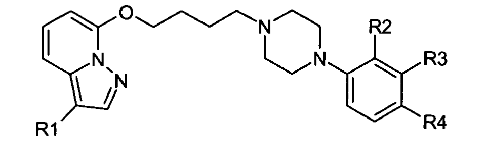 | | | | | |
| E57 | H | Cl | Cl | H | B |
| E58 | H | OMe | H | H | B |
| E59 | H | Cl | H | H | B |
| E60 | CHO | Cl | Cl | H | A |
| E61 | CHO | OMe | H | H | A |
| E62 | CHO | Cl | H | H | B |
| 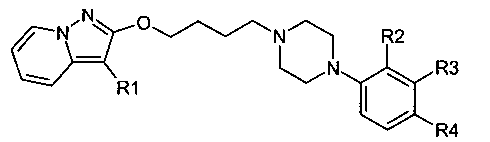 | | | | | |
| E1 | H | Cl | Cl | H | A |
| E2 | H | OMe | H | H | A |
| E3 | H | Cl | H | H | A |
| E4 | CHO | Cl | Cl | H | B |
| E5 | CHO | OMe | H | H | B |
| E6 | CHO | Cl | H | H | B |
| 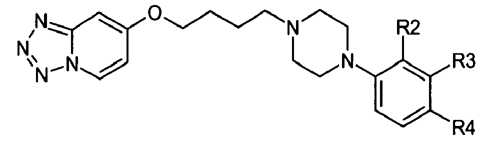 | | | | | |
| E63 | H | Cl | Cl | H | n.d |
| E97 | H | OMe | H | H | n.d |
| E98 | H | Cl | H | H | n.d |

| | | | | | |
|---|---|---|---|---|---|
| n.d = values were not determined | | | | | |

## Claims

**1.** Compounds of the general formula I, 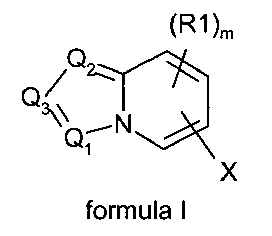 wherein:
Q1 and Q2 are independently of each other N, CH or C-R1;
Q3 is CH or C-R1 if at least one of Q1 and Q2 is different from nitrogen, and Q3 is selected from CH, C-R1 and N if Q1 and Q2 are both nitrogen;
m is 0, 1, 2 or 3;
any R1 is bonded to a C-atom of the heterocycle of formula I and is independently of each other selected from the group comprising of hydroxy, formyl, oxime, cyano, nitro, amino, NR7R8, carboxy, carbamoyl, alkyl, cycloalkyl, alkyloxy, alkylthio, alkenyl, alkynyl, phenyl, heteroaryl, phenoxy, halogen, trifluoromethyl, alkylcarbonyl, alkylaminocarbonyl, dialkylaminocarbonyl, phenylcarbonyl, phenylalkyl, phenylalkyloxy, phenylalkylcarbonyl, phenylalkyloxycarbonyl, alkyloxycarbonyl, alkylsulfonyl, phenylsulfonyl,sulfamoyl, alkylaminosulfonyl, dialkylaminosulfonyl, phenylsulfonylamino and alkylsulfonylamino; wherein each alkyl, alkenyl or alkynyl may be unsubstituted or substituted with one or more residues selected from among hydroxyl, alkyloxy, halogen, and NR7R8; and wherein each heteroaryl is a monocyclic ring and wherein each phenyl or heteroaryl can be unsubstituted or substituted with one or more residues selected from among hydroxyl, alkyloxy, halogen, alkyl, cycloalkyl, carboxy, NR7R8, cyano, trifluoromethyl and nitro; and
wherein each cycloalkyl can be unsubstituted or substituted with hydroxyl, alkyloxy, halogen, alkyl, phenyl, amino and NR7R8.
X is a group having the formula 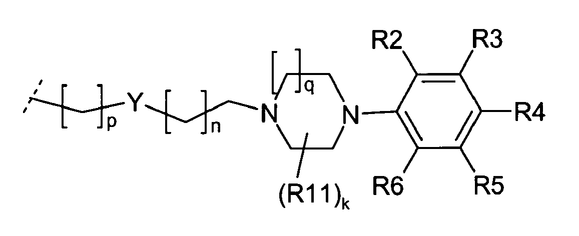
wherein:
Y is chosen from among S and O;
k is 0, 1 or 2;
n is 1-5;
p is 0, 1 or 2;
q is 1 or 2;
R2, R3, R4, R5 and R6 are in each case and independently of each other selected from the group comprising hydrogen, hydroxy, formyl, oxime, cyano, nitro, amino, NR7R8, carboxy, carbamoyl, alkyl, alkyloxy, alkylthio, alkenyl, alkynyl, cycloalkyl, phenyl, heteroaryl, phenoxy, halogen, trifluoromethyl, alkylcarbonyl, alkylaminocarbonyl, dialkylaminocarbonyl, phenylcarbonyl, phenylalkyl, phenylalkyloxy, phenylalkylcarbonyl, phenylalkyloxycarbonyl, alkyloxycarbonyl, alkylsulfonyl, phenylsulfonyl, sulfamoyl, alkylaminosulfonyl, dialkylaminosulfonyl, phenylsulfonylamino and alkylsulfonylamino; wherein each alkyl, alkenyl or alkynyl may be unsubstituted or substituted with one or more residues selected from among hydroxyl, alkyloxy, halogen, and NR7R8; and wherein each heteroaryl is a monocyclic ring and wherein each phenyl or heteroaryl can be unsubstituted or substituted with one or more residues selected from among hydroxyl, alkyloxy, halogen, alkyl, cycloalkyl, carboxy, NR7R8, cyano, trifluormethyl and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, alkyl, cycloalkyl, phenyl, heteroaryl, phenylalkyl, alkylsulfonyl, phenylsulfonyl, heteroarylsulfonyl, alkylcarbonyl, alkoxycarbonyl, aminocarbonyl, alkylaminocarbonyl, phenylcarbonyl, and heteroarylcarbonyl; wherein each alkyl may be unsubstituted or substituted with one or more residues selected from among hydroxyl, alkyloxy, phenyl, fluoro, carboxy, and NR9R10; and wherein R7 and R8 may form a 5- to 7- membered cycle; and wherein each heterocycle is a monocyclic ring and wherein phenyl or heteroaryl can be unsubstituted or substituted with one or more residues selected from among hydroxyl, alkyloxy, halogen, alkyl, carboxy, NR9R10, cyano, trifluormethyl and nitro;
R9 and R10 are independently selected from among hydrogen and alkyl;
R11 is hydroxyl, (C1-C3)alkyl, hydroxy-(C1-C3)alkyl, halogen-(C1-C3)alkyl or oxo;
provided that the heterocycle of formula I carries precisely one group X;
in the form of the free base, their physiologically acceptable salts, possible conformational isomers, enatiomers, and diastereomers.

**2.** A compound according to claim 1, selected from the group of formulas IIa-IId 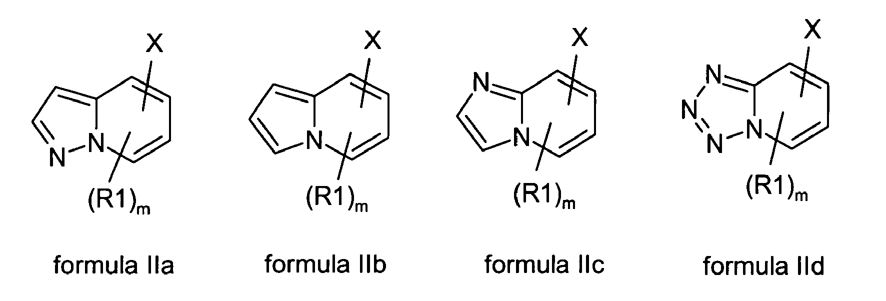 wherein:
m is 0, 1, 2 or 3;
any R1 is bonded to a C-atom of a heterocycle of formulas IIa-IId and is independently of each other selected from the group comprising of hydroxy, formyl, oxime, cyano, nitro, amino, NR7R8, carboxy, carbamoyl, (C1-C10)alkyl, (C3-C7) cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C7) cycloalkyl, carboxy, NR7R8, cyano, trifluoromethyl, and nitro;
X is a group having the formula 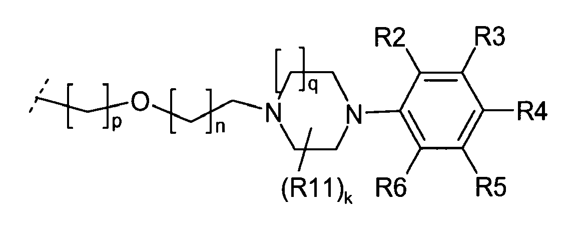
wherein:
k is 0, 1 or 2;
n is 1-5;
p is 0, 1 or 2;
q is 1 or 2;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C3-C7)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C7)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C7)cycloalkyl; phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
provided that each heterocycle of formulas IIa-IId carries precisely one group X;
in the form of the free base, their physiologically acceptable salts and possible enatiomers and diastereomers.

**3.** A compound according to anyone of the preceding claims, wherein q is 1.

**4.** A compound according to anyone of the preceding claims, wherein k is 0.

**5.** A compound according to anyone of the preceding claims, wherein R4 is hydrogen.

**6.** A compound according to anyone of the preceding claims, wherein
m is 0, 1 or 2;
p is 0 or 1;
n is 2, 3 or 4;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
wherein R7 and R8 are selected from among hydrogen, (C1-C3)alkyl, (C1-C3)alkylcarbonyl and phenyl(C1-C3)alkyl.

**7.** A compound according to anyone of the preceding claims, wherein
m is 0, 1 or 2;
p is 0 or 1;
n is 2, 3 or 4;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8;
R5 and R6 are independently selected from hydrogen, or fluoro;
wherein R7 and R8 are independently selected from hydrogen, (C1-C3)alkylcarbonyl, (C1-C3)alkyl, and phenyl(C1-C3)alkyl.

**8.** A compound according to anyone of the preceding claims, having the formula IIa 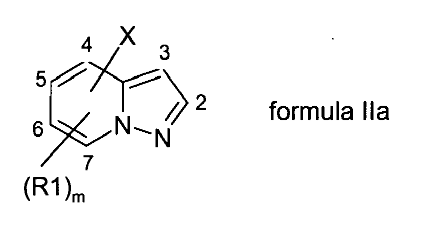 wherein:
R1, X, and m have the meaning as described in the previous claims.

**9.** A compound according to claim 7, wherein X is bonded to the 2-, 4-, 5- or 6-position of the heteroaromatic ring system of formula IIa.

**10.** A compound according to anyone of claims 7-8, wherein m is 1, and R1 is in 3-position of the heteroaromatic ring system of the general formula IIa.

**11.** A compound according to anyone of the preceding claims, wherein X is 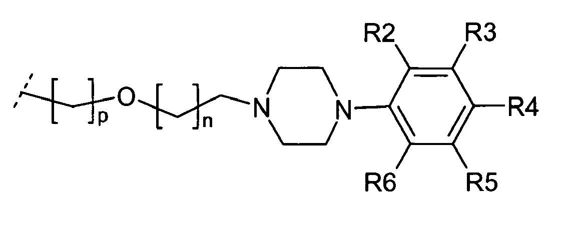 and wherein p is 0 or 1, and the sum of p and n is 3;
R2-R6 have the meaning as defined in any of the preceding claims.

**12.** A compound according to claim 1, and having the formula III 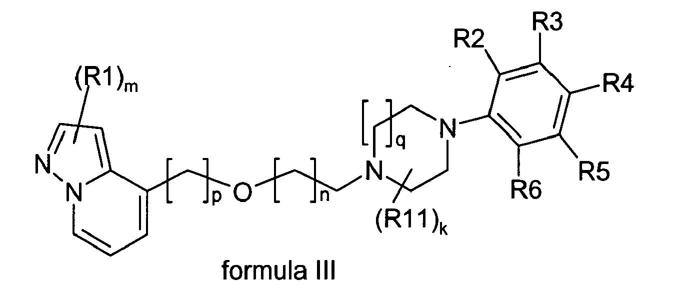 wherein:
k is 0, 1, or 2;
m is 0, 1, 2 or 3;
n is 1-5;
p is 0, 1 or 2;
q is 1 or 2, and is preferably 1;
any R1 is bonded to a C-atom of the heterocycle of formula III, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C6)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
provided that the heterocycle of formula III carries precisely one group X;
in the form of the free base, their physiologically acceptable salts and possible enatiomers and diastereomers.

**13.** A compound according to claim 12, wherein
k is 0 or 1;
m is 0, 1 or 2;
p is 0 or 1;
n is 2, 3 or 4;
q is 1;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8;
R5 and R6 are independently selected from hydrogen, or fluoro;
wherein R7 and R8 are independently selected from hydrogen, (C1-C3)alkylcarbonyl, (C1-C3)alkyl, and phenyl(C1-C3)alkyl.

**14.** A compound according to claims 12 or 13, wherein
k is 0;
m is 0 or 1;
n is 3;
p is 0;
qis1;
R1 is hydrogen, or formyl,
R2 and R3 are independently selected from hydrogen, methoxy, chloro, amino, and acetylamino;
R4, R5 and R6 are all hydrogen.

**15.** A compound according to claim 1, and having the formula IV 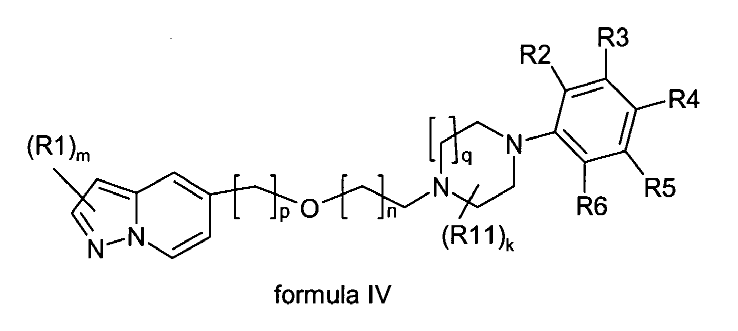 wherein:
k is 0,1 or 2;
m is 0, 1, 2 or 3;
n is 1-5;
p is 0, 1 or 2;
q is 1 or 2, and is preferably 1;
any R1 is bonded to a C-atom of the heterocycle of formula IV, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C7)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
provided that the heterocycle of formula IV carries precisely one group X;
in the form of the free base, their physiologically acceptable salts and possible enatiomers and diastereomers.

**16.** A compound according to claim 15, wherein
k is 0 or 1;
m is 0, 1 or 2;
p is 0 or 1;
n is 2, 3 or 4;
q is 1;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8;
R5 and R6 are independently selected from hydrogen, or fluoro;
wherein R7 and R8 are independently selected from hydrogen, (C1-C3)alkylcarbonyl, (C1-C3)alkyl, and phenyl(C1-C3)alkyl.

**17.** A compound according to claims 15 or 16, wherein
k is 0;
m is 0 or 1;
n is 3;
p is 0;
q is 1;
R1 is hydrogen, or formyl, cyano, oxime, (C1-C3)alkyloxycarbonyl, chloro, bromo, iodo, hydroxymethyl, nitro, NR7R8;
R2 and R3 are independently selected from hydrogen, methoxy, chloro, amino, and acetylamino;
R4 is selected from hydrogen, fluoro and chloro
R5 and R6 are both hydrogen.

**18.** A compound according to claim 1, and having the formula V 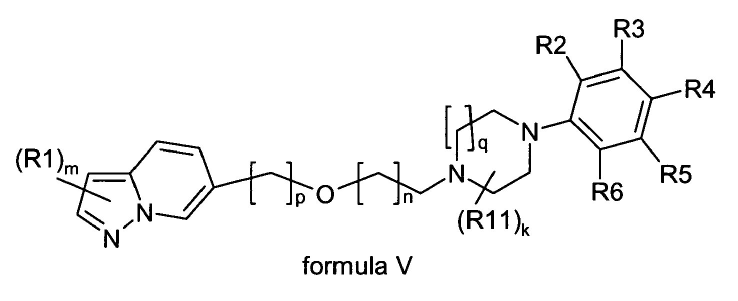 wherein
k is 0, 1 or 2;
m is 0, 1, 2 or 3;
n is 1-5;
p is 0, 1 or 2;
q is 1 or 2, and is preferably 0;
any R1 is bonded to a C-atom of the heterocycle of formulas V, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C1-C10)alkyloxy, (C1-C10)alkylthio, (C2-C10)alkenyl, (C2-C10)alkynyl, phenyl, (C3-C6)cycloalkyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C6)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
provided that the heterocycle of formula V carries precisely one group X;
in the form of the free base, their physiologically acceptable salts and possible enatiomers and diastereomers.

**19.** A compound according to claim 18, wherein
kis0or1;
m is 0, 1 or 2;
pis0or1;
n is 2, 3 or 4;
q is 1;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8;
R5 and R6 are independently selected from hydrogen, or fluoro;
wherein R7 and R8 are independently selected from hydrogen, (C1-C3)alkylcarbonyl, (C1-C3)alkyl, and phenyl(C1-C3)alkyl.

**20.** A compound according to claims 18 or 19, wherein
K is 0;
m is 0 or 1
n is 3;
p is 0;
q is 1;
R1 is hydrogen, or formyl,
R2 and R3 are independently selected from hydrogen, methoxy, chloro, amino, and acetylamino;
R4, R5 and R6 are all hydrogen.

**21.** A compound according to claim 1, and having the formula VI, VII or VIII 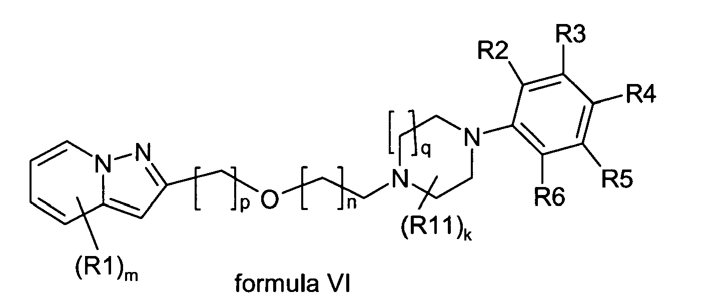 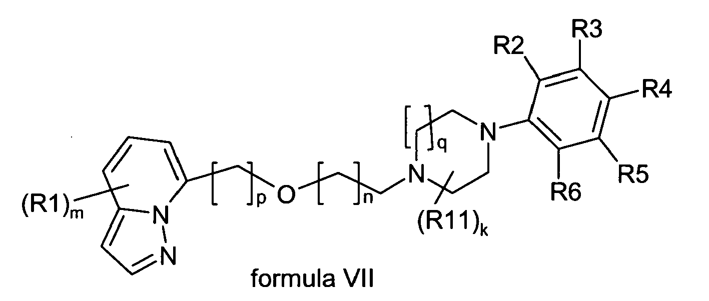 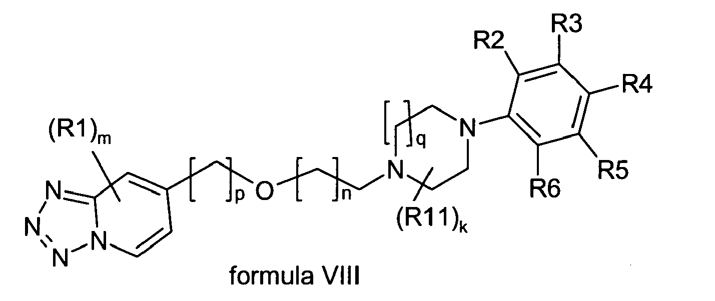 wherein:
k is 0,1 or 2;
m is 0, 1, 2 or 3;
n is 1-5;
p is 0, 1 or 2;
q is 1 or 2, and is preferably 1;
any R1 is bonded to a C-atom of the heterocycle of formula VI, VII or VIII, and is independently of each other selected from the group comprising of hydroxy, formyl, oxime, cyano, nitro, amino, NR7R8, carboxy, carbamoyl, (C1-C10)alkyl, (C3-C6)cycloalkyl, (C1-C10)alkyloxy, (C1-C10)alkylthio, (C2-C10)alkenyl, (C2-C10)alkynyl, phenyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C10)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherin each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro;
R2, R3, R4, R5 and R6 are in each case and independently of each other selected from the group comprising hydrogen, hydroxy, formyl, oxime, cyano, nitro, amino, NR7R8, carboxy, carbamoyl, (C1-C10)alkyl, (C1-C10)alkyloxy, (C1-C10)alkylthio, (C2-C10)alkenyl, (C2-C10)alkynyl, phenyl, (C3-C6)cycloalkyl, heteroaryl, phenoxy, halogen, trifluoromethyl, (C1-C10)alkylcarbonyl, (C1-C10)alkylaminocarbonyl, di(C1-C10)alkylaminocarbonyl, phenylcarbonyl, phenyl(C1-C10)alkyl, phenyl(C1-C10)alkyloxy, phenyl(C1-C10)alkylcarbonyl, phenyl(C1-C10)alkyloxycarbonyl, (C1-C10)alkyloxycarbonyl, (C1-C19)alkylsulfonyl, phenylsulfonyl, sulfamoyl, (C1-C10)alkylaminosulfonyl, di(C1-C10)alkylaminosulfonyl, phenylsulfonylamino, and (C1-C10)alkylsulfonylamino; wherein each alkyl, alkenyl or alkynyl may be unsubstituted or substituted with one or more residues selected from among hydroxyl, (C1-C6)alkyloxy, halogen, and NR7R8; and wherein each heteroaryl is a monocyclic ring and wherein each phenyl or heteroaryl can be unsubstituted or substituted with one or more residues selected from among hydroxyl, (C1-C6)alkyloxy, halogen, (C1-C6)alkyl, (C3-C6)cycloalkyl, carboxy, NR7R8, cyano, trifluoromethyl, and nitro; and wherein two vicinal residues R2, R3, R4, R5 and R6 together with the C-atoms of the phenyl ring to which they are bonded, can form an oxygen and/or nitrogen-containing 5-, 6- or 7-membered ring;
R7 and R8 are independently selected from hydrogen, (C1-C6)alkyl, (C3-C6)cycloalkyl, phenyl, heteroaryl, phenyl(C1-C6)alkyl, (C1-C6)alkylsulfonyl, phenylsulfonyl, heteroarylsulfonyl, (C1-C6)alkylcarbonyl, (C1-C6)alkyloxycarbonyl, aminocarbonyl, (C1-C6)alkylaminocarbonyl, phenylcarbonyl, and heteroarylcarbonyl; wherein each alkyl may be unsubstituted or substituted with one or more residues selected from among hydroxyl, (C1-C6)alkyloxy, phenyl, fluoro, carboxy, and NR9R10; and wherein R7 and R8 may form a 5- to 7-membered cycle; and wherein each heterocycle is a monocyclic ring and wherein each phenyl or heteroaryl be unsubstituted or substituted with one or more residues selected from among hydroxyl, (C1-C6)alkyloxy, halogen, (C1-C6)alkyl, carboxy, NR9R10, cyano, trifluoromethyl, and nitro;
R9 and R10 are independently selected from among hydrogen, and alkyl;
R11 is methyl, ethyl, hydroxyl, hydroxymethyl, or oxo;
provided that the heterocycle of any compound of formula VI, VII or VIII carries precisely one group X;
in the form of the free base, their physiologically acceptable salts and possible enatiomers and diastereomers.

**22.** A compound according to claim 21, wherein
k is 0 or 1;
m is 0, 1 or 2;
p is 0 or 1;
n is 2, 3 or 4;
q is 1;
each R1 is independently selected from hydrogen, formyl, cyano, oxime, (C1-C3)alkylcarbonyl, (C1-C3)alkyloxycarbonyl, chloro, bromo, iodo, hydroxymethyl, nitro, NR7R8;
R2 and R3 are independently selected from among hydrogen, fluoro, chloro, bromo, trifluoromethyl, (C1-C3)alkyloxy, (C1-C3)alkyl, -NR7R8, or R2 and R3 form together with the phenylring to which they are attached a dihydrobenzofurane, chromane, tetrahydrobenzoxepine or benzodioxole group;
R4 is selected from hydrogen, (C1-C3)alkyloxy, hydroxy, chloro, fluoro, trifluoromethyl, or NR7R8;
R5 and R6 are independently selected from hydrogen, or fluoro;
wherein R7 and R8 are independently selected from hydrogen, (C1-C3)alkylcarbonyl, (C1-C3)alkyl, and phenyl(C1-C3)alkyl.

**23.** A compound according to claims 21 or 22, wherein
k is 0;
m is 0 or 1;
n is 3;
p is 0;
q is 1;
R1 is hydrogen, or formyl,
R2 and R3 are independently selected from hydrogen, methoxy, chloro, amino, and acetylamino;
R4, R5 and R6 are all hydrogen.

**24.** A compound selected from
- 2-[4-[4-(2,3-Dichlorophenyl)piperazin-1-yl]butoxy]pyrazolo[1,5-a]pyridine
- 2-[4-[4-(2-Methoxyphenyl)piperazin-1-yl]butoxy]pyrazolo[1,5-a]pyridine
- 2-[4-[4-(2-Chlorophenyl)piperazin-1-yl]butoxy]pyrazolo[1,5-a]pyridine
- 2-[4-[4-(2,3-Dichlorophenyl)piperazin-1-yl]butoxy]pyrazolo[1,5-a]pyridin-3-carbaldehyde
- 2-[4-[4-(2-Methoxyphenyl)piperazin-1-yl]butoxy]pyrazolo[1,5-a]pyridin-3-carbaldehyde
- 2-[4-[4-(2-Chlorophenyl)piperazin-1-yl]butoxy]pyrazolo[1,5-a]pyridin-3-carbaldehyde
- 4-[4-[4-(2,3-Dichlorophenyl)piperazin-1-yl]butoxy]pyrazolo[1,5-a]pyridine
- 4-[4-[4-(2-Methoxyphenyl)piperazin-1-yl]butoxy]pyrazolo[1,5-a]pyridine
- 4-[4-[4-(2-Chlorophenyl)piperazin-1-yl]butoxy]pyrazolo[1,5-a]pyridine
- 4-[4-[4-(2,3-Dichlorophenyl)piperazin-1-yl]butoxy]pyrazolo[1,5-a]pyridin-3-carbaldehyde
- 4-[4-[4-(2-Methoxyphenyl)piperazin-1-yl]butoxy]pyrazolo[1,5-a]pyridin-3-carbaldehyde
- 4-[4-[4-(2-Chlorophenyl)piperazin-1-yl]butoxy]pyrazolo[1,5-a]pyridin-3-carbaldehyde
- 5-[3-[4-(2,3-Dichlorophenyl)piperazin-1-yl]propoxymethyl]pyrazolo[1,5-a]pyridine
- 5-[3-[4-(2,3-Dichlorophenyl)piperazin-1-yl]propoxymethyl]pyrazolo[1,5-a]pyridin-3-carbaldehyde
- 5-[4-[4-(2,3-Dichlorophenyl)piperazin-1-yl]butoxy]pyrazolo[1,5-a]pyridine
- 5-[4-[4-(2-Methoxyphenyl)piperazin-1-yl]butoxy]pyrazolo[1,5-a]pyridine
- 5-[4-[4-(2-Chlorophenyl)piperazin-1-yl]butoxy]pyrazolo[1,5-a]pyridine
- 5-[4-[4-(4-Methoxyphenyl)piperazin-1-yl]butoxy]pyrazolo[1,5-a]pyridine
- 5-[4-[4-(4-Chlorophenyl)piperazin-1-yl]butoxy]pyrazolo[1,5-a]pyridine
- 5-[4-[4-(4-Hydroxyphenyl)piperazin-1-yl]butoxy]pyrazolo[1,5-a]pyridine
- 5-[4-[4-(2,3-Dichlorophenyl)piperazin-1-yl]butoxy]pyrazolo[1,5-a]pyridin-3-carbaldeyhde
- 5-[4-[4-(2-Methoxyphenyl)piperazin-1-yl]butoxy]pyrazolo[1,5-a]pyridin-3-carbaldeyhde
- 5-[4-[4-(2-Chlorophenyl)piperazin-1-yl]butoxy]pyrazolo[1,5-a]pyridin-3-carbaldeyhde
- 5-[4-[4-(4-Methoxyphenyl)piperazin-1-yl]butoxy]pyrazolo[1,5-a]pyridin-3-carbaldeyhde
- 5-[4-[4-(4-Chlorophenyl)piperazin-1-yl]butoxy]pyrazolo[1,5-a]pyridin-3-carbaldeyhde
- 5-[4-[4-(4-Hydroxyphenyl)piperazin-1-yl]butoxy]pyrazolo[1,5-a]pyridin-3-carbaldeyhde
- 1-[5-[4-[4-(2,3-Dichlorophenyl)piperazin-1-yl]butoxy]pyrazolo[1,5-a]pyridin-3-yl]ethanone
- 1-[5-[4-[4-(2-Methoxyphenyl)piperazin-1-yl]butoxy]pyrazolo[1,5-a]pyridin-3-yl]ethanone
- 1-[5-[4-[4-(2-Chlorophenyl)piperazin-1-yl]butoxy]pyrazolo[1,5-a]pyridin-3-yl]ethanone
- *(s-trans)*-5-[4-[4-(2,3-Dichlorophenyl)piperazin-1-yl]butoxy]pyrazolo[1,5-a]pyridin-3-carbaldehyde oxime
- *(s-trans)*-5-[4-[4-(2-Methoxyphenyl)piperazin-1-yl]butoxy]pyrazolo[1,5-a]pyridin-3-carbaldehyde oxime
- *(s-trans)*-5-[4-[4-(2-Chlorophenyl)piperazin-1-yl]butoxy]pyrazolo[1,5-a]pyridin-3-carbaldehyde oxime
- *(s-cis)*-5-[4-[4-(2,3-Dichlorophenyl)piperazin-1-yl]butoxy]pyrazolo[1,5-a]pyridin-3-carbaldehyde oxime
- *(s-cis)*-5-[4-[4-(2-Methoxyphenyl)piperazin-1-yl]butoxy]pyrazolo[1,5-a]pyridin-3-carbaldehyde oxime
- *(s-cis)*-5-[4-[4-(2-Chlorophenyl)piperazin-1-yl]butoxy]pyrazolo[1,5-a]pyridin-3-carbaldehyde oxime
- 5-[4-[4-(2,3-Dichlorophenyl)piperazin-1-yl]butoxy]pyrazolo[1,5-a]pyridin-3-carbonitrile
- 5-[4-[4-(2-Methoxyphenyl)piperazin-1-yl]butoxy]pyrazolo[1,5-a]pyridin-3-carbonitrile
- 5-[4-[4-(2-Chlorophenyl)piperazin-1-yl]butoxy]pyrazolo[1,5-a]pyridin-3-carbonitrile
- 5-[4-[4-(2,3-Dichlorophenyl)piperazin-1-yl]butoxy]pyrazolo[1,5-a]pyridin-3-carboxylic acid ethyl ester
- 5-[4-[4-(2-Methoxyphenyl)piperazin-1-yl]butoxy]pyrazolo[1,5-a]pyridin-3-carboxylic acid ethyl ester
- 5-[4-[4-(2-Chlorophenyl)piperazin-1-yl]butoxy]pyrazolo[1,5-a]pyridin-3-carboxylic acid ethyl ester
- 3-Bromo-5-[4-[4-(2,3-dichlorophenyl)piperazin-1-yl]butoxy]pyrazolo[1,5-a]pyridine
- 3-Bromo-5-[4-[4-(2-methoxyphenyl)piperazin-1-yl]butoxy]pyrazolo[1,5-a]pyridine
- 3-Bromo-5-[4-[4-(2-chlorophenyl)piperazin-1-yl]butoxy]pyrazolo[1,5-a]pyridine
- 3-Chloro-5-[4-[4-(2,3-dichlorophenyl)piperazin-1-yl]butoxy]pyrazolo[1,5-a]pyridine
- 3-Chloro-5-[4-[4-(2-methoxyphenyl)piperazin-1-yl]butoxy]pyrazolo[1,5-a]pyridine
- 3-Chloro-5-[4-[4-(2-chlorophenyl)piperazin-1-yl]butoxy]pyrazolo[1,5-a]pyridine
- 5-[4-[4-(2,3-Dichlorophenyl)piperazin-1-yl]butoxy]-3-iodopyrazolo[1,5-a]pyridine
- 3-lodo-5-[4-[4-(2-methoxyphenyl)piperazin-1 -yl]butoxy]pyrazolo[1,5-a]pyridine
- 5-[4-[4-(2-Chlorophenyl)piperazin-1-yl]butoxy]-3-iodopyrazolo[1,5-a]pyridine
- 6-[4-[4-(2,3-Dichlorophenyl)piperazin-1-yl]butoxy]pyrazolo[1,5-a]pyridine
- 6-[4-[4-(2-Methoxyphenyl)piperazin-1-yl]butoxy]pyrazolo[1,5-a]pyridine
- 6-[4-[4-(2-Chlorophenyl)piperazin-1-yl]butoxy]pyrazolo[1,5-a]pyridine
- 6-[4-[4-(2,3-Dichlorophenyl)piperazin-1-yl]butoxy]pyrazolo[1,5-a]pyridin-3-carbaldehyde
- 6-[4-[4-(2-Methoxyphenyl)piperazin-1-yl]butoxy]pyrazolo[1,5-a]pyridin-3-carbaldehyde
- 6-[4-[4-(2-Chlorophenyl)piperazin-1-yl]butoxy]pyrazolo[1,5-a]pyridin-3-carbaldehyde
- 7-[4-[4-(2,3-Dichlorophenyl)piperazin-1-yl]butoxy]pyrazolo[1,5-a]pyridine
- 7-[4-[4-(2-Methoxyphenyl)piperazin-1-yl]butoxy]pyrazolo[1,5-a]pyridine
- 7-[4-[4-(2-Chlorophenyl)piperazin-1-yl]butoxy]pyrazolo[1,5-a]pyridine
- 7-[4-[4-(2,3-Dichlorophenyl)piperazin-1-yl]butoxy]pyrazolo[1,5-a]pyridin-3-carbaldehyde
- 7-[4-[4-(2-Methoxyphenyl)piperazin-1-yl]butoxy]pyrazolo[1,5-a]pyridin-3-carbaldehyde
- 7-[4-[4-(2-Chlorophenyl)piperazin-1-yl]butoxy]pyrazolo[1,5-a]pyridin-3-carbaldehyde
- 7-[4-[4-(2,3-Dichlorophenyl)piperazin-1-yl]butoxy]tetrazolo[1,5-a]pyridine
- 7-[4-[4-(2-Methoxyphenyl)piperazin-1-yl]butoxy]tetrazolo[1,5-a]pyridine
- 7-[4-[4-(2-Chloroyphenyl)piperazin-1-yl]butoxy]tetrazolo[1,5-a]pyridine
- 5-[4-[4-(3-Trif)uoromethylphenyl)piperazin-1-yl]butoxy]pyrazolo[1,5-a]pyridine
- 5-[4-[4-(3-Nitrophenyl)piperazin-1-yl]butoxy]pyrazolo[1,5-a]pyridine
- 5-[4-[4-(3-Aminophenyl)piperazin-1-yl]butoxy]pyrazolo[1,5-a]pyridine
- *N*-[3-[4-[4-(Pyrazolo[1,5-a]pyridin-5-yloxy)butyl]piperazin-1-yl]phenyl]acetamide
- 5-[4-[4-(3-Trifluoromethylphenyl)piperazin-1-yl]butoxy]pyrazolo[1,5-a]pyridin-3-carbaldehyde
- 5-[4-[4-(3-Nitrophenyl)piperazin-1-yl]butoxy]pyrazolo[1,5-a]pyridin-3-carbaldehyde
- *N*-[3-[4-[4-(3-Formylpyrazolo[1,5-a]pyridin-5-yloxy)butyl]piperazin-1-yl]phenyl]acetamide
- 5-[4-[4-(2,3-Dichlorophenyl)piperazin-1-yl]butoxy]-3-hydroxymethylpyrazolo[1,5-a]pyridine
- 3-Hydroxymethyl-5-[4-[4-(2-methoxyphenyl)piperazin-1-yl]butoxy]methylpyrazolo[1,5-a]pyridine
- 5-[4-[4-(2-Chlorophenyl)piperazin-1-yl]butoxy]-3-hydroxymethylpyrazolo[1,5-a]pyridine
- 5-[4-[4-(2,3-Dichlorophenyl)piperazin-1-yl]butoxy]-3-methylpyrazolo[1,5-a]pyridine
- 5-[4-[4-(2-Methoxyphenyl)piperazin-1-yl]butoxy]-3-methylpyrazolo[1,5-a]pyridine
- 5-[4-[4-(2-Chlorophenyl)piperazin-1-yl]butoxy]-3-methylpyrazolo[1,5-a]pyridine
- 3-Aminomethyl-5-[4-[4-(2,3-dichlorophenyl)piperazin-1-yl]butoxy]pyrazolo[1,5-a]pyridine
- 3-Aminomethyl-5-[4-[4-(2-methoxyphenyl)piperazin-1-yl]butoxy]pyrazolo[1,5-a]pyridine
- 3-Aminomethyl-5-[4-[4-(2-chlorophenyl)piperazin-1-yl]butoxy]pyrazolo[1,5-a]pyridine
- *N*-[5-[4-[4-(2,3-Dichlorophenyl)piperazin-1-yl]butoxy]pyrazolo[1,5-a]pyridin-3-ylmethyl]acetamide
- *N*-[5-[4-[4-(2-Methoxyphenyl)piperazin-1-yl]butoxy]pyrazolo[1,5-a]pyridin-3-ylmethyl]acetamide
- *N*-[5-[4-[4-(2-Chlorophenyl)piperazin-1-yl]butoxy]pyrazolo[1,5-a]pyridin-3-ylmethyl]acetamide
- 5-[4-(4-(2,3-Dihydrobenzofuran-7-yl)piperazin-1-yl)butoxy]pyrazolo[1,5-a]pyridine
- 5-[4-(4-(Chroman-8-yl)piperazin-1-yl)butoxy]pyrazolo[1,5-a]pyridine
- 5-[4-(4-(2,3-Dichlorophenyl)-1,4-diazepan-1-yl)butoxy]pyrazolo[1,5-a]pyridine
- 5-[4-(4-(2-Methoxyphenyl)-1,4-diazepan-1-yl)butoxy]pyrazolo[1,5-a]pyridine
- 5-[4-(4-(2-Chlorophenyl)-1,4-diazepan-1-yl)butoxy]pyrazolo[1,5-a]pyridine
- 5-[4-(4-(2,3-Dihydrobenzofuran-7-yl)-1,4-diazepan-1-yl)butoxy]pyrazolo[1,5-a]pyridine
- 5-[4-(4-(Chroman-8-yl)-1,4-diazepan-1-yl)butoxy]pyrazolo[1,5-a]pyridine
- 5-[4-(4-(2,3-Dihydrobenzofuran-7-yl)piperazin-1-yl)butoxy]pyrazolo[1,5-a]pyridin-3-carbaldehyde
- 5-[4-(4-(Chroman-8-yl)piperazin-1-yl)butoxy]pyrazolo[1,5-a]pyridin-3-carbaldehyde
- 5-[4-(4-(2,3-Dichlorophenyl)-1,4-diazepan-1-yl)butoxy]pyrazolo[1,5-a]pyridin-3-carbaldehyde
- 5-[4-(4-(2-Methoxyphenyl)-1,4-diazepan-1-yl)butoxy]pyrazolo[1,5-a]pyridin-3-carbaldehyde
- 5-[4-(4-(2-Chlorophenyl)-1,4-diazepan-1-yl)butoxy]pyrazolo[1,5-a]pyridin-3-carbaldehyde
- 5-[4-(4-(2,3-Dihydrobenzofuran-7-y))-1,4-diazepan-1-yl)butoxy]pyrazolo[1,5-a]pyridin-3-carbaldehyde
- 5-[4-(4-(Chroman-8-yl)-1,4-diazepan-1-yl)butoxy]pyrazolo[1,5-a]pyridin-3-carbaldehyde
- 7-[4-[4-(2,3-Dichlorophenyl)piperazin-1-yl]butoxy]indolizine
- 7-[4-[4-(2-Methoxyphenyl)piperazin-1-yl]butoxy]indolizine
- 7-[4-[4-(2-Chloroyphenyl)piperazin-1-yl]butoxy]indolizine
- 6-[4-[4-(2,3-Dichlorophenyl)piperazin-1-yl]butoxy]imidazo[1,2-a]pyridine
- 6-[4-[4-(2-Methoxyphenyl)piperazin-1-yl]butoxy]imidazo[1,2-a]pyridine
- 6-[4-[4-(2-Chlorophenyl)piperazin-1-yl]butoxy]imidazo[1,2-a]pyridine
- 8-[4-[4-(2,3-Dichlorophenyl)piperazin-1-yl]butoxy]imidazo[1,2-a]pyridine
- 8-[4-[4-(2-Methoxyphenyl)piperazin-1-yl]butoxy]imidazo[1,2-a]pyridine
- 8-[4-[4-(2-Chlorophenyl)piperazin-1-yl]butoxy]imidazo[1,2-a]pyridine

**21.** A medicine comprising a compound of anyone of claims 1-24.

**22.** Pharmaceutical composition comprising a compound of anyone of claims 1-24 and a pharmaceutical acceptable carrier.

**23.** Use of a compound according to anyone of claims 1-24 for preparing a medicament for the treatment of a disease selected from psychotic diseases including manic phases of bipolar disorder, acute idiopathic psychotic illnesses, psychoses associated with other diseases, drug-induced psychoses, and schizophrenia; attention deficit hyperactivity disorder (ADHD); autism; bipolar disorder; cognitive impairment; idiopathic or drug-induced movement disorders such as akinesia and diskinesia; Parkinson's disease; mood disorders including major depressive disorders, substance-induced mood disorders or other forms of depression; anxiety disorders including panic attack, social phobia, or generalized anxiety disorders; obsessive-compulsive disorders; stress-related disorders; addiction disorders; sleep disorders; sexual dysfunction; amnesic and/or cognitive disorders, especially dementia; eating disorders including anorexia and bulemia; pain; and neurodegenerative diseases including Chorea Huntington and multiple sclerosis.

**24.** Use acc to claim 27, wherein the disease is schizophrenia.

**25.** Method of treating a patient having a disease selected from psychotic diseases including manic phases of bipolar disorder, acute idiopathic psychotic illnesses, psychoses associated with other diseases, drug-induced psychoses, and schizophrenia; attention deficit hyperactivity disorder (ADHD); autism; bipolar disorder; cognitive impairment; idiopathic or drug-induced movement disorders such as akinesia and diskinesia; Parkinson's disease; mood disorders including major depressive disorders, substance-induced mood disorders or other forms of depression; anxiety disorders including panic attack, social phobia, or generalized anxiety disorders; obsessive-compulsive disorders; stress-related disorders; addiction disorders; sleep disorders; sexual dysfunction; amnesic and/or cognitive disorders, especially dementia; eating disorders including anorexia and bulemia; pain; and neurodegenerative diseases including Chorea Huntington and multiple sclerosis by administering a compound of anyone of claims 1-24.

**26.** Method according to claim 29, wherein the disease is schizophrenia.

**27.** Method according to claims 29-30, wherein the patient is selected for said treatment of said disease based on a prior diagnosis of said disease.

**28.** Kit comprising a medicine according to claim 25, and instructions for its use.

**29.** A method of producing a compound according to anyone of claims 1-24 comprising the steps of
a. reacting a compound of formula XI with an activated alkylene having the formula XII to give a compound of formula XIII 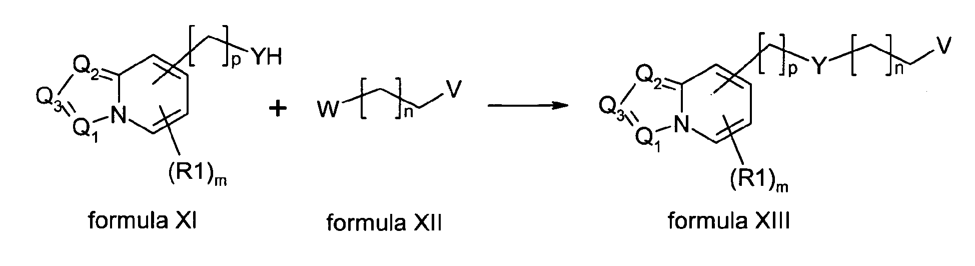 which compound of formula XIII is then combined with a compound of formula XIV 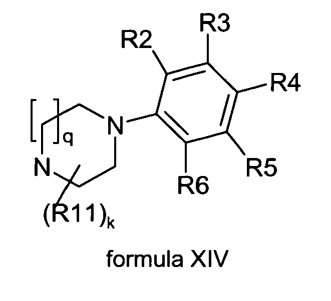 to give a compound of formula XV 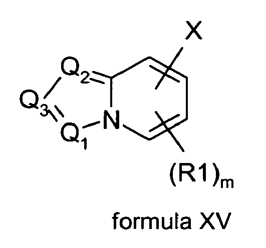 wherein in anyone of formulas XI, XII, XIII, XIV, and XV
R1, Q1, Q2, X, Y, n, R11, q, k, R2, R3, R4, R5, and R6 are as defined in anyone of claims 1-20 for the compounds of formulas I, IIa, IIb, IIc, IId, III, IV, V, VI, VII or VIII; and
W and V are activating groups;
and
(b) optionally adding to the compound of formula XV one or more additional groups R1 to give a compound of anyone of formula I, IIa, IIb, IIc, IId, III, IV, V, VI, VII or VIII according to anyone of claims 1-24.

**34.** A method according to claim 33, wherein the activating groups W and V are independently selected from among bromo, chloro, iodine, mesylate, triflate or tosylate.

**35.** A method according to claim 33, wherein in step (b) to a compound of formula XVa an additional group R1' is added to give a compound of formula XVI, which compound of formula XVI is dentical to or can be further transformed into a compound of formula I, IIa, IIb, IIc, IId, III, IV, V, VI, VII or VIII according to anyone of claims 1, 2, 12, 15, 18 or 21 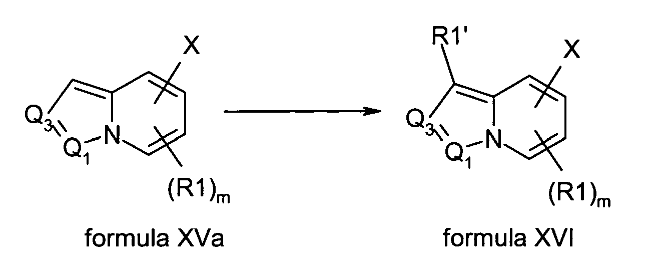 wherein in formulas XVa and XVI
R1' is selected from among bromo, chloro, iodine, formyl, hydroxymethyl, alkyl, oxime, cyano, aminomethyl, acylaminomethyl; and
R1, Q1, Q3, X and m are as defined in anyone of claims 1-23.

**36.** A method of producing a compound of formula XVI according to claim 35 wherein R1' is selected from among bromo, chloro, iodine and formyl and R1' is introduced by the reaction of a compound of formula XVa with an activated precursor to get a compound of formula XVI

**37.** A method according to claim 33, comprising the steps of
(b1) adding to a compound of formula XVa a formyl group to give a compound of formula XVIa 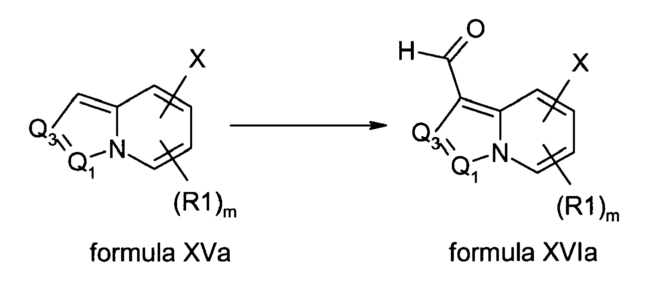 and
(b2) transforming the formyl group of formula XVIa to give a compound of formula XVII which compound of formula XVII is comprised by formula I, IIa, IIb, IIc, IId, III, IV, V, VI, VII or VIII according to anyone of claims 1, 2, 12, 15, 18, or 21, 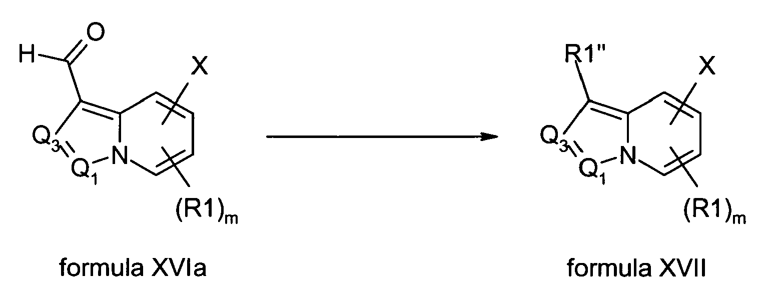 wherein R1" is selected from among hydroxymethyl, alkyl, oxime, cyano, aminomethyl and acylaminomethyl,
and Q1, Q3, X and m as as defined in any of the claims 1-20.
